# EUROPEAN PATENT SPECIFICATION

(11) **EP 3 035 960 B1**
(45) Date of publication and mention of the grant of the patent: **03.07.2019**
(21) Application number: 14755336.6
(22) Date of filing: 21.08.2014
(51) Int. Cl.: A61K 39/155

(54) **RESPIRATORY SYNCYTIAL VIRUS (RSV) VACCINE**
IMPFSTOFF GEGEN DAS RESPIRATORISCHE SYNZYTIALVIRUS
VACCIN CONTRE LE VIRUS RESPIRATOIRE SYNCYTIAL

(30) Priority: 21.08.2013 WO PCT/EP2013/002518
(43) Date of publication of application: 29.06.2016
(60) Divisional application: 19173393.0
(73) Proprietor: CureVac AG, 72076 Tübingen (DE)
(72) Inventor: KRAMPS, Thomas, 72706 Tübingen (DE); SCHNEE, Margit, 72706 Tübingen (DE); VOSS, Daniel, 72706 Tübingen (DE); PETSCH, Benjamin, 72706 Tübingen (DE)
(74) Representative: Graf von Stosch, Andreas
(86) International application number: PCT/EP2014/002301
(87) International publication number: WO 2015/024668

(56) References cited:
- WO-A1-2012/019630
- WO-A1-2012/116714
- WO-A1-2012/116715
- WO-A2-2006/015789
- WO-A2-2006/050280
- BENJAMIN PETSCH ET AL: "Protective efficacy of in vitro synthesized, specific mRNA vaccines against influenza A virus infection", NATURE BIOTECHNOLOGY, vol. 30, no. 12, 1 January 2012 (2012-01-01), pages 1210-1216, XP055051005, ISSN: 1087-0156, DOI: 10.1038/nbt.2436
- SCHLAKE THOMAS ET AL: "Developing mRNA-vaccine technologies.", RNA BIOLOGY NOV 2012, vol. 9, no. 11, November 2012 (2012-11), pages 1319-1330, XP055098782, ISSN: 1555-8584
- FOTIN-MLECZEK MARIOLA ET AL: "Highly potent mRNA based cancer vaccines represent an attractive platform for combination therapies supporting an improved therapeutic effect.", THE JOURNAL OF GENE MEDICINE JUN 2012, vol. 14, no. 6, June 2012 (2012-06), pages 428-439, XP002719717, ISSN: 1521-2254
- BAUER ASLI PETRA ET AL: "The impact of intragenic CpG content on gene expression.", NUCLEIC ACIDS RESEARCH JUL 2010, vol. 38, no. 12, July 2010 (2010-07), pages 3891-3908, XP002719888, ISSN: 1362-4962
- OOMENS ANTONIUS G P ET AL: "The cytoplasmic tail of the human respiratory syncytial virus F protein plays critical roles in cellular localization of the F protein and infectious progeny production.", JOURNAL OF VIROLOGY NOV 2006, vol. 80, no. 21, November 2006 (2006-11), pages 10465-10477, XP002719889, ISSN: 0022-538X
- HIGGINS DEBORAH ET AL: "Advances in RSV vaccine research and development - A global agenda", VACCINE, ELSEVIER, AMSTERDAM, NL, vol. 34, no. 26, 19 April 2016 (2016-04-19), pages 2870-2875, XP029560780, ISSN: 0264-410X, DOI: 10.1016/J.VACCINE.2016.03.109
- ANDERSON L J ET AL: "Strategic priorities for respiratory syncytial virus (RSV) vaccine development", VACCINE, ELSEVIER, AMSTERDAM, NL, vol. 31, 16 April 2013 (2013-04-16), XP028579217, ISSN: 0264-410X, DOI: 10.1016/J.VACCINE.2012.11.106
- Bart G. Jones ET AL: "Sendai virus-based RSV vaccine protects African green monkeys from RSV infection", Vaccine, vol. 30, no. 5, 1 January 2012 (2012-01-01), pages 959-968, XP055471614, AMSTERDAM, NL ISSN: 0264-410X, DOI: 10.1016/j.vaccine.2011.11.046
- MCLELLAN, JASON S: "Structure and Function of Respiratory Syncytial Virus Surface Glycoproteins", CURR TOP MICROBIOL IMMUNOL, vol. 372, 28 October 2014 (2014-10-28), pages 83-104,
- Koichi Hashimoto ET AL: "Neutralizing epitopes of RSV and palivizumab resistance in Japan", FUKUSHIMA JOURNAL OF MEDICAL SCIENCE, 1 January 2017 (2017-01-01), pages 127-134, XP055527102, DOI: 10.5387/fms.2017-09 Retrieved from the Internet: URL:https://www.jstage.jst.go.jp/article/f ms/63/3/63_2017-09/_pdf/-char/en
- Anne M Hause ET AL: "Sequence variability of the respiratory syncytial virus (RSV) fusion gene among contemporary and historical genotypes of RSV/A and RSV/B", PLoS ONE, 1 April 2017 (2017-04-01), XP055527105, DOI: 10.1371/journal.pone.0175792 Retrieved from the Internet: URL:https://journals.plos.org/plosone/arti cle/file?id=10.1371/journal.pone.0175792&t ype=printable

## Description

The present invention is defined by the attached claims and relates to an mRNA sequence comprising a coding region encoding a mutant of fusion protein F of Respiratory syncytial virus (RSV),), wherein the amino acid residues 554-574 of the amino acid sequence of the fusion protein F are deleted in the mutant of fusion protein F, wherein the G/C content of the coding region is increased by substituting at least 70% of the substitutable codons in the coding region compared with the G/C content of the coding region of the wild type mRNA, and wherein the coded amino acid sequence of said GC-enriched mRNA is not being modified compared with the coded amino acid sequence of the wild type mRNA. Additionally the present invention relates to a composition comprising a plurality of mRNA sequences as defined above. Furthermore it also discloses the use of the mRNA sequence or the composition comprising a plurality of mRNA sequences for the preparation of a pharmaceutical composition, especially a vaccine, e.g. for use in the prophylaxis or treatment of RSV infections. The invention further relates to a kit or kit of parts comprising the mRNA sequence or the (pharmaceutical) composition described herein. Moreover, the present invention further provides the mRNA sequence, the (pharmaceutical) composition or the kit or kit of parts described herein for use as a medicament, in particular for use in the treatment or prophylaxis of RSV infections.
The global medical need and economic impact of RSV is very high. It is the most important cause of acute lower respiratory tract infections (ALRIs) that result in hospital visits during infancy and early childhood. For example, in the United States, more than 60% of infants are infected by RSV during their first RSV season, and nearly all have been infected by 2-3 years of age. Approximately 2.1 million US children less than 5 years of age are treated for RSV disease each year: 3% as inpatients, 25% in emergency departments, and 73% in pediatric practices. Globally, among children less than five years of age, RSV causes an estimated 33.8 million ALRIs each year (more than 22% of all ALRIs), resulting in 66 000-199 000 deaths, 99% of which occur in developing countries. RSV is also a common cause of respiratory disease among the elderly, resulting in as many hospitalizations as influenza in a heavily influenza-immunized population. RSV spreads by respiratory droplets and close contact with infected persons or contaminated objects. In temperate climates, there is an annual winter epidemic. Infants are at highest risk for severe RSV disease in their first 6 months, and hospitalization peaks at 2-3 months of age. Preterm birth and cardiopulmonary disease are risk factors for severe RSV disease. RSV infection of infants elicits partially protective immunity, which appears to wane more rapidly than immunity against most other respiratory viruses. Most children infected with RSV during their first year are re-infected the next year, generally with less severe disease. Re-infections continue throughout life, often with upper respiratory tract symptoms, and sometimes with lower respiratory tract or sinus involvement. Recommended treatment of RSV bronchiolitis consists primarily of respiratory support and hydration. No specific anti-viral therapy is recommended. The neutralizing monoclonal antibody Palivizumab is used for prophylaxis of infants at highest risk for severe infection but is too expensive and impractical for universal use. Currently, there is no licensed RSV vaccine, and developing a safe and effective RSV vaccine is a global public health priority.
In a vaccine trial in the 1960s, infants and young children were immunized with a formalin-inactivated whole virion RSV preparation (FIRSV) or an equivalent paramyxovirus preparation (FIPIV). Five percent of the subjects who were immunized with FI-PIV and then naturally infected by RSV during the next RSV season were hospitalized; 80% of those who were immunized with FI-RSV and then infected by RSV were hospitalized, and two children died. This enhancement of an RSV infection due to vaccination is a specific problem for the development of vaccines against RSV infections (reviewed in Shaw et al. Curr Opin Virol. 2013 Jun;3(3):332-42. doi: 10.1016/j.coviro.2013.05.003. Epub 2013 May 30.).

Therefore, Respiratory syncytial virus (RSV) infections are the greatest remaining unmet infant vaccine need in developed countries and an important unmet infant vaccine need worldwide. More than 40 years of effort have not yet resulted in a licensed RSV vaccine for humans.

In summary, RSV which belongs to the virus family of *Paramyxoviridae,* is one of the most contagious pathogens and makes a substantial contribution to severe respiratory tract infections in infants, the elderly and immunocompromised patients.

As mentioned above, currently a humanised monoclonal antibody against the viral surface F protein is the only prophylactic product on the market which is recommended for infants considered at high risk including pre-term infants and infants with chronic lung disease (The IMpact-RSV Study Group. 1998. Palivizumab, a Humanized Respiratory Syncytial Virus Monoclonal Antibody, Reduces Hospitalization From Respiratory Syncytial Virus Infection in High-risk Infants. Pediatrics, 102(3), S.531-537., Tablan et al. 2003. Guidelines for preventing health-care--associated pneumonia, 2003: recommendations of CDC and the Healthcare Infection Control Practices Advisory Committee. MMWR. Recommendations and Reports: Morbidity and Mortality Weekly Report. Recommendations and Reports / Centers for Disease Control, 53(RR-3), S.1-36.).

RSV G and F proteins are required for virus infectivity and it was found that the cytoplasmic tail of the F protein plays critical roles in cellular localization of the F protein and in the generation of infectious virus progeny (Oomens et al. 2006. J. Virol. 80(21):10465-77). Recent studies with animal models demonstrated that sufficient amounts of neutralising antibodies targeting RSV F protein limit viral replication leading to a less severe course of disease (Singh, S.R. et al., 2007. Immunogenicity and efficacy of recombinant RSV-F vaccine in a mouse model. Vaccine, 25(33), S.6211-6223., Zhan, X. et al., 2007. Respiratory syncytial virus (RSV) F protein expressed by recombinant Sendai virus elicits B-cell and T-cell responses in cotton rats and confers protection against RSV subtypes A and B. Vaccine, 25(52), S.8782-8793., Vaughan, K., et al., 2005. DNA immunization against respiratory syncytial virus (RSV) in infant rhesus monkeys. Vaccine, 23(22), S.2928-2942).

Moreover, it could be shown that a balanced regulatory and effector T cell function is required for viral clearance and reduction of severity of illness (Liu, J. et al., 2010. Epitope-specific regulatory CD4 T cells reduce virus-induced illness while preserving CD8 T-cell effector function at the site of infection. journal of Virology, 84(20), S.10501-10509).

Despite the above mentioned humanised monoclonal antibody, live-attenuated vaccine viruses were developed which elicit a strong immune response, but which are not recommended for use in the specific target groups (infants, children, the elderly and immunocompromised patients). Also, DNA vectors expressing RSV F protein which bears B-cell epitopes were used to induce the production of neutralizing antibodies. In this context, WO 2008/077527 and WO 96/040945 disclose vectors comprising DNA sequences encoding RSV F protein for the use as vaccines. However, the use of DNA as a vaccine may be dangerous due to unwanted insertion into the genome, possibly leading to interruption of functional genes and cancer or the formation of anti-DNA antibodies. WO 2012/116714 describes vaccines comprising an antigen encoding mRNA for use in elderly patients.

Therefore it is the object of the underlying invention to provide an mRNA sequence coding for antigenic peptides or proteins of Respiratory syncytial virus (RSV) for the use as vaccine for prophylaxis or treatment of RSV infections, particularly in infants, the elderly and immunocompromised patients.

These objects are solved by the subject matter of the attached claims. Particularly, the objects underlying the present invention are solved according to a first aspect by an inventive mRNA sequence as defined by the claims.

For the sake of clarity and readability the following scientific background information and definitions are provided. Additional definitions and explanations can be provided in the context of this disclosure.

Immune system: The immune system may protect organisms from infection. If a pathogen breaks through a physical barrier of an organism and enters this organism, the innate immune system provides an immediate, but non-specific response. If pathogens evade this innate response, vertebrates possess a second layer of protection, the adaptive immune system. Here, the immune system adapts its response during an infection to improve its recognition of the pathogen. This improved response is then retained after the pathogen has been eliminated, in the form of an immunological memory, and allows the adaptive immune system to mount faster and stronger attacks each time this pathogen is encountered. According to this, the immune system comprises the innate and the adaptive immune system. Each of these two parts contains so called humoral and cellular components.

Immune response: An immune response may typically either be a specific reaction of the adaptive immune system to a particular antigen (so called specific or adaptive immune response) or an unspecific reaction of the innate immune system (so called unspecific or innate immune response). The invention relates to the core to specific reactions (adaptive immune responses) of the adaptive immune system. Particularly, it relates to adaptive immune responses to infections by viruses like e.g. RSV infections. However, this specific response can be supported by an additional unspecific reaction (innate immune response). Therefore, the invention also relates to a compound for simultaneous stimulation of the innate and the adaptive immune system to evoke an efficient adaptive immune response.

Adaptive immune system: The adaptive immune system is composed of highly specialized, systemic cells and processes that eliminate or prevent pathogenic growth. The adaptive immune response provides the vertebrate immune system with the ability to recognize and remember specific pathogens (to generate immunity), and to mount stronger attacks each time the pathogen is encountered. The system is highly adaptable because of somatic hypermutation (a process of increased frequency of somatic mutations), and V(D)J recombination (an irreversible genetic recombination of antigen receptor gene segments). This mechanism allows a small number of genes to generate a vast number of different antigen receptors, which are then uniquely expressed on each individual lymphocyte. Because the gene rearrangement leads to an irreversible change in the DNA of each cell, all of the progeny (offspring) of that cell will then inherit genes encoding the same receptor specificity, including the Memory B cells and Memory T cells that are the keys to long-lived specific immunity. Immune network theory is a theory of how the adaptive immune system works, that is based on interactions between the variable regions of the receptors of T cells, B cells and of molecules made by T cells and B cells that have variable regions.

Adaptive immune response: The adaptive immune response is typically understood to be antigen-specific. Antigen specificity allows for the generation of responses that are tailored to specific antigens, pathogens or pathogen-infected cells. The ability to mount these tailored responses is maintained in the body by "memory cells". Should a pathogen infect the body more than once, these specific memory cells are used to quickly eliminate it. In this context, the first step of an adaptive immune response is the activation of naïve antigen-specific T cells or different immune cells able to induce an antigen-specific immune response by antigen-presenting cells. This occurs in the lymphoid tissues and organs through which naive T cells are constantly passing. Cell types that can serve as antigen-presenting cells are inter alia dendritic cells, macrophages, and B cells. Each of these cells has a distinct function in eliciting immune responses. Dendritic cells take up antigens by phagocytosis and macropinocytosis and are stimulated by contact with e.g. a foreign antigen to migrate to the local lymphoid tissue, where they differentiate into mature dendritic cells. Macrophages ingest particulate antigens such as bacteria and are induced by infectious agents or other appropriate stimuli to express MHC molecules. The unique ability of B cells to bind and internalize soluble protein antigens via their receptors may also be important to induce T cells. Presenting the antigen on MHC molecules leads to activation of T cells which induces their proliferation and differentiation into armed effector T cells. The most important function of effector T cells is the killing of infected cells by CD8+ cytotoxic T cells and the activation of macrophages by Th1 cells which together make up cell-mediated immunity, and the activation of B cells by both Th2 and Th1 cells to produce different classes of antibody, thus driving the humoral immune response. T cells recognize an antigen by their T cell receptors which do not recognize and bind antigen directly, but instead recognize short peptide fragments e.g. of pathogen-derived protein antigens, which are bound to MHC molecules on the surfaces of other cells.

Cellular immunity/cellular immune response: Cellular immunity relates typically to the activation of macrophages, natural killer cells (NK), antigen-specific cytotoxic T-lymphocytes, and the release of various cytokines in response to an antigen. In a more general way, cellular immunity is not related to antibodies but to the activation of cells of the immune system. A cellular immune response is characterized e.g. by activating antigen-specific cytotoxic T-lymphocytes that are able to induce apoptosis in body cells displaying epitopes of an antigen on their surface, such as virus-infected cells, cells with intracellular bacteria, and cancer cells displaying tumor antigens; activating macrophages and natural killer cells, enabling them to destroy pathogens; and stimulating cells to secrete a variety of cytokines that influence the function of other cells involved in adaptive immune responses and innate immune responses.

Humoral immunity/humoral immune response: Humoral immunity refers typically to antibody production and the accessory processes that may accompany it. A humoral immune response may be typically characterized, e.g., by Th2 activation and cytokine production, germinal center formation and isotype switching, affinity maturation and memory cell generation. Humoral immunity also typically may refer to the effector functions of antibodies, which include pathogen and toxin neutralization, classical complement activation, and opsonin promotion of phagocytosis and pathogen elimination.

Innate immune system: The innate immune system, also known as non-specific immune system, comprises the cells and mechanisms that defend the host from infection by other organisms in a non-specific manner. This means that the cells of the innate system recognize and respond to pathogens in a generic way, but unlike the adaptive immune system, it does not confer long-lasting or protective immunity to the host. The innate immune system may be e.g. activated by ligands of pathogen-associated molecular patterns (PAMP) receptors, e.g. Toll-like receptors (TLRs) or other auxiliary substances such as lipopolysaccharides, TNF-alpha, CD40 ligand, or cytokines, monokines, lymphokines, interleukins or chemokines, IL-1, IL-2, IL-3, IL-4, IL-5, IL-6, IL-7, IL-8, IL-9, IL-10, IL-12, IL-13, IL-14, IL-15, IL-16, IL-17, IL-18, IL-19, IL-20, IL-21, IL-22, IL-23, IL-24, IL-25, IL-26, IL-27, IL-28, IL-29, IL-30, IL-31, IL-32, IL-33, IFN-alpha, IFN-beta, IFN-gamma, GM-CSF, G-CSF, M-CSF, LT-beta, TNF-alpha, growth factors, and hGH, a ligand of human Toll-like receptor TLR1, TLR2, TLR3, TLR4, TLR5, TLR6, TLR7, TLR8, TLR9, TLR10, a ligand of murine Toll-like receptor TLR1, TLR2, TLR3, TLR4, TLR5, TLR6, TLR7, TLR8, TLR9, TLR10, TLR11, TLR12 or TLR13, a ligand of a NOD-like receptor, a ligand of a RIG-I like receptor, an immunostimulatory nucleic acid, an immunostimulatory RNA (isRNA), a CpG-DNA, an antibacterial agent, or an anti-viral agent. Typically a response of the innate immune system includes recruiting immune cells to sites of infection, through the production of chemical factors, including specialized chemical mediators, called cytokines; activation of the complement cascade; identification and removal of foreign substances present in organs, tissues, the blood and lymph, by specialized white blood cells; activation of the adaptive immune system through a process known as antigen presentation; and/or acting as a physical and chemical barrier to infectious agents.

Adjuvant / adjuvant component: An adjuvant or an adjuvant component in the broadest sense is typically a (e.g. pharmacological or immunological) agent or composition that may modify, e.g. enhance, the efficacy of other agents, such as a drug or vaccine. Conventionally the term refers in the context of the invention to a compound or composition that serves as a carrier or auxiliary substance for immunogens and/or other pharmaceutically active compounds. It is to be interpreted in a broad sense and refers to a broad spectrum of substances that are able to increase the immunogenicity of antigens incorporated into or co-administered with an adjuvant in question. In the context of the present invention an adjuvant will preferably enhance the specific immunogenic effect of the active agents of the present invention. Typically, "adjuvant" or "adjuvant component" has the same meaning and can be used mutually. Adjuvants may be divided, e.g., into immuno potentiators, antigenic delivery systems or even combinations thereof.

The term "adjuvant" is typically understood not to comprise agents which confer immunity by themselves. An adjuvant assists the immune system unspecifically to enhance the antigen-specific immune response by e.g. promoting presentation of an antigen to the immune system or induction of an unspecific innate immune response. Furthermore, an adjuvant may preferably e.g. modulate the antigen-specific immune response by e.g. shifting the dominating Th2-based antigen specific response to a more Th1-based antigen specific response or vice versa. Accordingly, an adjuvant may favourably modulate cytokine expression/secretion, antigen presentation, type of immune response etc.

Immunostimulatory RNA: An immunostimulatory RNA (isRNA) in the context of the invention may typically be a RNA that is able to induce an innate immune response itself. It usually does not have an open reading frame and thus does not provide a peptide-antigen or immunogen but elicits an innate immune response e.g. by binding to a specific kind of Toll-like-receptor (TLR) or other suitable receptors. However, of course also mRNAs having an open reading frame and coding for a peptide/protein (e.g. an antigenic function) may induce an innate immune response.

Antigen: According to the present invention, the term "antigen" refers typically to a substance which may be recognized by the immune system and may be capable of triggering an antigen-specific immune response, e.g. by formation of antibodies or antigen-specific T-cells as part of an adaptive immune response. An antigen may be a protein or peptide. In this context, the first step of an adaptive immune response is the activation of naive antigen-specific T cells by antigen-presenting cells. This occurs in the lymphoid tissues and organs through which naïve T cells are constantly passing. The three cell types that can serve as antigen-presenting cells are dendritic cells, macrophages, and B cells. Each of these cells has a distinct function in eliciting immune responses. Tissue dendritic cells take up antigens by phagocytosis and macropinocytosis and are stimulated by infection to migrate to the local lymphoid tissue, where they differentiate into mature dendritic cells. Macrophages ingest particulate antigens such as bacteria and are induced by infectious agents to express MHC class II molecules. The unique ability of B cells to bind and internalize soluble protein antigens via their receptors may be important to induce T cells. By presenting the antigen on MHC molecules leads to activation of T cells which induces their proliferation and differentiation into armed effector T cells. The most important function of effector T cells is the killing of infected cells by CD8⁺ cytotoxic T cells and the activation of macrophages by TH1 cells which together make up cell-mediated immunity, and the activation of B cells by both TH2 and TH1 cells to produce different classes of antibody, thus driving the humoral immune response. T cells recognize an antigen by their T cell receptors which does not recognize and bind antigen directly, but instead recognize short peptide fragments e.g. of pathogens' protein antigens, which are bound to MHC molecules on the surfaces of other cells.

T cells fall into two major classes that have different effector functions. The two classes are distinguished by the expression of the cell-surface proteins CD4 and CD8. These two types of T cells differ in the class of MHC molecule that they recognize. There are two classes of MHC molecules - MHC class I and MHC class II molecules - which differ in their structure and expression pattern on tissues of the body. CD4⁺ T cells bind to a MHC class II molecule and CD8⁺ T cells to a MHC class I molecule. MHC class I and MHC class II molecules have distinct distributions among cells that reflect the different effector functions of the T cells that recognize them. MHC class I molecules present peptides of cytosolic and nuclear origin e.g. from pathogens, commonly viruses, to CD8⁺ T cells, which differentiate into cytotoxic T cells that are specialized to kill any cell that they specifically recognize. Almost all cells express MHC class I molecules, although the level of constitutive expression varies from one cell type to the next. But not only pathogenic peptides from viruses are presented by MHC class I molecules, also self-antigens like tumour antigens are presented by them. MHC class I molecules bind peptides from proteins degraded in the cytosol and transported in the endoplasmic reticulum. The CD8⁺ T cells that recognize MHC class I:peptide complexes at the surface of infected cells are specialized to kill any cells displaying foreign peptides and so rid the body of cells infected with viruses and other cytosolic pathogens. The main function of CD4⁺ T cells (CD4⁺ helper T cells) that recognize MHC class II molecules is to activate other effector cells of the immune system. Thus MHC class II molecules are normally found on B lymphocytes, dendritic cells, and macrophages, cells that participate in immune responses, but not on other tissue cells. Macrophages, for example, are activated to kill the intravesicular pathogens they harbour, and B cells to secrete immunoglobulins against foreign molecules. MHC class II molecules are prevented from binding to peptides in the endoplasmic reticulum and thus MHC class II molecules bind peptides from proteins which are degraded in endosomes. They can capture peptides from pathogens that have entered the vesicular system of macrophages, or from antigens internalized by immature dendritic cells or the immunoglobulin receptors of B cells. Pathogens that accumulate in large numbers inside macrophage and dendritic cell vesicles tend to stimulate the differentiation of TH1 cells, whereas extracellular antigens tend to stimulate the production of TH2 cells. TH1 cells activate the microbicidal properties of macrophages and induce B cells to make IgG antibodies that are very effective of opsonising extracellular pathogens for ingestion by phagocytic cells, whereas TH2 cells initiate the humoral response by activating naïve B cells to secrete IgM, and induce the production of weakly opsonising antibodies such as IgG1 and IgG3 (mouse) and IgG2 and IgG4 (human) as well as IgA and IgE (mouse and human).

Epitope (also called "antigen determinant"): T cell epitopes or parts of the proteins in the context of the present invention may comprise fragments preferably having a length of about 6 to about 20 or even more amino acids, e.g. fragments as processed and presented by MHC class I molecules, preferably having a length of about 8 to about 10 amino acids, e.g. 8, 9, or 10, (or even 11, or 12 amino acids), or fragments as processed and presented by MHC class II molecules, preferably having a length of about 13 or more amino acids, e.g. 13, 14, 15, 16, 17, 18, 19, 20 or even more amino acids, wherein these fragments may be selected from any part of the amino acid sequence. These fragments are typically recognized by T cells in form of a complex consisting of the peptide fragment and an MHC molecule.

B cell epitopes are typically fragments located on the outer surface of (native) protein or peptide antigens as defined herein, preferably having 5 to 15 amino acids, more preferably having 5 to 12 amino acids, even more preferably having 6 to 9 amino acids, which may be recognized by antibodies, i.e. in their native form.

Such epitopes of proteins or peptides may furthermore be selected from any of the herein mentioned variants of such proteins or peptides. In this context antigenic determinants can be conformational or discontinuous epitopes which are composed of segments of the proteins or peptides as defined herein that are discontinuous in the amino acid sequence of the proteins or peptides as defined herein but are brought together in the three-dimensional structure or continuous or linear epitopes which are composed of a single polypeptide chain.

Vaccine: A vaccine is typically understood to be a prophylactic or therapeutic material providing at least one antigen or antigenic function. The antigen or antigenic function may stimulate the body's adaptive immune system to provide an adaptive immune response.

Antigen-providing mRNA: An antigen-providing mRNA in the context of the invention may typically be an mRNA, having at least one open reading frame that can be translated by a cell or an organism provided with that mRNA. The product of this translation is a peptide or protein that may act as an antigen, preferably as an immunogen. The product may also be a fusion protein composed of more than one immunogen, e.g. a fusion protein that consist of two or more epitopes, peptides or proteins derived from the same or different virus-proteins, wherein the epitopes, peptides or proteins may be linked by linker sequences.

Bi-/multicistronic mRNA: mRNA, that typically may have two (bicistronic) or more (multicistronic) open reading frames (ORF). An open reading frame in this context is a sequence of several nucleotide triplets (codons) that can be translated into a peptide or protein. Translation of such a mRNA yields two (bicistronic) or more (multicistronic) distinct translation products (provided the ORFs are not identical). For expression in eukaryotes such mRNAs may for example comprise an internal ribosomal entry site (IRES) sequence.

5'-CAP-Structure: A 5'-CAP is typically a modified nucleotide, particularly a guanine nucleotide, added to the 5' end of an mRNA-molecule. Preferably, the 5'-CAP is added using a 5'-5'-triphosphate linkage (also named m7GpppN). Further examples of 5'-CAP structures include glyceryl, inverted deoxy abasic residue (moiety), 4',5' methylene nucleotide, 1-(beta-D-erythrofuranosyl) nucleotide, 4'-thio nucleotide, carbocyclic nucleotide, 1,5-anhydrohexitol nucleotide, L-nucleotides, alpha-nucleotide, modified base nucleotide, threo-pentofuranosyl nucleotide, acyclic 3',4'-seco nucleotide, acyclic 3,4-dihydroxybutyl nucleotide, acyclic 3,5 dihydroxypentyl nucleotide, 3'-3'-inverted nucleotide moiety, 3'-3'-i.nverted abasic moiety, 3'-2'-inverted nucleotide moiety, 3'-2'-inverted abasic moiety, 1,4-butanediol phosphate, 3'-phosphoramidate, hexylphosphate, aminohexyl phosphate, 3'-phosphate, 3'phosphorothioate, phosphorodithioate, or bridging or non-bridging methylphosphonate moiety. These modified 5'-CAP structures may be used in the context of the present invention to modify the inventive mRNA sequence. Further modified 5'-CAP structures which may be used in the context of the present invention are CAP1 (methylation of the ribose of the adjacent nucleotide of m7GpppN), CAP2 (methylation of the ribose of the 2^{nd} nucleotide downstream of the m7GpppN), CAP3 (methylation of the ribose of the 3^{rd} nucleotide downstream of the m7GpppN), CAP4 (methylation of the ribose of the 4^{th} nucleotide downstream of the m7GpppN), ARCA (anti-reverse CAP analogue, modified ARCA (e.g. phosphothioate modified ARCA), inosine, N1-methyl-guanosine, 2'-fluoro-guanosine, 7-deaza-guanosine, 8-oxo-guanosine, 2-amino-guanosine, LNA-guanosine, and 2-azido-guanosine.

Fragments of proteins: "Fragments" of proteins or peptides in the context of the present invention may, typically, comprise a sequence of a protein or peptide as defined herein, which is, with regard to its amino acid sequence (or its encoded nucleic acid molecule), N-terminally and/or C-terminally truncated compared to the amino acid sequence of the original (native) protein (or its encoded nucleic acid molecule). Such truncation may thus occur either on the amino acid level or correspondingly on the nucleic acid level. A sequence identity with respect to such a fragment as defined herein may therefore preferably refer to the entire protein or peptide as defined herein or to the entire (coding) nucleic acid molecule of such a protein or peptide.
Fragments of proteins or peptides in the context of the present invention may furthermore comprise a sequence of a protein or peptide as defined herein, which has a length of for example at least 5 amino acids, preferably a length of at least 6 amino acids, preferably at least 7 amino acids, more preferably at least 8 amino acids, even more preferably at least 9 amino acids; even more preferably at least 10 amino acids; even more preferably at least 11 amino acids; even more preferably at least 12 amino acids; even more preferably at least 13 amino acids; even more preferably at least 14 amino acids; even more preferably at least 15 amino acids; even more preferably at least 16 amino acids; even more preferably at least 17 amino acids; even more preferably at least 18 amino acids; even more preferably at least 19 amino acids; even more preferably at least 20 amino acids; even more preferably at least 25 amino acids; even more preferably at least 30 amino acids; even more preferably at least 35 amino acids; even more preferably at least 50 amino acids; or most preferably at least 100 amino acids. For example such fragment may have a length of about 6 to about 20 or even more amino acids, e.g. fragments as processed and presented by MHC class I molecules, preferably having a length of about 8 to about 10 amino acids, e.g. 8, 9, or 10, (or even 6, 7, 11, or 12 amino acids), or fragments as processed and presented by MHC class II molecules, preferably having a length of about 13 or more amino acids, e.g. 13, 14, 15, 16, 17, 18, 19, 20 or even more amino acids, wherein these fragments may be selected from any part of the amino acid sequence. These fragments are typically recognized by T-cells in form of a complex consisting of the peptide fragment and an MHC molecule, i.e. the fragments are typically not recognized in their native form. Fragments of proteins or peptides may comprise at least one epitope of those proteins or peptides. Furthermore also domains of a protein, like the extracellular domain, the intracellular domain or the transmembrane domain and shortened or truncated versions of a protein may be understood to comprise a fragment of a protein.

Variants of proteins: "Variants" of proteins or peptides as defined in the context of the present invention may be generated, having an amino acid sequence which differs from the original sequence in one or more mutation(s), such as one or more substituted, inserted and/or deleted amino acid(s). Preferably, these fragments and/or variants have the same biological function or specific activity compared to the full-length native protein, e.g. its specific antigenic property. "Variants" of proteins or peptides as defined in the context of the present invention may comprise conservative amino acid substitution(s) compared to their native, i.e. non-mutated physiological, sequence. Those amino acid sequences as well as their encoding nucleotide sequences in particular fall under the term variants as defined herein. Substitutions in which amino acids, which originate from the same class, are exchanged for one another are called conservative substitutions. In particular, these are amino acids having aliphatic side chains, positively or negatively charged side chains, aromatic groups in the side chains or amino acids, the side chains of which can enter into hydrogen bridges, e.g. side chains which have a hydroxyl function. This means that e.g. an amino acid having a polar side chain is replaced by another amino acid having a likewise polar side chain, or, for example, an amino acid characterized by a hydrophobic side chain is substituted by another amino acid having a likewise hydrophobic side chain (e.g. serine (threonine) by threonine (serine) or leucine (isoleucine) by isoleucine (leucine)). Insertions and substitutions are possible, in particular, at those sequence positions which cause no modification to the three-dimensional structure or do not affect the binding region. Modifications to a three-dimensional structure by insertion(s) or deletion(s) can easily be determined e.g. using CD spectra (circular dichroism spectra) (Urry, 1985, Absorption, Circular Dichroism and ORD of Polypeptides, in: Modern Physical Methods in Biochemistry, Neuberger et al. (ed.), Elsevier, Amsterdam).
A "variant" of a protein or peptide may have at least 70%, 75%, 80%, 85%, 90%, 95%, 98% or 99% amino acid identity over a stretch of 10, 20, 30, 50, 75 or 100 amino acids of such protein or peptide.
Furthermore, variants of proteins or peptides as defined herein, which may be encoded by a nucleic acid molecule, may also comprise those sequences, wherein nucleotides of the encoding nucleic acid sequence are exchanged according to the degeneration of the genetic code, without leading to an alteration of the respective amino acid sequence of the protein or peptide, i.e. the amino acid sequence or at least part thereof may not differ from the original sequence in one or more mutation(s) within the above meaning.

Identity of a sequence: In order to determine the percentage to which two sequences are identical, e.g. nucleic acid sequences or amino acid sequences as defined herein, preferably the amino acid sequences encoded by a nucleic acid sequence of the polymeric carrier as defined herein or the amino acid sequences themselves, the sequences can be aligned in order to be subsequently compared to one another. Therefore, e.g. a position of a first sequence may be compared with the corresponding position of the second sequence. If a position in the first sequence is occupied by the same component (residue) as is the case at a position in the second sequence, the two sequences are identical at this position. If this is not the case, the sequences differ at this position. If insertions occur in the second sequence in comparison to the first sequence, gaps can be inserted into the first sequence to allow a further alignment. If deletions occur in the second sequence in comparison to the first sequence, gaps can be inserted into the second sequence to allow a further alignment. The percentage to which two sequences are identical is then a function of the number of identical positions divided by the total number of positions including those positions which are only occupied in one sequence. The percentage to which two sequences are identical can be determined using a mathematical algorithm. A preferred, but not limiting, example of a mathematical algorithm which can be used is the algorithm of Karlin et al. (1993), PNAS USA, 90:5873-5877 or Altschul et al. (1997), Nucleic Acids Res., 25:3389-3402. Such an algorithm is integrated in the BLAST program. Sequences which are identical to the sequences of the present invention to a certain extent can be identified by this program.

Derivative of a protein or peptide: A derivative of a peptide or protein is typically understood to be a molecule that is derived from another molecule, such as said peptide or protein. A "derivative" of a peptide or protein also encompasses fusions comprising a peptide or protein used in the present invention. For example, the fusion comprises a label, such as, for example, an epitope, e.g., a FLAG epitope or a V5 epitope. For example, the epitope is a FLAG epitope. Such a tag is useful for, for example, purifying the fusion protein.

Monocistronic mRNA: A monocistronic mRNA may typically be an mRNA, that encodes only one open reading frame. An open reading frame in this context is a sequence of several nucleotide triplets (codons) that can be translated into a peptide or protein.

Nucleic acid: The term nucleic acid means any DNA- or RNA-molecule and is used synonymous with polynucleotide. Wherever herein reference is made to a nucleic acid or nucleic acid sequence encoding a particular protein and/or peptide, said nucleic acid or nucleic acid sequence, respectively, preferably also comprises regulatory sequences allowing in a suitable host, e.g. a human being, its expression, i.e. transcription and/or translation of the nucleic acid sequence encoding the particular protein or peptide.

Peptide: A peptide is a polymer of amino acid monomers. Usually the monomers are linked by peptide bonds. The term "peptide" does not limit the length of the polymer chain of amino acids. In some embodiments of the present invention a peptide may for example contain less than 50 monomer units. Longer peptides are also called polypeptides, typically having 50 to 600 monomeric units, more specifically 50 to 300 monomeric units.

Pharmaceutically effective amount: A pharmaceutically effective amount in the context of the invention is typically understood to be an amount that is sufficient to induce an immune response.

Protein: A protein typically consists of one or more peptides and/or polypeptides folded into 3-dimensional form, facilitating a biological function.

Poly (C) sequence: A poly-(C)-sequence is typically a long sequence of cytosine nucleotides, typically about 10 to about 200 cytosine nucleotides, preferably about 10 to about 100 cytosine nucleotides, more preferably about 10 to about 70 cytosine nucleotides or even more preferably about 20 to about 50 or even about 20 to about 30 cytosine nucleotides. A poly(C) sequence may preferably be located 3' of the coding region comprised by a nucleic acid.

Poly-A-tail: A poly-A-tail also called "3'-poly(A) tail" is typically a long sequence of adenosine nucleotides of up to about 400 adenosine nucleotides, e.g. from about 25 to about 400, preferably from about 50 to about 400, more preferably from about 50 to about 300, even more preferably from about 50 to about 250, most preferably from about 60 to about 250 adenosine nucleotides, added to the 3' end of a RNA.

Stabilized nucleic acid: A stabilized nucleic acid, typically, exhibits a modification increasing resistance to *in vivo* degradation (e.g. degradation by an exo- or endo-nuclease) and/or ex vivo degradation (e.g. by the manufacturing process prior to vaccine administration, e.g. in the course of the preparation of the vaccine solution to be administered). Stabilization of RNA can, e.g., be achieved by providing a 5'-CAP-Structure, a Poly-A-Tail, or any other UTR-modification. It can also be achieved by backbone-modification or modification of the G/C-content of the nucleic acid. Various other methods are known in the art and conceivable in the context of the invention.

Carrier / polymeric carrier: A carrier in the context of the invention may typically be a compound that facilitates transport and/or complexation of another compound. Said carrier may form a complex with said other compound. A polymeric carrier is a carrier that is formed of a polymer.

Cationic component: The term "cationic component" typically refers to a charged molecule, which is positively charged (cation) at a pH value of typically about 1 to 9, preferably of a pH value of or below 9 (e.g. 5 to 9), of or below 8 (e.g. 5 to 8), of or below 7 (e.g. 5 to 7), most preferably at physiological pH values, e.g. about 7.3 to 7.4. Accordingly, a cationic peptide, protein or polymer according to the present invention is positively charged under physiological conditions, particularly under physiological salt conditions of the cell *in vivo.* A cationic peptide or protein preferably contains a larger number of cationic amino acids, e.g. a larger number of Arg, His, Lys or Orn than other amino acid residues (in particular more cationic amino acids than anionic amino acid residues like Asp or Glu) or contains blocks predominantly formed by cationic amino acid residues. The definition "cationic" may also refer to "polycationic" components.

Vehicle: An agent, e.g. a carrier, that may typically be used within a pharmaceutical composition or vaccine for facilitating administering of the components of the pharmaceutical composition or vaccine to an individual.

3'-untranslated region (3'UTR): A 3'UTR is typically the part of an mRNA which is located between the protein coding region (i.e. the open reading frame) and the poly(A) sequence of the mRNA. A 3'UTR of the mRNA is not translated into an amino acid sequence. The 3'UTR sequence is generally encoded by the gene which is transcribed into the respective mRNA during the gene expression process. The genomic sequence is first transcribed into pre-mature mRNA, which comprises optional introns. The pre-mature mRNA is then further processed into mature mRNA in a maturation process. This maturation process comprises the steps of 5'-Capping, splicing the pre-mature mRNA to excise optional introns and modifications of the 3'-end, such as polyadenylation of the 3'-end of the pre-mature mRNA and optional endo- or exonuclease cleavages etc. In the context of the present invention, a 3'UTR corresponds to the sequence of a mature mRNA which is located 3' to the stop codon of the protein coding region, preferably immediately 3' to the stop codon of the protein coding region, and which extends to the 5'-side of the poly(A) sequence, preferably to the nucleotide immediately 5' to the poly(A) sequence. The term "corresponds to" means that the 3'UTR sequence may be an RNA sequence, such as in the mRNA sequence used for defining the 3'UTR sequence, or a DNA sequence which corresponds to such RNA sequence. In the context of the present invention, the term "a 3'UTR of a gene", such as "a 3'UTR of an albumin gene", is the sequence which corresponds to the 3'UTR of the mature mRNA derived from this gene, i.e. the mRNA obtained by transcription of the gene and maturation of the pre-mature mRNA. The term "3'UTR of a gene" encompasses the DNA sequence and the RNA sequence of the 3'UTR.

5'-untranslated region (5'UTR): A 5'-UTR is typically understood to be a particular section of messenger RNA (mRNA). It is located 5' of the open reading frame of the mRNA. Typically, the 5'UTR starts with the transcriptional start site and ends one nucleotide before the start codon of the open reading frame. The 5'-UTR may comprise elements for controlling gene expression, also called regulatory elements. Such regulatory elements may be, for example, ribosomal binding sites or a 5'-Terminal Oligopyrimidine Tract. The 5'UTR may be posttranscriptionally modified, for example by addition of a 5'-CAP. In the context of the present invention, a 5'UTR corresponds to the sequence of a mature mRNA which is located between the 5'-CAP and the start codon. Preferably, the 5'UTR corresponds to the sequence which extends from a nucleotide located 3' to the 5'-CAP, preferably from the nucleotide located immediately 3' to the 5'-CAP, to a nucleotide located 5' to the start codon of the protein coding region, preferably to the nucleotide located immediately 5' to the start codon of the protein coding region. The nucleotide located immediately 3' to the 5'-CAP of a mature mRNA typically corresponds to the transcriptional start site. The term "corresponds to" means that the 5'UTR sequence may be an RNA sequence, such as in the mRNA sequence used for defining the 5'UTR sequence, or a DNA sequence which corresponds to such RNA sequence. In the context of the present invention, the term "a 5'UTR of a gene", such as "a 5'UTR of a TOP gene", is the sequence which corresponds to the 5'UTR of the mature mRNA derived from this gene, i.e. the mRNA obtained by transcription of the gene and maturation of the pre-mature mRNA. The term "5'UTR of a gene" encompasses the DNA sequence and the RNA sequence of the 5'UTR.

5'Terminal Oligopyrimidine Tract (TOP): The 5'terminal oligopyrimidine tract (TOP) is typically a stretch of pyrimidine nucleotides located at the 5' terminal region of a nucleic acid molecule, such as the 5' terminal region of certain mRNA molecules or the 5' terminal region of a functional entity, e.g. the transcribed region, of certain genes. The sequence starts with a cytidine, which usually corresponds to the transcriptional start site, and is followed by a stretch of usually about 3 to 30 pyrimidine nucleotides. For example, the TOP may comprise 3, 4, 5, 6, 7, 8, 9, 10, 11, 12, 13, 14, 15, 16, 17, 18, 19, 20, 21, 22, 23, 24, 25, 26, 27, 28, 29, 30 or even more nucleotides. The pyrimidine stretch and thus the 5' TOP ends one nucleotide 5' to the first purine nucleotide located downstream of the TOP. Messenger RNA that contains a 5'terminal oligopyrimidine tract is often referred to as TOP mRNA. Accordingly, genes that provide such messenger RNAs are referred to as TOP genes. TOP sequences have, for example, been found in genes and mRNAs encoding peptide elongation factors and ribosomal proteins.

TOP motif: In the context of the present invention, a TOP motif is a nucleic acid sequence which corresponds to a 5'TOP as defined above. Thus, a TOP motif in the context of the present invention is preferably a stretch of pyrimidine nucleotides having a length of 3-30 nucleotides. Preferably, the TOP-motif consists of at least 3 pyrimidine nucleotides, preferably at least 4 pyrimidine nucleotides, preferably at least 5 pyrimidine nucleotides, more preferably at least 6 nucleotides, more preferably at least 7 nucleotides, most preferably at least 8 pyrimidine nucleotides, wherein the stretch of pyrimidine nucleotides preferably starts at its 5'end with a cytosine nucleotide. In TOP genes and TOP mRNAs, the TOP-motif preferably starts at its 5'end with the transcriptional start site and ends one nucleotide 5' to the first purin residue in said gene or mRNA. A TOP motif in the sense of the present invention is preferably located at the 5'end of a sequence which represents a 5'UTR or at the 5'end of a sequence which codes for a 5'UTR. Thus, preferably, a stretch of 3 or more pyrimidine nucleotides is called "TOP motif" in the sense of the present invention if this stretch is located at the 5'end of a respective sequence, such as the inventive mRNA, the 5'UTR element of the inventive mRNA, or the nucleic acid sequence which is derived from the 5'UTR of a TOP gene as described herein. In other words, a stretch of 3 or more pyrimidine nucleotides which is not located at the 5'-end of a 5'UTR or a 5'UTR element but anywhere within a 5'UTR or a 5'UTR element is preferably not referred to as "TOP motif".

TOP gene: TOP genes are typically characterised by the presence of a 5' terminal oligopyrimidine tract. Furthermore, most TOP genes are characterized by a growth-associated translational regulation. However, also TOP genes with a tissue specific translational regulation are known. As defined above, the 5'UTR of a TOP gene corresponds to the sequence of a 5'UTR of a mature mRNA derived from a TOP gene, which preferably extends from the nucleotide located 3' to the 5'-CAP to the nucleotide located 5' to the start codon. A 5'UTR of a TOP gene typically does not comprise any start codons, preferably no upstream AUGs (uAUGs) or upstream open reading frames (uORFs). Therein, upstream AUGs and upstream open reading frames are typically understood to be AUGs and open reading frames that occur 5' of the start codon (AUG) of the open reading frame that should be translated. The 5'UTRs of TOP genes are generally rather short. The lengths of 5'UTRs of TOP genes may vary between 20 nucleotides up to 500 nucleotides, and are typically less than about 200 nucleotides, preferably less than about 150 nucleotides, more preferably less than about 100 nucleotides. Exemplary 5'UTRs of TOP genes in the sense of the present invention are the nucleic acid sequences extending from the nucleotide at position 5 to the nucleotide located immediately 5' to the start codon (e.g. the ATG) in the sequences according to SEQ ID Nos. 1-1363, SEQ ID NO. 1395, SEQ ID NO. 1421 and SEQ ID NO. 14221-1363 of the patent application PCT/EP2012/002448WO2013/143700 or homologs or variants thereof, whose disclosure is incorporated herewith by reference. In this context a particularly preferred fragment of a 5'UTR of a TOP gene is a 5'UTR of a TOP gene lacking the 5'TOP motif. The term '5'UTR of a TOP gene' preferably refers to the 5'UTR of a naturally occurring TOP gene.

Fragment of a nucleic acid sequence, particularly an mRNA: A fragment of a nucleic acid sequence consists of a continuous stretch of nucleotides corresponding to a continuous stretch of nucleotides in the full-length nucleic acid sequence which is the basis for the nucleic acid sequence of the fragment, which represents at least 20%, preferably at least 30%, more preferably at least 40%, more preferably at least 50%, even more preferably at least 60%, even more preferably at least 70%, even more preferably at least 80%, and most preferably at least 90% of the full-length nucleic acid sequence. Such a fragment, in the sense of the present invention, is preferably a functional fragment of the full-length nucleic acid sequence.

Variant of a nucleic acid sequence, particularly an mRNA: A variant of a nucleic acid sequence refers to a variant of nucleic acid sequences which forms the basis of a nucleic acid sequence. For example, a variant nucleic acid sequence may exhibit one or more nucleotide deletions, insertions, additions and/or substitutions compared to the nucleic acid sequence from which the variant is derived. Preferably, a variant of a nucleic acid sequence is at least 40%, preferably at least 50%, more preferably at least 60%, more preferably at least 70%, even more preferably at least 80%, even more preferably at least 90%, most preferably at least 95% identical to the nucleic acid sequence the variant is derived from. Preferably, the variant is a functional variant. A "variant" of a nucleic acid sequence may have at least 70%, 75%, 80%, 85%, 90%, 95%, 98% or 99% nucleotide identity over a stretch of 10, 20, 30, 50, 75 or 100 nucleotide of such nucleic acid sequence.

Homolog of a nucleic acid sequence: The term "homolog" of a nucleic acid sequence refers to sequences of other species than the particular sequence. It is particular preferred that the nucleic acid sequence is of human origin and therefore it is preferred that the homolog is a homolog of a human nucleic acid sequence.

Jet injection: The term "jet injection", as used herein, refers to a needle-free injection method, wherein a fluid containing at least one inventive mRNA sequence and, optionally, further suitable excipients is forced through an orifice, thus generating an ultra-fine liquid stream of high pressure that is capable of penetrating mammalian skin and, depending on the injection settings, subcutaneous tissue or muscle tissue. In principle, the liquid stream forms a hole in the skin, through which the liquid stream is pushed into the target tissue. Preferably, jet injection is used for intradermal, subcutaneous or intramuscular injection of the mRNA sequence according to the invention. In a preferred embodiment, jet injection is used for intramuscular injection of the inventive mRNA. In a further preferred embodiment, jet injection is used for intradermal injection of the inventive mRNA.

The present invention is based on the surprising finding of the present inventors that an mRNA sequence comprising a coding region, encoding a mutant of fusion protein F of Respiratory syncytial virus (RSV), wherein the amino acid residues 554-574 of the amino acid sequence of the fusion protein F are deleted in the mutant of fusion protein F, wherein the G/C content of the coding region is increased by substituting at least 70% of the substitutable codons in the coding region compared with the G/C content of the coding region of the wild type mRNA, and wherein the coded amino acid sequence of said GC-enriched mRNA is not being modified compared with the coded amino acid sequence of the wild type mRNA, induces antigen-specific immune responses and therefore prevent or at least minimize Respiratory syncytial virus (RSV) infections. It was very surprising for the inventors that the inventive mRNA sequence induces at least the same immune responses than vaccines based on inactivated RSV which consists of the whole virus. Even more surprisingly the inventive mRNA sequence as defined by the claims induced antigen-specific CD8+-T cells in contrast to a vaccine based on an inactivated RSV. Additionally, in a cotton rat RSV challenge model, the virus titers in the nose and in the lung of mRNA vaccinated animals were much lower compared to animals vaccinated with vaccines based on an inactivated RSV virus. With regard to safety the inventors could show that the mRNA-based RSV vaccine showed no hints for vaccine-mediated disease enhancement, in terms of lung pathology, compared to a vaccine based on formalin-inactivated virus. Furthermore, it has surprisingly been found by the inventors that already one single vaccination with the inventive mRNA sequence was sufficient for eliciting an immune response against the administered antigen(s). Specifically, it has been found that one single administration, preferably by intradermal or intramuscular injection, of the inventive mRNA is highly efficient in reducing viral titers in the lung after challenge with RSV virus.

In summary, the inventive mRNA sequence defined by the attached claims could provide an effective and safe vaccine, particularly for infants, the elderly and immunocompromised patients.

Also disclosed herein is an mRNA sequence that comprises a coding region, encoding at least one antigenic peptide or protein derived from the fusion protein F, the glycoprotein G, the short hydrophobic protein SH, the matrix protein M, the nucleoprotein N, the large polymerase L, the M2-1 protein, the M2-2 protein, the phosphoprotein P, the non-structural protein NS1 or the non-structural protein NS2 of Respiratory syncytial virus (RSV) or a fragment, variant or derivative thereof.

The coding region of the mRNA sequence according to the first aspect of the present disclosure may occur as a mono-, bi-, or even multicistronic mRNA, i.e. an mRNA sequence which carries the coding sequences of one, two or more proteins or peptides. Such coding sequences in bi-, or even multicistronic mRNAs may be separated by at least one internal ribosome entry site (IRES) sequence, e.g. as described herein or by signal peptides which induce the cleavage of the resulting polypeptide which comprises several proteins or peptides.

According to the first aspect of the present disclosure, the mRNA sequence comprises a coding region, encoding at least one antigenic peptide or protein derived from the fusion protein F, the glycoprotein G, the short hydrophobic protein SH, the matrix protein M, the nucleoprotein N, the large polymerase L, the M2-1 protein, the M2-2 protein, the phosphoprotein P, the non-structural protein NS1 or the non-structural protein NS2 of Respiratory syncytial virus (RSV) or a fragment, variant or derivative thereof. In an embodiment disclosed herein, the mRNA sequence comprises a coding region, encoding at least one antigenic peptide or protein derived from the fusion protein F, the nucleoprotein N, or the M2-1 protein of Respiratory syncytial virus (RSV) or a fragment, variant or derivative thereof.

In this context, the amino acid sequence of the at least one antigenic peptide or protein may be selected from any peptide or protein derived from the fusion protein F, the glycoprotein G, the short hydrophobic protein SH, the matrix protein M, the nucleoprotein N, the large polymerase L, the M2-1 protein, the M2-2 protein, the phosphoprotein P, the non-structural protein NS1 or the non-structural protein NS2 of any RSV isolate or from any synthetically engineered RSV peptide or protein or from a fragment, variant or derivative thereof.
In a non-claimed embodiment, the full-length protein of the fusion protein F, the glycoprotein G, the short hydrophobic protein SH, the matrix protein M, the nucleoprotein N, the large polymerase L, the M2-1 protein, the M2-2 protein, the phosphoprotein P, the non-structural protein NS1 or the non-structural protein NS2 of Respiratory syncytial virus (RSV) is encoded by the coding region comprised in the inventive mRNA.
In this non-claimed embodiment, the full-length protein from the fusion protein F and the nucleoprotein N are particularly preferred. Furthermore a mutant of the F protein with a deletion of the cytoplasmic tail is particularly preferred. An example of such a deletion mutant is the RSV-Fdel 554-574 long protein according to (Oomens et a/. 2006. J. Virol. 80(21):10465-77).

In a further embodiment disclosed herein, a fragment comprising at least one epitope of the fusion protein F, the glycoprotein G, the short hydrophobic protein SH, the matrix protein M, the nucleoprotein N, the large polymerase L, the M2-1 protein, the M2-2 protein, the phosphoprotein P, the non-structural protein NS1 or the non-structural protein NS2 of Respiratory syncytial virus (RSV) is encoded by the coding region comprised in the mRNA.

Disclosed herein are the amino acid sequences of the RSV strain long (ATCC VR-26) according to the NCBI accession No. AY911262:
**Fusion protein F of the RSV strain ATCC VR-26 long:**
   Amino acid sequence according to SEQ ID No.1:
**Glycoprotein G of the RSV strain ATCC VR-26 long:**
   Amino acid sequence according to SEQ ID No.2:
**Short hydrophobic protein SH of the RSV strain ATCC VR-26 long:**
   Amino acid sequence according to SEQ ID No.3:
**Matrix protein M of the RSV strain ATCC VR-26 long:**
   Amino acid sequence according to SEQ ID No.4:
**Nucleoprotein N of the RSV strain ATCC VR-26 long:**
   **Amino acid sequence according to SEQ ID No.5:**
**Large polymerase L of the RSV strain ATCC VR-26 long:**
   Amino acid sequence according to SEQ ID No.6:
**M2-1 protein of the RSV strain ATCC VR-26 long:**
   Amino acid sequence according to SEQ ID No.7:
**M2-2 protein of the RSV strain ATCC VR-26 long:**
   Amino acid sequence according to SEQ ID No.8:
**Phosphoprotein P of the RSV strain ATCC VR-26 long:**
   Amino acid sequence according to SEQ ID No.9:
**Non-structural protein NS1 of the RSV strain ATCC VR-26 long:**
   Amino acid sequence according to SEQ ID No.10:
**Non-structural protein NS2 of the RSV strain ATCC VR-26 long:**
   Amino acid sequence according to SEQ ID No.11:

In the context of the present disclosure, additionally to the here disclosed amino acid sequences according to SEQ ID Nos. 1-11, also amino acid sequences of different Respiratory syncytial virus (RSV) isolates can be used. These Respiratory syncytial virus (RSV) isolates show preferably an identity of at least 70%, more preferably of at least 80% and most preferably of at least 90% with the amino acid sequences according to SEQ ID Nos. 1-11.

Furthermore, in this context the coding region encoding at least one antigenic peptide or protein derived from the fusion protein F, the glycoprotein G, the short hydrophobic protein SH, the matrix protein M, the nucleoprotein N, the large polymerase L, the M2-1 protein, the M2-2 protein, the phosphoprotein P, the non-structural protein NS1 or the non-structural protein NS2 of Respiratory syncytial virus (RSV) or a fragment, variant or derivative thereof, may be selected from any nucleic acid sequence comprising a coding region derived from any Respiratory syncytial virus (RSV) isolate or a fragment or variant thereof.

Disclosed herein are the wild type mRNA sequences of the coding regions of the RSV strain long (ATCC VR-26) according to the NCBI accession No. AY911262:
**mRNA coding for the fusion protein F of the RSV strain ATCC VR-26 long:**
   mRNA sequence according to SEQ ID No.12:
mRNA coding for the glycoprotein G of the RSV strain ATCC VR-26 long:
   mRNA sequence according to SEQ ID No.13:
mRNA coding for the Short hydrophobic protein SH of the RSV strain ATCC VR-26 long:
   mRNA sequence according to SEQ ID No.14:
mRNA coding for the matrixprotein M of the RSV strain ATCC VR-26 long:
   mRNA sequence according to SEQ ID No.15:
mRNA coding for the nucleoprotein N of the RSV strain ATCC VR-26 long:
   mRNA sequence according to SEQ ID No.16:
mRNA coding for the Large polymerase L of the RSV strain ATCC VR-26 long:
   mRNA sequence according to SEQ ID No.17:
mRNA coding for the protein M2-1 of the RSV strain ATCC VR-26 long:
   mRNA sequence according to SEQ ID No.18:
mRNA coding for the protein M2-2 of the RSV strain ATCC VR-26 long:
   mRNA sequence according to SEQ ID No.19:
mRNA coding for the phosphoprotein P of the RSV strain ATCC VR-26 long:
   mRNA sequence according to SEQ ID No.20:
mRNA coding for the non-structural protein NS1 of the RSV strain ATCC VR-26 long:
   mRNA sequence according to SEQ ID No.21:
mRNA coding for the non-structural protein NS2 of the RSV strain ATCC VR-26 long:
   mRNA sequence according to SEQ ID No.22:

In the context of the present disclosure, additionally to the here disclosed nucleic acid sequences, also nucleic acid sequences of different Respiratory syncytial virus (RSV) isolates are incorporated herewith. These different Respiratory syncytial virus (RSV) isolates show preferably an identity of at least 50%, 60%, 70%, more preferably of at least 80% and most preferably of at least 90% with the nucleic acid sequences according to SEQ ID Nos. 12-22 or of fragments thereof.

In a preferred embodiment, the mRNA sequence according to the invention does not comprise a reporter gene or a marker gene. Preferably, the mRNA sequence according to the invention does not encode, for instance, luciferase; green fluorescent protein (GFP) and its variants (such as eGFP, RFP or BFP); α-globin; hypoxanthine-guanine phosphoribosyltransferase (HGPRT); β-galactosidase; galactokinase; alkaline phosphatase; secreted embryonic alkaline phosphatase (SEAP)) or a resistance gene (such as a resistance gene against neomycin, puromycin, hygromycin and zeocin). In a preferred embodiment, the mRNA sequence according to the invention does not encode luciferase. In another embodiment, the mRNA sequence according to the invention does not encode GFP or a variant thereof.

In a further preferred embodiment, the mRNA sequence according to the invention does not encode a protein (or a fragment of a protein) derived from a virus belonging to the family of Orthomyxoviridae. Preferably the mRNA sequence does not encode a protein that is derived from an influenza virus, more preferably an influenza A virus. Preferably, the mRNA sequence according to the invention does not encode an influenza A protein selected from the group consisting of hemagglutinin (HA), neuraminidase (NA), nucleoprotein (NP), M1, M2, NS1, NS2 (NEP: nuclear export protein), PA, PB1 (polymerase basic 1), PB1-F2 and PB2. In another preferred embodiment, the mRNA according to the invention does not encode ovalbumin (OVA) or a fragment thereof. Preferably, the mRNA sequence according to the invention does not encode an influenza A protein or ovalbumin.

By a further embodiment, the inventive mRNA preferably comprises at least one of the following structural elements: a 5'- and/or 3'- untranslated region element (UTR element), particularly a 5'-UTR element which comprises or consists of a nucleic acid sequence which is derived from the 5'-UTR of a TOP gene or from a fragment, homolog or a variant thereof, or a 5'- and/or 3'-UTR element which may be derivable from a gene that provides a stable mRNA or from a homolog, fragment or variant thereof; a histone-stem-loop structure, preferably a histone-stem-loop in its 3' untranslated region; a 5'-CAP structure; a poly-A tail; or a poly(C) sequence.

In a preferred embodiment of the first aspect of the present invention, the inventive mRNA comprises at least one 5'- or 3'-UTR element. In this context, an UTR element comprises or consists of a nucleic acid sequence which is derived from the 5'- or 3'-UTR of any naturally occurring gene or which is derived from a fragment, a homolog or a variant of the 5'- or 3'-UTR of a gene. Preferably, the 5'- or 3'-UTR element used according to the present invention is heterologous to the coding region of the inventive mRNA sequence. Even if 5'- or 3'-UTR elements derived from naturally occurring genes are preferred, also synthetically engineered UTR elements may be used in the context of the present invention.

In a particularly preferred embodiment of the first aspect of the present invention, the inventive mRNA sequence comprises at least one 5'-untranslated region element (5'UTR element) which comprises or consists of a nucleic acid sequence which is derived from the 5'UTR of a TOP gene or which is derived from a fragment, homolog or variant of the 5'UTR of a TOP gene.

It is particularly preferred that the 5'UTR element does not comprise a TOP-motif or a 5'TOP, as defined above.

In some embodiments, the nucleic acid sequence of the 5'UTR element which is derived from a 5'UTR of a TOP gene terminates at its 3'-end with a nucleotide located at position 1, 2, 3, 4, 5, 6, 7, 8, 9 or 10 upstream of the start codon (e.g. A(U/T)G) of the gene or mRNA it is derived from. Thus, the 5'UTR element does not comprise any part of the protein coding region. Thus, preferably, the only protein coding part of the inventive mRNA is provided by the coding region.

The nucleic acid sequence, which is derived from the 5'UTR of a TOP gene, is derived from a eukaryotic TOP gene, preferably a plant or animal TOP gene, more preferably a chordate TOP gene, even more preferably a vertebrate TOP gene, most preferably a mammalian TOP gene, such as a human TOP gene.

For example, the 5'UTR element is prefereably selected from 5'-UTR elements comprising or consisting of a nucleic acid sequence, which is derived from a nucleic acid sequence selected from the group consisting of SEQ ID Nos. 1-1363, SEQ ID NO. 1395, SEQ ID NO. 1421 and SEQ ID NO. 1422 of the patent application WO2013/143700, from the homologs of SEQ ID Nos. 1-1363, SEQ ID NO. 1395, SEQ ID NO. 1421 and SEQ ID NO. 1422 of the patent application WO2013/143700, from a variant thereof, or preferably from a corresponding RNA sequence. The term "homologs of SEQ ID Nos. 1-1363, SEQ ID NO. 1395, SEQ ID NO. 1421 and SEQ ID NO. 1422 of the patent application WO2013/143700" refers to sequences of other species than homo sapiens, which are homologous to the sequences according to SEQ ID Nos. 1-1363, SEQ ID NO. 1395, SEQ ID NO. 1421 and SEQ ID NO. 1422 of the patent application WO2013/143700.

In a preferred embodiment, the 5'UTR element comprises or consists of a nucleic acid sequence which is derived from a nucleic acid sequence extending from nucleotide position 5 (i.e. the nucleotide that is located at position 5 in the sequence) to the nucleotide position immediately 5' to the start codon (located at the 3' end of the sequences), e.g. the nucleotide position immediately 5' to the ATG sequence, of a nucleic acid sequence selected from SEQ ID Nos. 1-1363, SEQ ID NO. 1395, SEQ ID NO. 1421 and SEQ ID NO. 1422 of the patent application WO2013/143700, from the homologs of SEQ ID Nos. 1-1363, SEQ ID NO. 1395, SEQ ID NO. 1421 and SEQ ID NO. 1422 of the patent application WO2013/143700, from a variant thereof, or a corresponding RNA sequence. It is particularly preferred that the 5' UTR element is derived from a nucleic acid sequence extending from the nucleotide position immediately 3' to the 5'TOP to the nucleotide position immediately 5' to the start codon (located at the 3' end of the sequences), e.g. the nucleotide position immediately 5' to the ATG sequence, of a nucleic acid sequence selected from SEQ ID Nos. 1-1363, SEQ ID NO. 1395, SEQ ID NO. 1421 and SEQ ID NO. 1422 of the patent application WO2013/143700, from the homologs of SEQ ID Nos. 1-1363, SEQ ID NO. 1395, SEQ ID NO. 1421 and SEQ ID NO. 1422 of the patent application WO2013/143700, from a variant thereof, or a corresponding RNA sequence.

In a particularly preferred embodiment, the 5'UTR element comprises or consists of a nucleic acid sequence, which is derived from a 5'UTR of a TOP gene encoding a ribosomal protein or from a variant of a 5'UTR of a TOP gene encoding a ribosomal protein. For example, the 5'UTR element comprises or consists of a nucleic acid sequence which is derived from a 5'UTR of a nucleic acid sequence according to any of SEQ ID NOs: 67, 170, 193, 244, 259, 554, 650, 675, 700, 721, 913, 1016, 1063, 1120, 1138, and 1284-1360 of the patent application WO2013/143700, a corresponding RNA sequence, a homolog thereof, or a variant thereof as described herein, preferably lacking the 5'TOP motif. As described above, the sequence extending from position 5 to the nucleotide immediately 5' to the ATG (which is located at the 3'end of the sequences) corresponds to the 5'UTR of said sequences.

Preferably, the 5'UTR element comprises or consists of a nucleic acid sequence which is derived from a 5'UTR of a TOP gene encoding a ribosomal Large protein (RPL) or from a homolog or variant of a 5'UTR of a TOP gene encoding a ribosomal Large protein (RPL). For example, the 5'UTR element comprises or consists of a nucleic acid sequence which is derived from a 5'UTR of a nucleic acid sequence according to any of SEQ ID NOs: 67, 259, 1284-1318, 1344, 1346, 1348-1354, 1357, 1358, 1421 and 1422 of the patent application WO2013/143700, a corresponding RNA sequence, a homolog thereof, or a variant thereof as described herein, preferably lacking the 5'TOP motif.

In a particularly preferred embodiment, the 5'UTR element comprises or consists of a nucleic acid sequence which is derived from the 5'UTR of a ribosomal protein Large 32 gene, preferably from a vertebrate ribosomal protein Large 32 (L32) gene, more preferably from a mammalian ribosomal protein Large 32 (L32) gene, most preferably from a human ribosomal protein Large 32 (L32) gene, or from a variant of the 5'UTR of a ribosomal protein Large 32 gene, preferably from a vertebrate ribosomal protein Large 32 (L32) gene, more preferably from a mammalian ribosomal protein Large 32 (L32) gene, most preferably from a human ribosomal protein Large 32 (L32) gene, wherein preferably the 5'UTR element does not comprise the 5'TOP of said gene.

Accordingly, in a particularly preferred embodiment, the 5'UTR element comprises or consists of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to the nucleic acid sequence according to SEQ ID No. 23 (5'-UTR of human ribosomal protein Large 32 lacking the 5' terminal oligopyrimidine tract: GGCGCTGCCTACGGAGGTGGCAGCCATCTCCTTCTCGGCATC; corresponding to SEQ ID No. 1368 of the patent application WO2013/143700) or preferably to a corresponding RNA sequence, or wherein the at least one 5'UTR element comprises or consists of a fragment of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to the nucleic acid sequence according to SEQ ID No. 23 or more preferably to a corresponding RNA sequence, wherein, preferably, the fragment is as described above, i.e. being a continuous stretch of nucleotides representing at least 20%, preferably at least 30%, more preferably at least 40%, more preferably at least 50%, even more preferably at least 60%, even more preferably at least 70%, even more preferably at least 80%, and most preferably at least 90% . of the full-length 5'UTR. Preferably, the fragment exhibits a length of at least about 20 nucleotides or more, preferably of at least about 30 nucleotides or more, more preferably of at least about 40 nucleotides or more. Preferably, the fragment is a functional fragment as described herein.

In some embodiments, the inventive mRNA comprises a 5'UTR element which comprises or consists of a nucleic acid sequence which is derived from the 5'UTR of a vertebrate TOP gene, such as a mammalian, e.g. a human TOP gene, selected from RPSA, RPS2, RPS3, RPS3A, RPS4, RPS5, RPS6, RPS7, RPS8, RPS9, RPS10, RPS11, RPS12, RPS13, RPS14, RPS15, RPS15A, RPS16, RPS17, RPS18, RPS19, RPS20, RPS21, RPS23, RPS24, RPS25, RPS26, RPS27, RPS27A, RPS28, RPS29, RPS30, RPL3, RPL4, RPL5, RPL6, RPL7, RPL7A, RPL8, RPL9, RPL10, RPL10A, RPL11, RPL12, RPL13, RPL13A, RPL14, RPL15, RPL17, RPL18, RPL18A, RPL19, RPL21, RPL22, RPL23, RPL23A, RPL24, RPL26, RPL27, RPL27A, RPL28, RPL29, RPL30, RPL31, RPL32, RPL34, RPL35, RPL35A, RPL36, RPL36A, RPL37, RPL37A, RPL38, RPL39, RPL40, RPL41, RPLP0, RPLP1, RPLP2, RPLP3, RPLPO, RPLP1, RPLP2, EEF1A1, EEF1B2, EEF1D, EEF1G, EEF2, EIF3E, EIF3F, EIF3H, EIF2S3, EIF3C, EIF3K, EIF3EIP, EIF4A2, PABPC1, HNRNPA1, TPT1, TUBB1, UBA52, NPM1, ATP5G2, GNB2L1, NME2, UQCRB, or from a homolog or variant thereof, wherein preferably the 5'UTR element does not comprise a TOP-motif or the 5'TOP of said genes, and wherein optionally the 5'UTR element starts at its 5'-end with a nucleotide located at position 1, 2, 3, 4, 5, 6, 7, 8, 9 or 10 downstream of the 5'terminal oligopyrimidine tract (TOP) and wherein further optionally the 5'UTR element which is derived from a 5'UTR of a TOP gene terminates at its 3'-end with a nucleotide located at position 1, 2, 3, 4, 5, 6, 7, 8, 9 or 10 upstream of the start codon (A(U/T)G) of the gene it is derived from.

In further particularly preferred embodiments, the 5'UTR element comprises or consists of a nucleic acid sequence which is derived from the 5'UTR of a ribosomal protein Large 32 gene (RPL32), a ribosomal protein Large 35 gene (RPL35), a ribosomal protein Large 21 gene (RPL21), an ATP synthase, H+ transporting, mitochondrial F1 complex, alpha subunit 1, cardiac muscle (ATP5A1) gene, an hydroxysteroid (17-beta) dehydrogenase 4 gene (HSD17B4), an androgen-induced 1 gene (AIG1), cytochrome c oxidase subunit VIc gene (COX6C), or a N-acylsphingosine amidohydrolase (acid ceramidase) 1 gene (ASAH1) or from a variant thereof, preferably from a vertebrate ribosomal protein Large 32 gene (RPL32), a vertebrate ribosomal protein Large 35 gene (RPL35), a vertebrate ribosomal protein Large 21 gene (RPL21), a vertebrate ATP synthase, H+ transporting, mitochondrial F1 complex, alpha subunit 1, cardiac muscle (ATP5A1) gene, a vertebrate hydroxysteroid (17-beta) dehydrogenase 4 gene (HSD17B4), a vertebrate androgen-induced 1 gene (AIG1), a vertebrate cytochrome c oxidase subunit VIc gene (COX6C), or a vertebrate N-acylsphingosine amidohydrolase (acid ceramidase) 1 gene (ASAH1) or from a variant thereof, more preferably from a mammalian ribosomal protein Large 32 gene (RPL32), a ribosomal protein Large 35 gene (RPL35), a ribosomal protein Large 21 gene (RPL21), a mammalian ATP synthase, H+ transporting, mitochondrial F1 complex, alpha subunit 1, cardiac muscle (ATP5A1) gene, a mammalian hydroxysteroid (17-beta) dehydrogenase 4 gene (HSD17B4), a mammalian androgen-induced 1 gene (AIG1), a mammalian cyto-chrome c oxidase subunit VIc gene (COX6C), or a mammalian N-acylsphingosine ami-dohydrolase (acid ceramidase) 1 gene (ASAH1) or from a variant thereof, most preferably from a human ribosomal protein Large 32 gene (RPL32), a human ribosomal protein Large 35 gene (RPL35), a human ribosomal protein Large 21 gene (RPL21), a human ATP syn-thase, H+ transporting, mitochondrial F1 complex, alpha subunit 1, cardiac muscle (ATP5A1) gene, a human hydroxysteroid (17-beta) dehydrogenase 4 gene (HSD17B4), a human androgen-induced 1 gene (AIG1), a human cytochrome c oxidase subunit VIc gene (COX6C), or a human N-acylsphingosine amidohydrolase (acid ceramidase) 1 gene (ASAH1) or from a variant thereof, wherein preferably the 5'UTR element does not comprise the 5'TOP of said gene.

Accordingly, in a particularly preferred embodiment, the 5'UTR element comprises or consists of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to the nucleic acid sequence according to SEQ ID No. 1368, or SEQ ID NOs 1412-1420 of the patent application WO2013/143700, or a corresponding RNA sequence, or wherein the at least one 5'UTR element comprises or consists of a fragment of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to the nucleic acid sequence according to SEQ ID No. 1368, or SEQ ID NOs 1412-1420 of the patent application WO2013/143700, wherein, preferably, the fragment is as described above, i.e. being a continuous stretch of nucleotides representing at least 20% etc. of the full-length 5'UTR. Preferably, the fragment exhibits a length of at least about 20 nucleotides or more, preferably of at least about 30 nucleotides or more, more preferably of at least about 40 nucleotides or more. Preferably, the fragment is a functional fragment as described herein.

Accordingly, in a particularly preferred embodiment, the 5'UTR element comprises or consists of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to the nucleic acid sequence according to SEQ ID No. 36 (5'-UTR of ATP5A1 lacking the 5' terminal oligopyrimidine tract: GCGGCTCGGCCATTTTGTCCCAGTCAGTCCGGAGGCTGCGGCTGCAGAAGTACCGCCT GCG-GAGTAACTGCAAAG; corresponding to SEQ ID No. 1414 of the patent application WO2013/143700) or preferably to a corresponding RNA sequence, or wherein the at least one 5'UTR element comprises or consists of a fragment of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to the nucleic acid sequence according to SEQ ID No. 26 or more preferably to a corresponding RNA sequence, wherein, preferably, the fragment is as described above, i.e. being a continuous stretch of nucleotides representing at least 20% etc. of the full-length 5'UTR. Preferably, the fragment exhibits a length of at least about 20 nucleotides or more, preferably of at least about 30 nucleotides or more, more preferably of at least about 40 nucleotides or more. Preferably, the fragment is a functional fragment as described herein.

In a further preferred embodiment, the inventive mRNA further comprises at least one 3'UTR element, which comprises or consists of a nucleic acid sequence derived from the 3'UTR of a chordate gene, preferably a vertebrate gene, more preferably a mammalian gene, most preferably a human gene, or from a variant of the 3'UTR of a chordate gene, preferably a vertebrate gene, more preferably a mammalian gene, most preferably a human gene.

The term '3'UTR element' refers to a nucleic acid sequence which comprises or consists of a nucleic acid sequence that is derived from a 3'UTR or from a variant of a 3'UTR. A 3'UTR element in the sense of the present invention may represent the 3'UTR of an mRNA. Thus, in the sense of the present invention, preferably, a 3'UTR element may be the 3'UTR of an mRNA, preferably of an artificial mRNA, or it may be the transcription template for a 3'UTR of an mRNA. Thus, a 3'UTR element preferably is a nucleic acid sequence which corresponds to the 3'UTR of an mRNA, preferably to the 3'UTR of an artificial mRNA, such as an mRNA obtained by transcription of a genetically engineered vector construct. Preferably, the 3'UTR element fulfils the function of a 3'UTR or encodes a sequence which fulfils the function of a 3'UTR.

Preferably, the inventive mRNA comprises a 3'UTR element which may be derivable from a gene that relates to an mRNA with an enhanced half-life (that provides a stable mRNA), for example a 3'UTR element as defined and described below.

In a particularly preferred embodiment, the 3'UTR element comprises or consists of a nucleic acid sequence which is derived from a 3'UTR of a gene selected from the group consisting of an albumin gene, an α-globin gene, a β-globin gene, a tyrosine hydroxylase gene, a lipoxygenase gene, and a collagen alpha gene, such as a collagen alpha 1(I) gene, or from a variant of a 3'UTR of a gene selected from the group consisting of an albumin gene, an α-globin gene, a β-globin gene, a tyrosine hydroxylase gene, a lipoxygenase gene, and a collagen alpha gene, such as a collagen alpha 1(I) gene according to SEQ ID No. 1369-1390 of the patent application WO2013/143700. In a particularly preferred embodiment, the 3'UTR element comprises or consists of a nucleic acid sequence which is derived from a 3'UTR of an albumin gene, preferably a vertebrate albumin gene, more preferably a mammalian albumin gene, most preferably a human albumin gene according to SEQ ID No. 24.
Human albumin 3'UTR SEQ ID No. 24:
CATCACATTT AAAAGCATCT CAGCCTACCA TGAGAATAAG AGAAAGAAAA TGAAGATCAA AAGCTTATTC ATCTGTTTTT CTTTTTCGTT GGTGTAAAGC CAACACCCTG TCTAAAAAAC ATAAATTTCT TTAATCATTT TGCCTCTTTT CTCTGTGCTT CAATTAATAA AAAATGGAAA GAATCT (corresponding to SEQ ID No: 1369 of the patent application WO2013/143700).

In this context, it is particularly preferred that the inventive mRNA comprises a 3'-UTR element comprising a corresponding RNA sequence derived from the nucleic acids according to SEQ ID No. 1369-1390 of the patent application WO2013/143700 or a fragment, homolog or variant thereof.

Most preferably the 3'-UTR element comprises the nucleic acid sequence derived from a fragment of the human albumin gene according to SEQ ID No. 25:
albumin7 3'UTR
CATCACATTTAAAAGCATCTCAGCCTACCATGAGAATAAGAGAAAGAAAATGAAGATCAA TAGCTTATTCATCTCTTTTTCTTTTTCGTTGGTGTAAAGCCAACACCCTGTCTAAAAAACAT AAATTTCTTTAATCATTTTGCCTCTTTTCTCTGTGCTTCAATTAATAAAAAATGGAAAGAACC T (SEQ ID No. 25 corresponding to SEQ ID No: 1376 of the patent application WO2013/143700)
In this context, it is particularly preferred that the 3'-UTR element of the inventive mRNA comprises or consists of a corresponding RNA sequence of the nucleic acid sequence according to SEQ ID No. 25.

In another particularly preferred embodiment, the 3'UTR element comprises or consists of a nucleic acid sequence which is derived from a 3'UTR of an α-globin gene, preferably a vertebrate α-or β-globin gene, more preferably a mammalian α-or β-globin gene, most preferably a human α-or β-gtobin gene according to SEQ ID No. 26-28:
3'-UTR of Homo sapiens hemoglobin, alpha 1 (HBA1)
   GCTGGAGCCTCGGTGGCCATGCTTCTTGCCCCTTGGGCCTCCCCCCAGCCCCTCCTCCC CTTCCTGCACCCGTACCCCCGTGGTCTTTGAATAAAGTCTGAGTGGGCGGC (SEQ ID No:26 corresponding to SEQ ID No. 1370 of the patent application WO2013/143700)
3'-UTR of Homo sapiens hemoglobin, alpha 2 (HBA2)
   GCTGGAGCCTCGGTAGCCGTTCCTCCTGCCCGCTGGGCCTCCCAACGGGCCCTCCTCCC CTCCTTGCACCGGCCCTTCCTGGTCTTTGAATAAAGTCTGAGTGGGCAG (SEQ ID No: 27 corresponding to SEQ ID No. 1371 of the patent application WO2013/143700)
3'-UTR of Homo sapiens hemoglobin, beta (HBB)
   GCTCGCTTTCTTGCTGTCCAATTTCTATTAAAGGTTCCTTTGTTCCCTAAGTCCAACTACTA AACTGGGGGATATTATGAAGGGCCTTGAGCATCTGGATTCTGCCTAATAAAAAACATTTA TTTTCATTGC (SEQ ID No: 28 corresponding to SEQ ID No. 1372 of the patent application WO2013/143700)

For example, the 3'UTR element may comprise or consist of the center, α-complex-binding portion of the 3'UTR of an α-globin gene, such as of a human α-globin gene, preferably according to SEQ ID No. 29:
Center, α-complex-binding portion of the 3'UTR of an α-globin gene (also named herein as "muag")
GCCCGATGGGCCTCCCAACGGGCCCTCCTCCCCTCCTTGCACCG (SEQ ID NO. 29 corresponding to SEQ ID No. 1393 of the patent application WO2013/143700).
In this context, it is particularly preferred that the 3'-UTR element of the inventive mRNA comprises or consists of a corresponding RNA sequence of the nucleic acid sequence according to SEQ ID No. 29 or a homolog, a fragment or variant thereof.

The term 'a nucleic acid sequence which is derived from the 3'UTR of a [...] gene' preferably refers to a nucleic acid sequence which is based on the 3'UTR sequence of a [...] gene or on a part thereof, such as on the 3'UTR of an albumin gene, an α-globin gene, a β-globin gene, a tyrosine hydroxylase gene, a lipoxygenase gene, or a collagen alpha gene, such as a collagen alpha 1(I) gene, preferably of an albumin gene or on a part thereof. This term includes sequences corresponding to the entire 3'UTR sequence, i.e. the full length 3'UTR sequence of a gene, and sequences corresponding to a fragment of the 3'UTR sequence of a gene, such as an albumin gene, α-globin gene, β-globin gene, tyrosine hydroxylase gene, lipoxygenase gene, or collagen alpha gene, such as a collagen alpha 1(I) gene, preferably of an albumin gene.

The term'a nucleic acid sequence which is derived from a variant of the 3'UTR of a [...] gene' preferably refers to a nucleic acid sequence which is based on a variant of the 3'UTR sequence of a gene, such as on a variant of the 3'UTR of an albumin gene, an α-globin gene, a β-globin gene, a tyrosine hydroxylase gene, a lipoxygenase gene, or a collagen alpha gene, such as a collagen alpha 1(I) gene, or on a part thereof as described above. This term includes sequences corresponding to the entire sequence of the variant of the 3'UTR of a gene, i.e. the full length variant 3'UTR sequence of a gene, and sequences corresponding to a fragment of the variant 3'UTR sequence of a gene. A fragment in this context preferably consists of a continuous stretch of nucleotides corresponding to a continuous stretch of nucleotides in the full-length variant 3'UTR, which represents at least 20%, preferably at least 30%, more preferably at least 40%, more preferably at least 50%, even more preferably at least 60%, even more preferably at least 70%, even more preferably at least 80%, and most preferably at least 90% of the full-length variant 3'UTR. Such a fragment of a variant, in the sense of the present invention, is preferably a functional fragment of a variant as described herein.

Preferably, the at least one 5'UTR element and the at least one 3'UTR element act synergistically to increase protein production from the inventive mRNA as described above.

In a particularly preferred embodiment, the inventive mRNA as defined by the attached claims comprises a histone stem-loop sequence/structure. Such histone stem-loop sequences are preferably selected from histone stem-loop sequences as disclosed in WO 2012/019780.

A histone stem-loop sequence, suitable to be used within the present invention, is preferably selected from at least one of the following formulae (I) or (II):
formula (I) (stem-loop sequence without stem bordering elements):
formula (II) (stem-loop sequence with stem bordering elements):
wherein:
- stem1 or stem2 bordering elements N₁₋₆: is a consecutive sequence of 1 to 6, preferably of 2 to 6, more preferably of 2 to 5, even more preferably of 3 to 5, most preferably of 4 to 5 or 5 N, wherein each N is independently from another selected from a nucleotide selected from A, U, T, G and C, or a nucleotide analogue thereof;
- stem1 [N₀₋₂GN₃₋₅]: is reverse complementary or partially reverse complementary with element stem2, and is a consecutive sequence between of 5 to 7 nucleotides;
wherein N₀₋₂ is a consecutive sequence of 0 to 2, preferably of 0 to 1, more preferably of 1 N, wherein each N is independently from another selected from a nucleotide selected from A, U, T, G and C or a nucleotide analogue thereof;
wherein N₃₋₅ is a consecutive sequence of 3 to 5, preferably of 4 to 5, more preferably of 4 N, wherein each N is independently from another selected from a nucleotide selected from A, U, T, G and C or a nucleotide analogue thereof, and
wherein G is guanosine or an analogue thereof, and may be optionally replaced by a cytidine or an analogue thereof, provided that its complementary nucleotide cytidine in stem2 is replaced by guanosine;
- loop sequence [N₀₋₄(U/T)N₀₋₄]: is located between elements stem1 and stem2, and is a consecutive sequence of 3 to 5 nucleotides, more preferably of 4 nucleotides;
wherein each N₀₋₄ is independent from another a consecutive sequence of 0 to 4, preferably of 1 to 3, more preferably of 1 to 2 N, wherein each N is independently from another selected from a nucleotide selected from A, U, T, G and C or a nucleotide analogue thereof; and wherein U/T represents uridine, or optionally thymidine;
- stem2 [N₃₋₅CN₀₋₂]: is reverse complementary or partially reverse complementary with element stem1, and is a consecutive sequence between of 5 to 7 nucleotides;
wherein N₃₋₅ is a consecutive sequence of 3 to 5, preferably of 4 to 5, more preferably of 4 N, wherein each N is independently from another selected from a nucleotide selected from A, U, T, G and C or a nucleotide analogue thereof;
wherein N₀₋₂ is a consecutive sequence of 0 to 2, preferably of 0 to 1, more preferably of 1 N, wherein each N is independently from another selected from a nucleotide selected from A, U, T, G or C or a nucleotide analogue thereof; and
wherein C is cytidine or an analogue thereof, and may be optionally replaced by a guanosine or an analogue thereof provided that its complementary nucleoside guanosine in stem1 is replaced by cytidine;
wherein
stem1 and stem2 are capable of base pairing with each other forming a reverse complementary sequence, wherein base pairing may occur between stem1 and stem2, e.g. by Watson-Crick base pairing of nucleotides A and U/T or G and C or by non-Watson-Crick base pairing e.g. wobble base pairing, reverse Watson-Crick base pairing, Hoogsteen base pairing, reverse Hoogsteen base pairing or are capable of base pairing with each other forming a partially reverse complementary sequence, wherein an incomplete base pairing may occur between stem1 and stem2, on the basis that one ore more bases in one stem do not have a complementary base in the reverse complementary sequence of the other stem.

According to a further preferred embodiment of the first inventive aspect, the inventive mRNA sequence may comprise at least one histone stem-loop sequence according to at least one of the following specific formulae (Ia) or (IIa):
formula (Ia) (stem-loop sequence without stem bordering elements):
formula (IIa) (stem-loop sequence with stem bordering elements):
wherein:
- N, C, G, T and U: are as defined above.

According to a further more particularly preferred embodiment of the first aspect, the inventive mRNA sequence may comprise at least one histone stem-loop sequence according to at least one of the following specific formulae (Ib) or (IIb):
formula (Ib) (stem-loop sequence without stem bordering elements):
formula (IIb) (stem-loop sequence with stem bordering elements):
wherein:
- N, C, G, T and U: are as defined above.

A particular preferred histone stem-loop sequence is the sequence according to SEQ ID NO: 30 CAAAGGCTCTTTTCAGAGCCACCA or more preferably the corresponding RNA sequence of the nucleic acid sequence according to SEQ ID NO: 30 (CAAAGGCUCUUUUCAGAGCCACCA SEQ ID NO: 37).

In a particular preferred embodiment of the first aspect of the present invention the inventive mRNA comprises additionally to the coding region as defined in the attached claims, a poly(A) sequence, also called poly-A-tail, preferably at the 3'-terminus of the inventive mRNA. When present, such a poly(A) sequence comprises a sequence of about 25 to about 400 adenosine nucleotides, preferably a sequence of about 50 to about 400 adenosine nucleotides, more preferably a sequence of about 50 to about 300 adenosine nucleotides, even more preferably a sequence of about 50 to about 250 adenosine nucleotides, most preferably a sequence of about 60 to about 250 adenosine nucleotides. In this context, the term "about" refers to a deviation of ± 10% of the value(s) it is attached to. This poly(A) sequence is preferably located 3' of the coding region comprised in the inventive mRNA according to the first aspect of the present invention.

According to a further preferred embodiment, the inventive mRNA can be modified by a sequence of at least 10 cytosines, preferably at least 20 cytosines, more preferably at least 30 cytosines (so-called "poly(C) sequence"). Particularly, the mRNA may contain a poly(C) sequence of typically about 10 to 200 cytosine nucleotides, preferably about 10 to 100 cytosine nucleotides, more preferably about 10 to 70 cytosine nucleotides or even more preferably about 20 to 50 or even 20 to 30 cytosine nucleotides. This poly(C) sequence is preferably located 3' of the coding region, more preferably 3' of an optional poly(A) sequence comprised in the inventive mRNA according to the first aspect of the present invention.

In this context, the inventive mRNA sequence may comprise in a specific embodiment:
a.) a 5'-CAP structure, preferably m7GpppN;
b.) a coding region encoding a mutant of fusion protein F of Respiratory syncytial virus (RSV), wherein the amino acid residues 554-574 of the amino acid sequence of the fusion protein F are deleted in the mutant of fusion protein F, wherein the G/C content of the coding region is increased by substituting at least 70% of the substitutable codons in the coding region compared with the G/C content of the coding region of the wild type mRNA, and wherein the coded amino acid sequence of said GC-enriched mRNA is not being modified compared with the coded amino acid sequence of the wild type mRNA;
c.) a poly(A) sequence preferably comprising 64 adenosines; and
d.) optionally, a poly(C) sequence, preferably comprising 30 cytosines.

In a particularly preferred embodiment of the first aspect of the present invention the inventive mRNA comprises preferably in 5'- to 3'-direction:
a.) a 5'-CAP structure, preferably m7GpppN;
b.) a coding region encoding a mutant of fusion protein F of Respiratory syncytial virus (RSV), wherein the amino acid residues 554-574 of the amino acid sequence of the fusion protein F are deleted in the mutant of fusion protein F, wherein the G/C content of the coding region is increased by substituting at least 70% of the substitutable codons in the coding region compared with the G/C content of the coding region of the wild type mRNA, and wherein the coded amino acid sequence of said GC-enriched mRNA is not being modified compared with the coded amino acid sequence of the wild type mRNA;
c.) a poly(A) sequence preferably comprising 64 adenosines;
d.) optionally, a poly(C) sequence, preferably comprising 30 cytosines; and
e.) a histone-stem-loop, preferably comprising the corresponding RNA sequence of the nucleic acid sequence according to SEQ ID NO: 30.

In a further particularly preferred embodiment of the first aspect of the present invention, the inventive mRNA comprises preferably in 5'- to 3'-direction:
a.) a 5'-CAP structure, preferably m7GpppN;
b.) a coding region encoding encoding a mutant of fusion protein F of Respiratory syncytial virus (RSV), wherein the amino acid residues 554-574 of the amino acid sequence of the fusion protein F are deleted in the mutant of fusion protein F, wherein the G/C content of the coding region is increased by substituting at least 70% of the substitutable codons in the coding region compared with the G/C content of the coding region of the wild type mRNA, and wherein the coded amino acid sequence of said GC-enriched mRNA is not being modified compared with the coded amino acid sequence of the wild type mRNA;
c.) optionally, a 3'-UTR element derived from an alpha globin gene, preferably comprising the corresponding RNA sequence of the nucleic acid sequence according to SEQ ID NO. 29, a homolog, a fragment, or a variant thereof;
d.) a poly(A) sequence preferably comprising 64 adenosines;
e.) optionally, a poly(C) sequence, preferably comprising 30 cytosines; and
f.) a histone-stem-loop, preferably comprising the corresponding RNA sequence of the nucleic acid sequence according to SEQ ID NO: 30.

In another particular preferred embodiment, the inventive mRNA comprises preferably in 5'-to 3'-direction:
a.) a 5'-CAP structure, preferably m7GpppN;
b.) optionally, a 5'-UTR element derived from a TOP gene, preferably derived from the corresponding RNA sequence of the nucleic acid sequence according to SEQ ID NO. 23 , a homolog, a fragment, or a variant thereof;
c.) a coding region encoding encoding a mutant of fusion protein F of Respiratory syncytial virus (RSV), wherein the amino acid residues 554-574 of the amino acid sequence of the fusion protein F are deleted in the mutant of fusion protein F, wherein the G/C content of the coding region is increased by substituting at least 70% of the substitutable codons in the coding region compared with the G/C content of the coding region of the wild type mRNA, and wherein the coded amino acid sequence of said GC-enriched mRNA is not being modified compared with the coded amino acid sequence of the wild type mRNA;
d.) optionally, a 3'UTR element derived of a gene providing a stable mRNA, preferably derived from the corresponding RNA sequence of a nucleic acid sequence according to SEQ ID NO. 25, a homolog, a fragment, or a variant thereof;
e.) a poly(A) sequence preferably comprising 64 adenosines;
f.) optionally, a poly(C) sequence, preferably comprising 30 cytosines; and
g.) a histone-stem-loop, preferably comprising the corresponding RNA sequence of the nucleic acid sequence according to SEQ ID NO: 30.

According to the present disclosure, the coding region might encode at least partially one of the amino acid sequences according to SEQ ID Nos. 1-11 or fragments, variants or derivatives thereof. Furthermore, the coding region of the mRNA may encode a combination of at least two of these amino acid sequences or a combination of fragments, variants or derivatives thereof. Also disclosed in this context is a combination of fusion protein F with nucleoprotein N and a combination of fusion protein F and M2-1 protein.

Additionally the coding region might be or might comprise at least partially one of the sequences according to SEQ ID No. 12 to SEQ ID No. 22, or fragments, homologs or variants thereof. Furthermore, the mRNA might comprise a combination of at least two of these sequences or a combination of fragments, homologs or variants thereof.

For further improvement of the resistance to e.g. *in vivo* degradation (e.g. by an exo- or endonuclease), the inventive mRNA may be provided as a stabilized nucleic acid, e.g. in the form of a modified nucleic acid. According to a further embodiment of the invention it is therefore preferred that the inventive mRNA is stabilized, preferably by backbone modifications, sugar modifications and/or base modifications, more preferred stabilized by modification of the G/C-content. All of these modifications may be introduced into the inventive mRNA without impairing the mRNA's function to be translated into the antigenic function derived from the Respiratory syncytial virus (RSV) peptide or protein.

A backbone modification in the context of the present invention is preferably a modification in which phosphates of the backbone of the nucleotides contained in the inventive mRNA are chemically modified, e.g. anionic internucleoside linkage, N3'→P5' modifications, replacement of non-bridging oxygen atoms by boranes, neutral internucleoside linkage, amide linkage of the nucleosides, methylene(methylimino) linkages, formacetal and thioformacetal linkages, introduction of sulfonyl groups, or the like.

A sugar modification in the context of the present invention is preferably a chemical modification of the sugar of the nucleotides of the inventive mRNA, e.g. methylation of the ribose residue or the like.

According to invention, the mRNA is modified and thus stabilized by increasing the G (guanosine)/C (cytosine) content of the coding region of the mRNA as defined in the claims.

Therein, the G/C content of the coding region of the inventive mRNA is particularly increased compared to the G/C content of the coding region of its particular wild type coding sequence, i.e. the unmodified mRNA. However, the encoded amino acid sequence of the inventive mRNA is not modified compared to the coded amino acid sequence of the particular wild type/unmodified mRNA.

The modification of the G/C-content of the inventive mRNA is based on the fact that RNA sequences having an increased G (guanosine)/C (cytosine) content are more stable than RNA sequences having an increased A (adenosine)/U (uracil) content. The codons of a coding sequence or a whole RNA might therefore be varied compared to the wild type coding sequence or mRNA, such that they include an increased amount of G/C nucleotides while the translated amino acid sequence is retained. In respect to the fact that several codons code for one and the same amino acid (so-called degeneration of the genetic code), the most favourable codons for the stability can be determined (so-called alternative codon usage). The G/C content of the coding region of the inventive mRNA according to the invention is increased by at least at least 70 %, preferably at least 80% and most preferably at least 90%, 95% or even 100% of the substitutable codons in the region coding for a protein as defined in the claims or the whole sequence of the wild type mRNA coding sequence are substituted, thereby increasing the G/C content of said sequence. In this context, it is particularly preferable to increase the G/C content of the inventive mRNA to the maximum (i.e. 100% of the substitutable codons), in particular in the coding region, compared to the wild type sequence.

According to a further preferred embodiment of the invention, the inventive mRNA is optimized for translation, preferably optimized for translation by replacing codons for less frequent tRNAs of a given amino acid by codons for more frequently occurring tRNAs of the respective amino acid. This is based on the finding that the translation efficiency is also determined by a different frequency in the occurrence of tRNAs in cells. Thus, if so-called "less frequent codons" are present in the inventive mRNA to an increased extent, the corresponding modified RNA is translated to a significantly poorer degree than in the case where codons coding for more frequent tRNAs are present. Preferably, the coding region of the inventive mRNA is modified compared to the corresponding region of the wild type RNA or coding sequence such that at least one codon of the wild type sequence which codes for a tRNA which is relatively rare or less frequent in the cell is exchanged for a codon which codes for a tRNA which is more or most frequent in the cell and carries the same amino acid as the relatively rare or less frequent tRNA. By this modification, the sequences of the inventive mRNA can be modified such that codons for which more frequently occurring tRNAs are available are inserted. In other words, according to the invention, by this modification all codons of the wild type sequence which code for a tRNA which is relatively rare in the cell can in each case be exchanged for a codon which codes for a respective tRNA which is relatively frequent in the cell and which, in each case, carries the same amino acid as the relatively rare tRNA. Furthermore, it is particularly preferable to link the sequential G/C content which is increased, in particular maximized, in the inventive mRNA with the "frequent" codons without modifying the amino acid sequence of the protein encoded by the coding region of the inventive mRNA or of the coding region. This preferred embodiment allows provision of a particularly efficiently translated and stabilized (modified) inventive mRNA.

Substitutions, additions or eliminations of bases are preferably carried out using a DNA matrix for preparation of the nucleic acid molecule by techniques of the well known site directed mutagenesis or with an oligonucleotide ligation. In such a process, for preparation of the at least one RNA of the combination vaccine as defined herein a corresponding DNA molecule may be transcribed *in vitro.* This DNA matrix preferably comprises a suitable promoter, e.g. a T7 or SP6 promoter, for *in vitro* transcription, which is followed by the desired nucleotide sequence for the at least one RNA to be prepared and a termination signal for *in vitro* transcription. The DNA molecule, which forms the matrix of the at least one RNA of interest, may be prepared by fermentative proliferation and subsequent isolation as part of a plasmid which can be replicated in bacteria. Plasmids which may be mentioned as suitable for the present invention are e.g. the plasmids pT7Ts (GenBank accession number U26404; Lai et al., Development 1995, 121: 2349 to 2360), pGEM® series, e.g. pGEM®-1 (GenBank accession number X65300; from Promega) and pSP64 (GenBank accession number X65327); cf. also Mezei and Storts, Purification of PCR Products, in: Griffin and Griffin (ed.), PCR Technology: Current Innovation, CRC Press, Boca Raton, FL, 2001.

In a particularly preferred embodiment, the inventive mRNA sequence according to the first aspect of the present invention comprises, preferably in 5'- to 3'- direction:
a) a 5'-CAP structure, as defined herein, preferably m7GpppN;
b) a coding region encoding a mutant of fusion protein F of Respiratory syncytial virus (RSV), wherein the amino acid residues 554-574 of the amino acid sequence of the fusion protein F are deleted in the mutant of fusion protein F, wherein the G/C content of the coding region is increased by substituting at least 70% of the substitutable codons in the coding region compared with the G/C content of the coding region of the wild type mRNA, and wherein the coded amino acid sequence of said GC-enriched mRNA is not being modified compared with the coded amino acid sequence of the wild type mRNA;
c) a 3'-UTR element as defined herein, preferably derived of a gene providing a stable mRNA, most preferably the corresponding RNA sequence of the nucleic acid sequence according to SEQ ID No. 29, or a homolog, a fragment or variant thereof;
d) a poly(A) sequence, preferably consisting of 64 adenosines
e) optionally a poly(C) sequence, preferably consisting of 30 cytosines.
f) at least one histone stem-loop sequence, preferably the corresponding RNA sequence of the nucleic acid sequence according to SEQ ID NO. 30.

In a further particularly preferred embodiment, the inventive mRNA sequence according to the first aspect of the present invention comprises preferably in 5' to 3' direction:
a) a 5'-CAP structure, as defined herein, preferably preferably m7GpppN;
b) a 5'-UTR element as defined herein, preferably a 5'-UTR element which comprises or consists of a nucleic acid sequence which is derived from the 5'-UTR of a TOP gene, preferably the 5'-UTR of human ribosomal protein Large 32 lacking the 5' terminal oligopyrimidine tract according to SEQ ID No. 23 or the corresponding RNA sequence; or a fragment, homolog or variant thereof;
c) a coding region encoding a mutant of fusion protein F of Respiratory syncytial virus (RSV), wherein the amino acid residues 554-574 of the amino acid sequence of the fusion protein F are deleted in the mutant of fusion protein F, wherein the G/C content of the coding region is increased by substituting at least 70% of the substitutable codons in the coding region compared with the G/C content of the coding region of the wild type mRNA, and wherein the coded amino acid sequence of said GC-enriched mRNA is not being modified compared with the coded amino acid sequence of the wild type mRNA;
d) a 3'-UTR element, preferably the 3'-UTR element of human albumin according to SEQ ID No. 24 or the corresponding RNA, or a homolog, a fragment or a variant thereof;
e) a poly(A) sequence, preferably consisting of 64 adenosines
f) optionally a poly(C) sequence, preferably consisting of 30 cytosines.
g) at least one histone stem-loop sequence, preferably the corresponding RNA sequence of the nucleic acid sequence according to SEQ ID NO. 30.

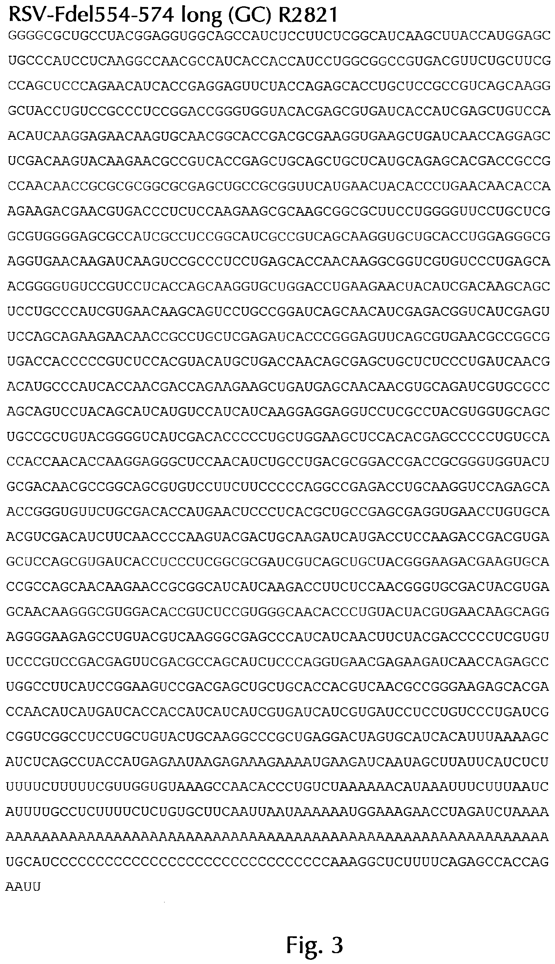

In an even more particularly preferred embodiment the inventive mRNA sequence comprises or consists of the sequences shown in Fig. 3 according to SEQ ID NO 33.

In further specific embodiments, the mRNA according to the invention may further comprise an internal ribosome entry site (IRES) sequence or IRES-motif, which may separate several open reading frames, for example if the inventive mRNA encodes for two or more antigenic peptides or proteins. An IRES-sequence may be particularly helpful if the mRNA is a bi- or multicistronic mRNA.

Additionally, the inventive mRNA may be prepared using any method known in the art, including synthetic methods such as e.g. solid phase synthesis, as well as *in vitro* methods, such as *in vitro* transcription reactions.

According to one embodiment of the present invention the mRNA comprising a coding region as defined in the claims may be administered naked without being associated with any further vehicle, transfection or complexation agent for increasing the transfection efficiency and/or the immunostimulatory properties of the inventive mRNA or of further comprised nucleic acid.

In a preferred embodiment, the inventive mRNA may be formulated together with a cationic or polycationic compound and/or with a polymeric carrier. Accordingly, in a further embodiment of the invention it is preferred that the inventive mRNA or any other nucleic acid comprised in the inventive pharmaceutical composition or the vaccine disclosed herein is associated with or complexed with a cationic or polycationic compound or a polymeric carrier, optionally in a weight ratio selected from a range of about 6:1 (w/w) to about 0.25:1 (w/w), more preferably from about 5:1 (w/w) to about 0.5:1 (w/w), even more preferably of about 4:1 (w/w) to about 1:1 (w/w) or of about 3:1 (w/w) to about 1:1 (w/w), and most preferably a ratio of about 3:1 (w/w) to about 2:1 (w/w) of mRNA or nucleic acid to cationic or polycationic compound and/or with a polymeric carrier; or optionally in a nitrogen/phosphate ratio of mRNA or nucleic acid to cationic or polycationic compound and/or polymeric carrier in the range of about 0.1-10, preferably in a range of about 0.3-4 or 0.3-1, and most preferably in a range of about 0.5-1 or 0.7-1, and even most preferably in a range of about 0.3-0.9 or 0.5-0.9.

Thereby, the inventive mRNA or any other nucleic acid comprised in the inventive pharmaceutical composition or the vaccine disclosed herein can also be associated with a vehicle, transfection or complexation agent for increasing the transfection efficiency and/or the immunostimulatory properties of the inventive mRNA or of optionally comprised further included nucleic acids.

Cationic or polycationic compounds, being particularly preferred agents in this context include protamine, nucleoline, spermine or spermidine, or other cationic peptides or proteins, such as poly-L-lysine (PLL), poly-arginine, basic polypeptides, cell penetrating peptides (CPPs), including HIV-binding peptides, HIV-1 Tat (HIV), Tat-derived peptides, Penetratin, VP22 derived or analog peptides, HSV VP22 (Herpes simplex), MAP, KALA or protein transduction domains (PTDs), PpT620, prolin-rich peptides, arginine-rich peptides, lysine-rich peptides, MPG-peptide(s), Pep-1, L-oligomers, Calcitonin peptide(s), Antennapedia-derived peptides (particularly from *Drosophila antennapedia*), pAntp, plsl, FGF, Lactoferrin, Transportan, Buforin-2, Bac715-24, SynB, SynB(1), pVEC, hCT-derived peptides, SAP, or histones.

In this context, protamine is particularly preferred.

Additionally, preferred cationic or polycationic proteins or peptides may be selected from the following proteins or peptides having the following total formula (III):

(Arg)ₗ;(Lys)ₘ;(His)ₙ;(Orn)ₒ;(Xaa)ₓ, (formula (III))

wherein I + m + n +o + x = 8-15, and l, m, n or o independently of each other may be any number selected from 0, 1, 2, 3, 4, 5, 6, 7, 8, 9, 10, 11, 12, 13, 14 or 15, provided that the overall content of Arg, Lys, His and Orn represents at least 50% of all amino acids of the oligopeptide; and Xaa may be any amino acid selected from native (= naturally occurring) or non-native amino acids except of Arg, Lys, His or Orn; and x may be any number selected from 0, 1, 2, 3 or 4, provided, that the overall content of Xaa does not exceed 50 % of all amino acids of the oligopeptide. Particularly preferred cationic peptides in this context are e.g. Arg₇, Arg₈, Arg₉, H₃R₉, R₉H₃, H₃R₉H₃, YSSR₉SSY, (RKH)₄, Y(RKH)₂R, etc. In this context the disclosure of WO 2009/030481 is incorporated herewith by reference.

Further preferred cationic or polycationic compounds, which can be used as transfection or complexation agent may include cationic polysaccharides, for example chitosan, polybrene, cationic polymers, e.g. polyethyleneimine (PEI), cationic lipids, e.g. DOTMA: [1-(2,3-sioleyloxy)propyl)]-N,N,N-trimethylammonium chloride, DMRIE, di-C14-amidine, DOTIM, SAINT, DC-Chol, BGTC, CTAP, DOPC, DODAP, DOPE: Dioleyl phosphatidylethanol-amine, DOSPA, DODAB, DOIC, DMEPC, DOGS: Dioctadecylamidoglicylspermin, DIMRI: Dimyristo-oxypropyl dimethyl hydroxyethyl ammonium bromide, DOTAP: dioleoyloxy-3-(trimethylammonio)propane, DC-6-14: O,O-ditetradecanoyl-N-(α-trimethylammonioacetyl)diethanolamine chloride, CLIP1: rac-[(2,3-dioctadecyloxypropyl)(2-hydroxyethyl)]-dimethylammonium chloride, CLIP6: rac-[2(2,3-dihexadecyloxypropyl-oxymethyloxy)ethyl]trimethylammonium, CLIP9: rac-[2(2,3-dihexadecyloxypropyl-oxysuccinyloxy)ethyl]-trimethylammonium, oligofectamine, or cationic or polycationic polymers, e.g. modified polyaminoacids, such as β-aminoacid-polymers or reversed polyamides, etc., modified polyethylenes, such as PVP (poly(N-ethyl-4-vinylpyridinium bromide)), etc., modified acrylates, such as pDMAEMA (poly(dimethylaminoethyl methylacrylate)), etc., modified amidoamines such as pAMAM (poly(amidoamine)), etc., modified polybetaaminoester (PBAE), such as diamine end modified 1,4 butanediol diacrylate-co-5-amino-1-pentanol polymers, etc., dendrimers, such as polypropylamine dendrimers or pAMAM based dendrimers, etc., polyimine(s), such as PEI: poly(ethyleneimine), poly(propyleneimine), etc., polyallylamine, sugar backbone based polymers, such as cyclodextrin based polymers, dextran based polymers, chitosan, etc., silan backbone based polymers, such as PMOXA-PDMS copolymers, etc., blockpolymers consisting of a combination of one or more cationic blocks (e.g. selected from a cationic polymer as mentioned above) and of one or more hydrophilic or hydrophobic blocks (e.g. polyethyleneglycole); etc.

A polymeric carrier used according to the invention might be a polymeric carrier formed by disulfide-crosslinked cationic components. The disulfide-crosslinked cationic components may be the same or different from each other. The polymeric carrier can also contain further components. It is also particularly preferred that the polymeric carrier used according to the present invention comprises mixtures of cationic peptides, proteins or polymers and optionally further components as defined herein, which are crosslinked by disulfide bonds as described herein. In this context, reference is made to the disclosure of WO 2012/013326.

In this context, the cationic components, which form basis for the polymeric carrier by disulfide-crosslinkage, are typically selected from any suitable cationic or polycationic peptide, protein or polymer suitable for this purpose, particular any cationic or polycationic peptide, protein or polymer capable to complex an mRNA or a nucleic acid as defined herein, and thereby preferably condensing the mRNA or the nucleic acid. The cationic or polycationic peptide, protein or polymer, is preferably a linear molecule, however, branched cationic or polycationic peptides, proteins or polymers may also be used.

Every disulfide-crosslinking cationic or polycationic protein, peptide or polymer of the polymeric carrier, which may be used to complex the inventive mRNA or any further nucleic acid comprised in the inventive pharmaceutical composition or the vaccine disclosed herein contains at least one -SH moiety, most preferably at least one cysteine residue or any further chemical group exhibiting an -SH moiety, capable to form a disulfide linkage upon condensation with at least one further cationic or polycationic protein, peptide or polymer as cationic component of the polymeric carrier as mentioned herein.

As defined above, the polymeric carrier, which may be used to complex the inventive mRNA or any further nucleic acid comprised in the inventive pharmaceutical composition or the vaccine disclosed herein may be formed by disulfide-crosslinked cationic (or polycationic) components.

Preferably, such cationic or polycationic peptides or proteins or polymers of the polymeric carrier, which comprise or are additionally modified to comprise at least one -SH moiety, are selected from, proteins, peptides and polymers as defined above for complexation agent.

In a further particular embodiment, the polymeric carrier which may be used to complex the inventive mRNA or any further nucleic acid comprised in the inventive pharmaceutical composition or the vaccine disclosed herein may be selected from a polymeric carrier molecule according to generic formula (IV):

L-P¹-S-[S-P²-S]ₙ-S-P³-L formula (IV)

wherein,
- P¹ and P³: are different or identical to each other and represent a linear or branched hydrophilic polymer chain, each P¹ and P³ exhibiting at least one -SH-moiety, capable to form a disulfide linkage upon condensation with component P², or alternatively with (AA), (AA)ₓ, or [(AA)ₓ]_{z} if such components are used as a linker between P¹ and P² or P³ and P²) and/or with further components (e.g. (AA), (AA)ₓ, [(AA)ₓ]_{z} or L), the linear or branched hydrophilic polymer chain selected independent from each other from polyethylene glycol (PEG), poly-*N*-(2-hydroxypropyl)methacrylamide, poly-2-(methacryloyloxy)ethyl phosphorylcholines, poly(hydroxyalkyl L-asparagine), poly(2-(methacryloyloxy)ethyl phosphorylcholine), hydroxyethylstarch or poly(hydroxyalkyl L-glutamine), wherein the hydrophilic polymer chain exhibits a molecular weight of about 1 kDa to about 100 kDa, preferably of about 2 kDa to about 25 kDa; or more preferably of about 2 kDa to about 10 kDa, e.g. about 5 kDa to about 25 kDa or 5 kDa to about 10 kDa;
- P²: is a cationic or polycationic peptide or protein, e.g. as defined above for the polymeric carrier formed by disulfide-crosslinked cationic components, and preferably having a length of about 3 to about 100 amino acids, more preferably having a length of about 3 to about 50 amino acids, even more preferably having a length of about 3 to about 25 amino acids, e.g. a length of about 3 to 10, 5 to 15, 10 to 20 or 15 to 25 amino acids, more preferably a length of about 5 to about 20 and even more preferably a length of about 10 to about 20; or
is a cationic or polycationic polymer, e.g. as defined above for the polymeric carrier formed by disulfide-crosslinked cationic components, typically having a molecular weight of about 0.5 kDa to about 30 kDa, including a molecular weight of about 1 kDa to about 20 kDa, even more preferably of about 1.5 kDa to about 10 kDa, or having a molecular weight of about 0.5 kDa to about 100 kDa, including a molecular weight of about 10 kDa to about 50 kDa, even more preferably of about 10 kDa to about 30 kDa;
each P² exhibiting at least two -SH-moieties, capable to form a disulfide linkage upon condensation with further components P² or component(s) P¹ and/or P³ or alternatively with further components (e.g. (AA), (AA)ₓ, or [(AA)ₓ]_{z});
- -S-S-: is a (reversible) disulfide bond (the brackets are omitted for better readability), wherein S preferably represents sulphur or a -SH carrying moiety, which has formed a (reversible) disulfide bond. The (reversible) disulfide bond is preferably formed by condensation of -SH-moieties of either components P¹ and P², P² and P², or P² and P³, or optionally of further components as defined herein (e.g. L, (AA), (AA)ₓ, [(AA)ₓ]_{z}, etc); The -SH-moiety may be part of the structure of these components or added by a modification as defined below;
- L: is an optional ligand, which may be present or not, and may be selected independent from the other from RGD, Transferrin, Folate, a signal peptide or signal sequence, a localization signal or sequence, a nuclear localization signal or sequence (NLS), an antibody, a cell penetrating peptide, (e.g. TAT or KALA), a ligand of a receptor (e.g. cytokines, hormones, growth factors etc), small molecules (e.g. carbohydrates like mannose or galactose or synthetic ligands), small molecule agonists, inhibitors or antagonists of receptors (e.g. RGD peptidomimetic analogues), or any further protein as defined herein, etc.;
- n: is an integer, typically selected from a range of about 1 to 50, preferably from a range of about 1, 2 or 3 to 30, more preferably from a range of about 1, 2, 3, 4, or 5 to 25, or a range of about 1, 2, 3, 4, or 5 to 20, or a range of about 1, 2, 3, 4, or 5 to 15, or a range of about 1, 2, 3, 4, or 5 to 10, including e.g. a range of about 4 to 9, 4 to 10, 3 to 20, 4 to 20, 5 to 20, or 10 to 20, or a range of about 3 to 15, 4 to 15, 5 to 15, or 10 to 15, or a range of about 6 to 11 or 7 to 10. Most preferably, n is in a range of about 1, 2, 3, 4, or 5 to 10, more preferably in a range of about 1, 2, 3, or 4 to 9, in a range of about 1, 2, 3, or 4 to 8, or in a range of about 1, 2, or 3 to 7.

In this context, reference is made to the disclosure of WO 2011/026641. Each of hydrophilic polymers P¹ and P³ typically exhibits at least one -SH-moiety, wherein the at least one -SH-moiety is capable to form a disulfide linkage upon reaction with component P² or with component (AA) or (AA)ₓ, if used as linker between P¹ and P² or P³ and P² as defined below and optionally with a further component, e.g. L and/or (AA) or (AA)ₓ, e.g. if two or more -SH-moieties are contained. The following subformulae "P¹S-S-P²" and "P²-S-S-P³" within generic formula (V) above (the brackets are omitted for better readability), wherein any of S, P¹ and P³ are as defined herein, typically represent a situation, wherein one-SH-moiety of hydrophilic polymers P¹ and P³ was condensed with one -SH-moiety of component P² of generic formula (V) above, wherein both sulphurs of these -SH-moieties form a disulfide bond -S-S- as defined herein in formula (V). These -SH-moieties are typically provided by each of the hydrophilic polymers P¹ and P³, e.g. via an internal cysteine or any further (modified) amino acid or compound which carries a -SH moiety. Accordingly, the subformulae "P¹-S-S-P²" and "P²-S-S-P³" may also be written as "P¹Cys-Cys-P²" and "P²-Cys-Cys-P³", if the -SH- moiety is provided by a cysteine, wherein the term Cys-Cys represents two cysteines coupled via a disulfide bond, not via a peptide bond. In this case, the term "-S-S-" in these formulae may also be written as "-S-Cys", as "-Cys-S" or as "-Cys-Cys-". In this context, the term "-Cys-Cys-" does not represent a peptide bond but a linkage of two cysteines via their -SH-moieties to form a disulfide bond. Accordingly, the term "-Cys-Cys-" also may be understood generally as "-(Cys-S)-(S-Cys)-", wherein in this specific case S indicates the sulphur of the -SH-moiety of cysteine. Likewise, the terms "-S-Cys" and "-Cys-S" indicate a disulfide bond between a -SH containing moiety and a cysteine, which may also be written as "-S-(S-Cys)" and "-(Cys-S)-S". Alternatively, the hydrophilic polymers P¹ and P³ may be modified with a -SH moiety, preferably via a chemical reaction with a compound carrying a -SH moiety, such that each of the hydrophilic polymers P¹ and P³ carries at least one such -SH moiety. Such a compound carrying a -SH moiety may be e.g. an (additional) cysteine or any further (modified) amino acid, which carries a -SH moiety. Such a compound may also be any non-amino compound or moiety, which contains or allows to introduce a -SH moiety into hydrophilic polymers P¹ and P³ as defined herein. Such non-amino compounds may be attached to the hydrophilic polymers P¹ and P³ of formula (VI) of the polymeric carrier according to the present invention via chemical reactions or binding of compounds, e.g. by binding of a 3-thio propionic acid or thioimolane, by amide formation (e.g. carboxylic acids, sulphonic acids, amines, etc), by Michael addition (e.g maleinimide moieties, α,β unsatured carbonyls, etc), by click chemistry (e.g. azides or alkines), by alkene/alkine methatesis (e.g. alkenes or alkines), imine or hydrozone formation (aldehydes or ketons, hydrazins, hydroxylamins, amines), complexation reactions (avidin, biotin, protein G) or components which allow Sₙ-type substitution reactions (e.g halogenalkans, thiols, alcohols, amines, hydrazines, hydrazides, sulphonic acid esters, oxyphosphonium salts) or other chemical moieties which can be utilized in the attachment of further components. A particularly preferred PEG derivate in this context is alpha-Methoxy-omega-mercapto poly(ethylene glycol). In each case, the SH-moiety, e.g. of a cysteine or of any further (modified) amino acid or compound, may be present at the terminal ends or internally at any position of hydrophilic polymers P¹ and P³. As defined herein, each of hydrophilic polymers P¹ and P³ typically exhibits at least one -SH-moiety preferably at one terminal end, but may also contain two or even more -SH-moieties, which may be used to additionally attach further components as defined herein, preferably further functional peptides or proteins e.g. a ligand, an amino acid component (AA) or (AA)ₓ, antibodies, cell penetrating peptides or enhancer peptides (e.g. TAT, KALA), etc.

In this context, it is particularly preferred that the inventive mRNA is complexed at least partially with a cationic or polycationic compound and/or a polymeric carrier, preferably cationic proteins or peptides. In this context, reference is made to the disclosure of WO 2010/037539 and WO 2012/113513. Partially means that only a part of the inventive mRNA is complexed with a cationic compound and that the rest of the inventive mRNA is (comprised in the inventive pharmaceutical compostion or the vaccine disclosed herein) in uncomplexed form ("free"). Preferably the ratio of complexed mRNA to: free mRNA (in the inventive pharmaceutical composition or the vaccine disclosed herein) is selected from a range of about 5:1 (w/w) to about 1:10 (w/w), more preferably from a range of about 4:1 (w/w) to about 1:8 (w/w), even more preferably from a range of about 3:1 (w/w) to about 1:5 (w/w) or 1:3 (w/w), and most preferably the ratio of complexed mRNA to free mRNA in the inventive pharmaceutical composition or the vaccine disclosed herein is selected from a ratio of about 1:1 (w/w).

The complexed mRNA in the inventive pharmaceutical composition or the vaccine disclosed herein, is preferably prepared according to a first step by complexing the inventive mRNA with a cationic or polycationic compound and/or with a polymeric carrier, preferably as defined herein, in a specific ratio to form a stable complex. In this context, it is highly preferable, that no free cationic or polycationic compound or polymeric carrier or only a negligibly small amount thereof remains in the component of the complexed mRNA after complexing the mRNA. Accordingly, the ratio of the mRNA and the cationic or polycationic compound and/or the polymeric carrier in the component of the complexed mRNA is typically selected in a range that the mRNA is entirely complexed and no free cationic or polycationic compound or polymeric carrier or only a negligibly small amount thereof remains in the composition.

Preferably the ratio of the mRNA to the cationic or polycationic compound and/or the polymeric carrier, preferably as defined herein, is selected from a range of about 6:1 (w/w) to about 0,25:1 (w/w), more preferably from about 5:1 (w/w) to about 0,5:1 (w/w), even more preferably of about 4:1 (w/w) to about 1:1 (w/w) or of about 3:1 (w/w) to about 1:1 (w/w), and most preferably a ratio of about 3:1 (w/w) to about 2:1 (w/w). Alternatively, the ratio of the mRNA to the cationic or polycationic compound and/or the polymeric carrier, preferably as defined herein, in the component of the complexed mRNA, may also be calculated on the basis of the nitrogen/phosphate ratio (N/P-ratio) of the entire complex. In the context of the present invention, an N/P-ratio is preferably in the range of about 0.1-10, preferably in a range of about 0.3-4 and most preferably in a range of about 0.5-2 or 0.7-2 regarding the ratio of mRNA: cationic or polycationic compound and/or polymeric carrier, preferably as defined herein, in the complex, and most preferably in a range of about 0.7-1,5, 0.5-1 or 0.7-1, and even most preferably in a range of about 0.3-0.9 or 0.5-0.9., preferably provided that the cationic or polycationic compound in the complex is a cationic or polycationic cationic or polycationic protein or peptide and/or the polymeric carrier as defined above. In this specific embodiment the complexed mRNA is also emcompassed in the term "adjuvant component".

In a further aspect, the invention provides for a composition comprising a plurality or more than one, preferably 2 to 10, more preferably 2 to 5, most preferably 2 to 4 of the mRNA sequences as defined herein. These inventive compositions comprise more than one mRNA sequence, preferably encoding different peptides or proteins which comprise preferably different pathogenic antigens or fragments, variants or derivatives thereof. Of these, at least one mRNA sequence comprises a coding region, encoding a mutant of fusion protein F of Respiratory syncytial virus (RSV), wherein the amino acid residues 554-574 of the amino acid sequence of the fusion protein F are deleted in the mutant of fusion protein F, wherein the G/C content of the coding region is increased by substituting at least 70% of the substitutable codons in the coding region compared with the G/C content of the coding region of the wild type mRNA, and wherein the coded amino acid sequence of said GC-enriched mRNA is not being modified compared with the coded amino acid sequence of the wild type mRNA and at least one mRNA sequence encodes at least one antigenic peptide or protein derived from another antigen of Respiratory syncytial virus (RSV), particularly of nucleoprotein N or M2-1 protein.

Combinations of antigens disclosed in this context are:
- F + G (serotype A) + G (serotype B)
- F + G (serotype A) + G (serotype B) + M2-1
- F + G (serotype A) + G (serotype B) + N
- F + G (serotype A) + G (serotype B) + N + M2-1
- F + M2-1 + N
- F + M2-1
- F + N
- F + G (serotype A) + G (serotype B) + N + M2-1 + P + M2-2 + M + L
- F + G (serotype A) + G (serotype B) + N + M2-1 + P + M2-2 + M
- F + G (serotype A) + G (serotype B) + N + M2-1 + P + M2-2 + L
- F + G (serotype A) + G (serotype B) + N + M2-1 + P + M + L
- F + G (serotype A) + G (serotype B) + N + M2-1 + M2-2 + M + L
- F + G (serotype A) + G (serotype B) + N + P + M2-2 + M + L
- F + G (serotype A) + G (serotype B) + M2-1 + P + M2-2 + M + L

Accordingly, in a further particular preferred aspect, the present invention also provides a pharmaceutical composition as defined by the attached claims, comprising at least one inventive mRNA sequence as defined herein or an inventive composition comprising a plurality of mRNA sequences as defined herein and optionally a pharmaceutically acceptable carrier and/or vehicle.

As a first ingredient, the inventive pharmaceutical composition comprises at least one inventive mRNA sequence as defined herein.

As a second ingredient, the inventive pharmaceutical composition may optionally comprise at least one additional pharmaceutically active component. A pharmaceutically active component in this connection is a compound that has a therapeutic effect to heal, ameliorate or prevent a particular indication or disease as mentioned herein, preferably RSV infections. Such compounds include, without implying any limitation, peptides or proteins, preferably as defined herein, nucleic acids, preferably as defined herein, (therapeutically active) low molecular weight organic or inorganic compounds (molecular weight less than 5000, preferably less than 1000), sugars, antigens or antibodies, preferably as defined herein, therapeutic agents already known in the prior art, antigenic cells, antigenic cellular fragments, cellular fractions; cell wall components (e.g. polysaccharides), modified, attenuated or deactivated (e.g. chemically or by irradiation) pathogens (virus, bacteria etc.), adjuvants, preferably as defined herein, etc. Particularly preferred in this context are RSV vaccines, or RSV immune globulines, e.g. Palivizumab (Synagis®).

The pharmaceutical composition may be administered orally, parenterally, by inhalation spray, topically, rectally, nasally, buccally, vaginally or via an implanted reservoir. The term parenteral as used herein includes subcutaneous, intravenous, intramuscular, intra-articular, intra-synovial, intrasternal, intrathecal, intrahepatic, intralesional, intracranial, transdermal, intradermal, intrapulmonal, intraperitoneal, intracardial, intraarterial, and sublingual injection or infusion techniques.

Particularly preferred is intradermal and intramuscular injection. Sterile injectable forms of the inventive pharmaceutical compositions may be aqueous or oleaginous suspension. These suspensions may be formulated according to techniques known in the art using suitable dispersing or wetting agents and suspending agents.

In a preferred embodiment, the inventive pharmaceutical composition is administered via intradermal or intramuscular injection, preferably by using conventional needle-based injection technique or by using a needle-free system, e.g. jet injection. In a further preferred embodiment, the inventive pharmaceutical composition may be administered by jet injection as defined herein. Preferably, the inventive pharmaceutical composition may be adiminstered intramuscularly by jet injection. According to another embodiment, the pharmaceutical composition is administered intradermally via jet injection.

In a preferred embodiment, the pharmaceutical composition may be administered once, twice or three times, preferably by intradermal or intramuscular injection, preferably by jet injection. According to a certain embodiment, a single administration of the inventive pharmaceutical composition, preferably via intradermal or intramuscular injection, preferably by using jet injection, is sufficient for eliciting an immune response against the at least one antigen encoded by the mRNA sequence according to the invention. In a preferred embodiment, the single administration of the pharmaceutical composition elicits an immune response resulting in virus neutralisation. In this context, one single intradermal or intramuscular injection of the pharmaceutical composition is particularly preferred. Preferably, further administrations of the pharmaceutical composition may optionally be carried out in order to enhance and/or prolong the immune response.

According to a specific embodiment, the inventive pharmaceutical composition may comprise an adjuvant. In this context, an adjuvant may be understood as any compound, which is suitable to initiate or increase an immune response of the innate immune system, i.e. a non-specific immune response. With other words, when administered, the inventive pharmaceutical composition preferably elicits an innate immune response due to the adjuvant, optionally contained therein. Preferably, such an adjuvant may be selected from an adjuvant known to a skilled person and suitable for the present case, i.e. supporting the induction of an innate immune response in a mammal, e.g. an adjuvant protein as defined above or an adjuvant as defined in the following.

Particularly preferred as adjuvants suitable for depot and delivery are cationic or polycationic compounds as defined above for the inventive mRNA sequence as vehicle, transfection or complexation agent.
Furthermore, the inventive pharmaceutical composition may comprise one or more additional adjuvants, which are suitable to initiate or increase an immune response of the innate immune system, i.e. a non-specific immune response, particularly by binding to pathogen-associated molecular patterns (PAMPs). With other words, when administered, the pharmaceutical composition or vaccine preferably elicits an innate immune response due to the adjuvant, optionally contained therein. Preferably, such an adjuvant may be selected from an adjuvant known to a skilled person and suitable for the present case, i.e. supporting the induction of an innate immune response in a mammal, e.g. an adjuvant protein as defined above or an adjuvant as defined in the following. According to one embodiment such an adjuvant may be selected from an adjuvant as defined above.

Also such an adjuvant may be selected from any adjuvant known to a skilled person and suitable for the present case, i.e. supporting the induction of an innate immune response in a mammal and/or suitable for depot and delivery of the components of the inventive pharmaceutical composition or the vaccine disclosed herein. Preferred as adjuvants suitable for depot and delivery are cationic or polycationic compounds as defined above. Likewise, the adjuvant may be selected from the group consisting of, e.g., cationic or polycationic compounds as defined above, from chitosan, TDM, MDP, muramyl dipeptide, pluronics, alum solution, aluminium hydroxide, ADJUMER™ (polyphosphazene); aluminium phosphate gel; glucans from algae; algammulin; aluminium hydroxide gel (alum); highly protein-adsorbing aluminium hydroxide gel; low viscosity aluminium hydroxide gel; AF or SPT (emulsion of squalane (5%), Tween 80 (0.2%), Pluronic L121 (1.25%), phosphate-buffered saline, pH 7.4); AVRIDINE™ (propanediamine); BAY R1005™ ((N-(2-deoxy-2-L-leucylaminob- D-glucopyranosyl)-N-octadecyl-dodecanoyl-amide hydroacetate); CALCITRIOL™ (1-alpha,25-dihydroxy-vitamin D3); calcium phosphate gel; CAP™ (calcium phosphate nanoparticles); cholera holotoxin, cholera-toxin-A1-protein-A-D-fragment fusion protein, sub-unit B of the cholera toxin; CRL 1005 (block copolymer P1205); cytokine-containing liposomes; DDA (dimethyldioctadecylammonium bromide); DHEA (dehydroepiandrosterone); DMPC (dimyristoylphosphatidylcholine); DMPG (dimyristoylphosphatidylglycerol); DOC/alum complex (deoxycholic acid sodium salt); Freund's complete adjuvant; Freund's incomplete adjuvant; gamma inulin; Gerbu adjuvant (mixture of: i) N-acetylglucosaminyl-(P1-4)-N-acetylmuramyl-L-alanyl-D35 glutamine (GMDP), ii) dimethyldioctadecylammonium chloride (DDA), iii) zinc-L-proline salt complex (ZnPro-8); GM-CSF); GMDP (N-acetylglucosaminyl-(b1-4)-N-acetylmuramyl-L47 alanyl-D-isoglutamine); imiquimod (1-(2-methypropyl)-1H-imidazo[4,5-c]quinoline-4-amine); ImmTher™ (N-acetylglucosaminyl-N-acetylmuramyl-L-Ala-D-isoGlu-L-Ala-glycerol dipalmitate); DRVs (immunoliposomes prepared from dehydration-rehydration vesicles); interferongamma; interleukin-1beta; interleukin-2; interleukin-7; interleukin-12; ISCOMS™; ISCOPREP 7.0.3.™; liposomes; LOXORIBINE™ (7-allyl-8-oxoguanosine); LT 5 oral adjuvant (*E.coli* labile enterotoxin-protoxin); microspheres and microparticles of any composition; MF59TM; (squalenewater emulsion); MONTANIDE ISA 51™ (purified incomplete Freund's adjuvant); MONTANIDE ISA 720™ (metabolisable oil adjuvant); MPL™ (3-Q-desacyl-4'-monophosphoryl lipid A); MTP-PE and MTP-PE liposomes ((N-acetyl-L-alanyl-D-isoglutaminyl-L-alanine-2-(1,2-dipalmitoyl-sn-glycero-3-(hydroxyphosphoryloxy))-ethylamide, monosodium salt); MURAMETIDE™ (Nac-Mur-L-Ala-D-Gln-OCH3); MURAPALMITINE™ and DMURAPALMITINE™ (Nac-Mur-L-Thr-D-isoGln-sn-glyceroldipalmitoyl); NAGO (neuraminidase- galactose oxidase); nanospheres or nanoparticles of any composition; NISVs (non-ionic surfactant vesicles); PLEURAN™ (β-glucan); PLGA, PGA and PLA (homo- and co-polymers of lactic acid and glycolic acid; microspheres/nanospheres); PLURONIC L121™; PMMA (polymethylmethacrylate); PODDS™ (proteinoid microspheres); polyethylene carbamate derivatives; poly-rA: poly-rU (polyadenylic acid-polyuridylic acid complex); polysorbate 80 (Tween 80); protein cochleates (Avanti Polar Lipids, Inc., Alabaster, AL); STIMULON™ (QS-21); Quil-A (Quil-A saponin); S-28463 (4-amino-otec-dimethyl-2-ethoxymethyl-1H-imidazo[4,5-c]quinoline-1-ethanol); SAF-1™ ("Syntex adjuvant formulation"); Sendai proteoliposomes and Sendai containing lipid matrices; Span-85 (sorbitan trioleate); Specol (emulsion of Marcol 52, Span 85 and Tween 85); squalene or Robane® (2,6,10,15,19,23-hexamethyltetracosan and 2,6,10,15,19,23-hexamethyl-2,6,10,14,18,22-tetracosahexane); stearyltyrosine (octadecyltyrosine hydrochloride); Theramid® (N-acetylglucosaminyl-N-acetylmuramyl-L-Ala-D-isoGlu-L-Aladipalmitoxypropylamide); Theronyl-MDP (Termurtide™ or [thr 1]-MDP; N-acetylmuramyl-Lthreonyl-D-isoglutamine); Ty particles (Ty-VLPs or virus-like particles); Walter-Reed liposomes (liposomes containing lipid A adsorbed on aluminium hydroxide), and lipopeptides, including Pam3Cys, in particular aluminium salts, such as Adju-phos, Alhydrogel, Rehydragel; emulsions, including CFA, SAF, IFA, MF59, Provax, TiterMax, Montanide, Vaxfectin; copolymers, including Optivax (CRL1005), L121, Poloaxmer4010), etc.; liposomes, including Stealth, cochleates, including BIORAL; plant derived adjuvants, including QS21, Quil A, Iscomatrix, ISCOM; adjuvants suitable for costimulation including Tomatine, biopolymers, including PLG, PMM, Inulin, microbe derived adjuvants, including Romurtide, DETOX, MPL, CWS, Mannose, CpG nucleic acid sequences, CpG7909, ligands of human TLR 1-10, ligands of murine TLR 1-13, ISS-1018, 35 IC31, Imidazoquinolines, Ampligen, Ribi529, IMOxine, IRIVs, VLPs, cholera toxin, heat-labile toxin, Pam3Cys, Flagellin, GPI anchor, LNFPIII/Lewis X, antimicrobial peptides, UC-1V150, RSV fusion protein, cdiGMP; and adjuvants suitable as antagonists including CGRP neuropeptide.

Particularly preferred, an adjuvant may be selected from adjuvants, which support induction of a Th1-immune response or maturation of naïve T-cells, such as GM-CSF, IL-12, IFNg, any immunostimulatory nucleic acid as defined above, preferably an immunostimulatory RNA, CpG DNA, etc.

In a further preferred embodiment, it is also possible that the inventive pharmaceutical composition contains besides the antigen-providing mRNA further components, which are selected from the group comprising: further antigens or further antigen-providing nucleic acids; a further immunotherapeutic agent; one or more auxiliary substances; or any further compound, which is known to be immunostimulating due to its binding affinity (as ligands) to human Toll-like receptors; and/or an adjuvant nucleic acid, preferably an immunostimulatory RNA (isRNA).

The inventive pharmaceutical composition can additionally contain one or more auxiliary substances in order to increase its immunogenicity or immunostimulatory capacity, if desired. A synergistic action of the inventive mRNA sequence as defined herein and of an auxiliary substance, which may be optionally contained in the inventive pharmaceutical composition, is preferably achieved thereby. Depending on the various types of auxiliary substances, various mechanisms can come into consideration in this respect. For example, compounds that permit the maturation of dendritic cells (DCs), for example lipopolysaccharides, TNF-alpha or CD40 ligand, form a first class of suitable auxiliary substances. In general, it is possible to use as auxiliary substance any agent that influences the immune system in the manner of a "danger signal" (LPS, GP96, etc.) or cytokines, such as GM-CFS, which allow an immune response to be enhanced and/or influenced in a targeted manner. Particularly preferred auxiliary substances are cytokines, such as monokines, lymphokines, interleukins or chemokines, that further promote the innate immune response, such as IL-1, IL-2, IL-3, IL-4, IL-5, IL-6, IL-7, IL-8, IL-9, IL-10, IL-12, IL-13, IL-14, IL-15, IL-16, IL-17, IL-18, IL-19, IL-20, IL-21, IL-22, IL-23, IL-24, IL-25, IL-26, IL-27, IL-28, IL-29, IL-30, IL-31, IL-32, IL-33, IFN-alpha, IFN-beta, IFN-gamma, GM-CSF, G-CSF, M-CSF, LT-beta or TNF-alpha, growth factors, such as hGH.

Further additives, which may be included in the inventive pharmaceutical composition, are emulsifiers, such as, for example, Tween®; wetting agents, such as, for example, sodium lauryl sulfate; colouring agents; taste-imparting agents, pharmaceutical carriers; tablet-forming agents; stabilizers; antioxidants; preservatives.

The inventive pharmaceutical composition can also additionally contain any further compound, which is known to be immunostimulating due to its binding affinity (as ligands) to human Toll-like receptors TLR1, TLR2, TLR3, TLR4, TLR5, TLR6, TLR7, TLR8, TLR9, TLR10, or due to its binding affinity (as ligands) to murine Toll-like receptors TLR1, TLR2, TLR3, TLR4, TLR5, TLR6, TLR7, TLR8, TLR9, TLR10, TLR11, TLR12 or TLR13.

In this context, it is particularly preferred that the optionally comprised adjuvant component comprises the same inventive mRNA as comprised in the inventive pharmaceutical composition as antigen-providing mRNA.

Despite, the inventive pharmaceutical composition may comprise further components for facilitating administration and uptake of components of the pharmaceutical composition. Such further components may be an appropriate carrier or vehicle, additional adjuvants for supporting any immune response, antibacterial and/or antiviral agents.

Accordingly, in a further embodiment, the inventive pharmaceutical composition furthermore comprises a pharmaceutically acceptable carrier and/or vehicle.

Such a pharmaceutically acceptable carrier typically includes the liquid or non-liquid basis of a composition comprising the components of the inventive pharmaceutical composition. If the composition is provided in liquid form, the carrier will typically be pyrogen-free water; isotonic saline or buffered (aqueous) solutions, e.g. phosphate, citrate etc. buffered solutions. The injection buffer may be hypertonic, isotonic or hypotonic with reference to the specific reference medium, i.e. the buffer may have a higher, identical or lower salt content with reference to the specific reference medium, wherein preferably such concentrations of the afore mentioned salts may be used, which do not lead to damage of cells due to osmosis or other concentration effects. Reference media are e.g. liquids occurring in *"in vivo"* methods, such as blood, lymph, cytosolic liquids, or other body liquids, or e.g. liquids, which may be used as reference media in *"in vitro"* methods, such as common buffers or liquids. Such common buffers or liquids are known to a skilled person. Ringer-Lactate solution is particularly preferred as a liquid basis.

However, one or more compatible solid or liquid fillers or diluents or encapsulating compounds, which are suitable for administration to a patient to be treated, may be used as well for the pharmaceutical composition according to the invention. The term "compatible" as used here means that these constituents of the inventive pharmaceutical composition are capable of being mixed with the components of the inventive pharmaceutical composition in such a manner that no interaction occurs which would substantially reduce the pharmaceutical effectiveness of the pharmaceutical compostion under typical use conditions.

A further component of the inventive pharmaceutical composition may be an immunotherapeutic agent that can be selected from immunoglobulins, preferably IgGs, monoclonal or polyclonal antibodies, polyclonal serum or sera, etc, most preferably immunoglobulins directed against Respiratory syncytial virus (RSV), e.g. Palivizumab. Preferably, such a further immunotherapeutic agent may be provided as a peptide/protein or may be encoded by a nucleic acid, preferably by a DNA or an RNA, more preferably an mRNA. Such an immunotherapeutic agent allows providing passive vaccination additional to active vaccination triggered by the inventive antigen-providing mRNA.

Furthermore, in a specific embodiment, additionally to the antigen-providing mRNA further antigens can be included in the inventive pharmaceutical composition and are typically substances such as cells, cell lysates, viruses, attenuated viruses, inactivated viruses, proteins, peptides, nucleic acids or other bio- or macromolecules or fragments thereof. Preferably, antigens may be proteins and peptides or fragments thereof, such as epitopes of those proteins or peptides, preferably having 5 to 15, more preferably 6 to 9, amino acids. Particularly, said proteins, peptides or epitopes may be derived from the fusion protein F, the glycoprotein G, the short hydrophobic protein SH, the matrix protein M, the nucleoprotein N, the large polymerase L, the M2-1 protein, the M2-2 protein, the phosphoprotein P, the non-structural protein NS1 or the non-structural protein NS2 of Respiratory syncytial virus (RSV), or from fragments, variants or derivatives thereof. Further, antigens may also comprise any other biomolecule, e.g., lipids, carbohydrates, etc. Preferably, the antigen is a protein or (poly-) peptide antigen, a nucleic acid, a nucleic acid encoding a protein or (poly-) peptide antigen, a polysaccharide antigen, a polysaccharide conjugate antigen, a lipid antigen, a glycolipid antigen, a carbohydrate antigen, a bacterium, a cell (vaccine), or killed or attenuated viruses.

The inventive pharmaceutical composition or the vaccine disclosed herein as defined herein may furthermore comprise further additives or additional compounds. Further additives, which may be included in the pharmaceutical composition, are emulsifiers, such as, for example, Tween®; wetting agents, such as, for example, sodium lauryl sulfate; colouring agents; taste-imparting agents, pharmaceutical carriers; tablet-forming agents; stabilizers; antioxidants; preservatives, RNase inhibitors and/or an anti-bacterial agent or an anti-viral agent. Additionally the inventive pharmaceutical composition may comprise small interfering RNA (siRNA) directed against genes of Respiratory syncytial virus (RSV), e.g. siRNA directed against the gene encoding the fusion protein F, the glycoprotein G, the short hydrophobic protein SH, the matrix protein M, the nucleoprotein N, the large polymerase L, the M2-1 protein, the M2-2 protein, the phosphoprotein P, the non-structural protein NS1 or the non-structural protein NS2 of Respiratory syncytial virus (RSV). The inventive pharmaceutical composition typically comprises a "safe and effective amount" of the components of the inventive pharmaceutical composition, particularly of the inventive mRNA sequence(s) as defined herein. As used herein, a "safe and effective amount" means an amount of the inventive mRNA sequence(s) as defined herein as such that is sufficient to significantly induce a positive modification of a disease or disorder or to prevent a disease, preferably RSV infections as defined herein. At the same time, however, a "safe and effective amount" is small enough to avoid serious side-effects and to permit a sensible relationship between advantage and risk. The determination of these limits typically lies within the scope of sensible medical judgment.

The inventive pharmaceutical composition may be used for human and also for veterinary medical purposes, preferably for human medical purposes, as a pharmaceutical composition in general or as a vaccine.

According to another particularly preferred aspect, the inventive pharmaceutical composition (or the inventive mRNA sequence as defined herein or the inventive composition comprising a plurality of mRNA sequences as defined herein) may be provided or used as a vaccine. Typically, such a vaccine is as defined above for pharmaceutical compositions. Additionally, such a vaccine typically contains the inventive mRNA sequence as defined herein or the inventive composition comprising a plurality of mRNA sequences as defined herein.

The vaccine disclosed herein may also comprise a pharmaceutically acceptable carrier, adjuvant, and/or vehicle as defined herein for the inventive pharmaceutical composition. In the specific context of the vaccine disclosed herein, the choice of a pharmaceutically acceptable carrier is determined in principle by the manner in which the vaccine disclosed herein is administered. The vaccine disclosed herein can be administered, for example, systemically or locally. Routes for systemic administration in general include, for example, transdermal, oral, parenteral routes, including subcutaneous, intravenous, intramuscular, intraarterial, intradermal and intraperitoneal injections and/or intranasal administration routes. Routes for local administration in general include, for example, topical administration routes but also intradermal, transdermal, subcutaneous, or intramuscular injections or intralesional, intracranial, intrapulmonal, intracardial, and sublingual injections. More preferably, vaccines may be administered by an intradermal, subcutaneous, or intramuscular route. Vaccines disclosed herein are therefore preferably formulated in liquid (or sometimes in solid) form.

In a preferred embodiment, the vaccine disclosed herein is administered via intradermal or intramuscular injection, preferably by using conventional needle-based injection technique or by using a needle-free system, e.g. jet injection. In a further preferred embodiment, the vaccine disclosed herein may be administered by jet injection as defined herein. Preferably, the vaccine disclosed herein is adiminstered intramuscularly by jet injection. According to another embodiment, the vaccine is administered intradermally via jet injection.

In a preferred embodiment, the vaccine may be administered once, twice or three times, preferably by intradermal or intramuscular injection, preferably by jet injection. According to a certain embodiment, a single administration of the vaccine, preferably via intradermal or intramuscular injection, preferably by using jet injection, is sufficient for eliciting an immune response against the at least one antigen encoded by the mRNA sequence according to the invention. In a preferred embodiment, the single administration of the vaccine elicits an immune response resulting in virus neutralisation. In this context, one single intradermal or intramuscular injection of the vaccine is particularly preferred. Preferably, further administrations of the vaccine may optionally be carried out in order to enhance and/or prolong the immune response.

The vaccine disclosed herein can additionally contain one or more auxiliary substances in order to increase its immunogenicity or immunostimulatory capacity, if desired. Particularly preferred are adjuvants as auxiliary substances or additives as defined for the pharmaceutical composition.

In a further aspect, the invention is directed to a kit or kit of parts comprising the inventive mRNA sequence, the inventive composition comprising a plurality of mRNA sequences as defined herein, the inventive pharmaceutical composition or the vaccine disclosed herein and optionally technical instructions with information on the administration and dosage of the components.

Besides the inventive mRNA sequence, the inventive composition comprising a plurality of mRNA sequences as defined herein, the inventive pharmaceutical composition or the vaccine disclosed herein the kit may additionally contain a pharmaceutically acceptable vehicle, an adjuvant and at least one further component as defined herein, as well as means for administration and technical instructions. The inventive mRNA sequence, the inventive composition comprising a plurality of mRNA sequences, the inventive pharmaceutical composition or vaccine and e.g. the adjuvant may be provided in lyophilized form. In a preferred embodiment, prior to use of the kit for vaccination, the provided vehicle is than added to the lyophilized components in a predetermined amount as written e.g. in the provided technical instructions. By doing so the inventive mRNA sequence, the inventive composition comprising a plurality of mRNA sequences as defined herein, the inventive pharmaceutical composition or the vaccine disclosed herein, according to the above described aspects of the invention is provided that can afterwards be used in a method as described above, also.

The present invention furthermore provides several applications and uses of the inventive mRNA sequence as defined herein, of the inventive composition comprising a plurality of mRNA sequences as defined herein, of the inventive pharmaceutical composition, of the vaccine as defined herein, all comprising the inventive mRNA sequence as defined herein or of kits comprising same.

In a further aspect, the invention provides an mRNA sequence as defined by the claims, a composition, a pharmaceutical composition, a vaccine and a kit, all comprising the mRNA sequence for use in a method of prophylactic and/or therapeutic treatment of RSV infections.

Consequently, in a further aspect, the present invention is directed to the first medical use of the inventive mRNA sequence, the inventive composition comprising a plurality of mRNA sequences as defined herein, the inventive pharmaceutical composition, the vaccine disclosed herein, and the inventive kit as defined herein as a medicament. Particularly, the invention provides the use of an mRNA sequence as defined in the claims for the preparation of a medicament.

According to another aspect, the present invention is directed to the second medical use of the mRNA sequence as defined in the claims, optionally in form of a composition comprising a plurality of mRNA sequences as defined herein, a pharmaceutical composition or vaccine, kit or kit of parts, for the treatment of RSV infections as defined herein. Particularly, the mRNA sequence to be used in a method as said above is a mRNA sequence as defined by the claims formulated together with a pharmaceutically acceptable vehicle and an optionally additional adjuvant and an optionally additional further component as defined above e.g. a further antigen or a RSV immune globuline. In this context particularly the (prophylactic) treatment of infants, the elderly and immunocompromised patients is preferred. And even more preferred is the (prophylactic) treatment of pre-term infants and infants with chronic lung disease.

The inventive mRNA sequence may alternatively be provided such that it is for use in preventing or treating RSV infections by several doses, each dose containing the inventive mRNA sequence as defined in the claims, e.g. the first dose containing at least one mRNA encoding a mutant of fusion protein F of Respiratory syncytial virus (RSV), wherein the amino acid residues 554-574 of the amino acid sequence of the fusion protein F are deleted in the mutant of fusion protein F and the second dose containing at least one mRNA sequence encoding at least one antigenic peptide or protein derived from a different antigen of Respiratory syncytial virus (RSV), preferably from the nucleoprotein N (or fragments, variants or derivatives thereof), from the M2-1 protein or the glycoprotein G (or fragments, variants or derivatives thereof). By that embodiment, both doses are administered in a staggered way, i.e. subsequently, shortly one after the other, e.g. within less than 10 minutes, preferably less than 2 minutes, and at the same site of the body to achieve the same immunological effect as for administration of one single composition containing both, e.g. the mRNA encoding the fusion protein F and the mRNA encoding the nucleoprotein N.

According to a specific embodiment, the inventive mRNA sequence, or the inventive pharmaceutical composition or the vaccine disclosed herein may be administered to the patient as a single dose. In certain embodiments, the inventive mRNA sequence or the inventive pharmaceutical composition or the vaccine disclosed herein may be administered to a patient as a single dose followed by a second dose later and optionally even a third, fourth (or more) dose subsequent thereto etc. In accordance with this embodiment, booster inoculations with the inventive mRNA sequence or the inventive pharmaceutical composition or the vaccine disclosed herein may be administered to a patient at specific time intervals, preferably as defined below, following the second (or third, fourth, etc.) inoculation. In this context, it is particularly preferred that several doses comprise the same mRNA sequence encoding the same antigenic peptide or protein of Respiratory syncytial virus (RSV), e.g. fusion protein F. In that embodiment the doses are given in a specific time period e.g. 20-30 days. For example for post-exposure prophylaxis at least 5 doses of the inventive mRNA sequence or inventive pharmaceutical composition or the vaccine disclosed herein can be administered in 20-30 days.

In a preferred embodiment, inventive mRNA sequence, inventive pharmaceutical composition or the vaccine disclosed herein is administered via intradermal or intramuscular injection, preferably by using conventional needle-based injection technique or by using a needle-free system, e.g. jet injection. In a further preferred embodiment, the inventive mRNA sequence, inventive pharmaceutical composition or the vaccine disclosed herein may be administered by jet injection as defined herein. Preferably, the inventive mRNA sequence, inventive pharmaceutical composition or the vaccine disclosed herein is adiminstered intramuscularly by jet injection. According to another embodiment, the inventive mRNA sequence, inventive pharmaceutical composition or the vaccine disclosed herein is administered intradermally via jet injection.

In a preferred embodiment, the inventive mRNA sequence, inventive pharmaceutical composition or the vaccine disclosed herein may be administered once, twice or three times, preferably by intradermal or intramuscular injection, preferably by jet injection. According to a certain embodiment, a single administration of the the inventive mRNA sequence, inventive pharmaceutical composition or the vaccine disclosed herein, preferably via intradermal or intramuscular injection, preferably by using jet injection, is sufficient for eliciting an immune response against the at least one antigen encoded by the mRNA sequence according to the invention. In a preferred embodiment, the single administration of the inventive mRNA sequence, inventive pharmaceutical composition or the vaccine disclosed herein elicits an immune response resulting in virus neutralisation. In this context, one single intradermal or intramuscular injection of the inventive mRNA sequence, inventive pharmaceutical composition or the vaccine disclosed herein is particularly preferred. Preferably, further administrations of the inventive mRNA sequence, inventive pharmaceutical composition or the vaccine disclosed herein may optionally be carried out in order to enhance and/or prolong the immune response.

In certain embodiments, such booster inoculations with the inventive mRNA sequence or inventive pharmaceutical composition or the vaccine disclosed herein may utilize an additional compound or component as defined for the inventive mRNA sequence or inventive pharmaceutical composition or the vaccine disclosed herein as defined herein.

According to a further aspect, disclosed herein is a non-claimed method for expression of an encoded antigenic peptide or protein derived from the fusion protein F, the glycoprotein G, the short hydrophobic protein SH, the matrix protein M, the nucleoprotein N, the large polymerase L, the M2-1 protein, the M2-2 protein, the phosphoprotein P, the non-structural protein NS1 or the non-structural protein NS2 of Respiratory syncytial virus (RSV) comprising the steps, e.g. a) providing the inventive mRNA sequence as defined herein or the inventive composition comprising a plurality of mRNA sequences as defined herein, b) applying or administering the inventive mRNA sequence as defined herein or the inventive composition comprising a plurality of mRNA sequences as defined herein to an expression system, e.g. to a cell-free expression system, a cell (e.g. an expression host cell or a somatic cell), a tissue or an organism. The method may be applied for laboratory, for research, for diagnostic, for commercial production of peptides or proteins and/or for therapeutic purposes. In this context, typically after preparing the inventive mRNA sequence as defined herein or of the inventive composition comprising a plurality of mRNA sequences as defined herein, it is typically applied or administered to a cell-free expression system, a cell (e.g. an expression host cell or a somatic cell), a tissue or an organism, e.g. in naked or complexed form or as a pharmaceutical composition or vaccine as described herein, preferably via transfection or by using any of the administration modes as described herein. The method may be carried out *in vitro, in vivo* or *ex vivo.* The method may furthermore be carried out in the context of the treatment of a specific disease, particularly in the treatment of infectious diseases, preferably RSV infections as defined herein.

In this context, *in vitro* is defined herein as transfection or transduction of the inventive mRNA as defined herein or of the inventive composition comprising a plurality of mRNA sequences as defined herein into cells in culture outside of an organism; *in vivo* is defined herein as transfection or transduction of the inventive mRNA or of the inventive composition comprising a plurality of mRNA sequences into cells by application of the inventive mRNA or of the inventive composition to the whole organism or individual and *ex vivo* is defined herein as transfection or transduction of the inventive mRNA or of the inventive composition comprising a plurality of mRNA sequences into cells outside of an organism or individual and subsequent application of the transfected cells to the organism or individual.

Likewise, according to another aspect, the present disclosure also provides the non-claimed use of the inventive mRNA sequence as defined herein or of the inventive composition comprising a plurality of mRNA sequences as defined herein, preferably for diagnostic or therapeutic purposes, for expression of an encoded antigenic peptide or protein, e.g. by applying or administering the inventive mRNA sequence as defined herein or of the inventive composition comprising a plurality of mRNA sequences as defined herein, e.g. to a cell-free expression system, a cell (e.g. an expression host cell or a somatic cell), a tissue or an organism. The use may be applied for laboratory, for research, for diagnostic for commercial production of peptides or proteins and/or for therapeutic purposes. In this context, typically after preparing the inventive mRNA sequence as defined herein or of the inventive composition comprising a plurality of mRNA sequences as defined herein, it is typically applied or administered to a cell-free expression system, a cell (e.g. an expression host cell or a somatic cell), a tissue or an organism, preferably in naked form or complexed form, or as a pharmaceutical composition or vaccine as described herein, preferably via transfection or by using any of the administration modes as described herein. The use may be carried out *in vitro, in vivo* or *ex vivo.* The use may furthermore be carried out in the context of the treatment of a specific disease, particularly in the treatment of RSV infections.

In a further aspect, the invention provides the inventive mRNA sequence, the composition comprising a plurality of mRNA sequences, the pharmaceutical composition or the kit or kit of parts comprising the inventive mRNA sequence for use in a method of treatment or prophlaxis of RSV infections comprising the steps:
a) providing the inventive mRNA sequence, the composition comprising a plurality of mRNA sequences as defined in the claims, the pharmaceutical composition or the kit or kit of parts comprising the inventive mRNA sequence as defined in the claims;
b) applying or administering the mRNA sequence, the composition, the pharmaceutical composition or the kit or kit of parts to a tissue or an organism;
c) optionally administering RSV immune globuline.

Taken together, the present disclosure provides in a certain aspect an mRNA sequence comprising a coding region as defined in the claims. The inventive mRNA sequence is for use in a method of prophylactic and/or therapeutic treatment of infections caused by syncytial virus (RSV). Accordingly, the invention relates to an mRNA sequence as defined herein for use in a method of prophylactic and/or therapeutic treatment of RSV infections.

### Brief description of the figures

The figures shown in the following are merely illustrative and shall describe the present invention in a further way. These figures shall not be construed to limit the present invention thereto.
- **Figure 1:**: G/C optimized mRNA sequence of R1691 coding for RSV-F protein of the RSV long strain (RSV-F long) as comprised in the RSV-F long mRNA vaccine (SEQ ID NO. 31).
- **Figure 2:**: G/C optimized mRNA sequence of R2510 coding for RSV-F protein of the RSV long strain (RSV-F long) as comprised in the RSV-F long mRNA vaccine (SEQ ID NO. 32).
- **Figure 3:**: G/C optimized mRNA sequence of R2821 coding for RSV-F del554-574 mutant protein of the RSV long strain (RSV-F long) (SEQ ID NO. 33).
- **Figure 4:**: G/C optimized mRNA sequence of R2831 coding for RSV-N protein of the RSV long strain (RSV-F long) (SEQ ID NO. 34).
- **Figure 5:**: G/C optimized mRNA sequence of R2833 coding for RSV-M₂₋₁ protein of the RSV long strain (RSV-F long) (SEQ ID NO. 35).

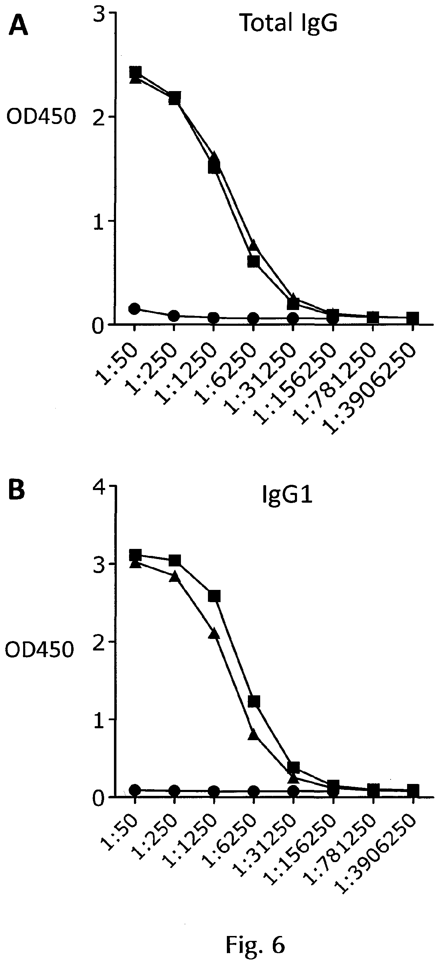

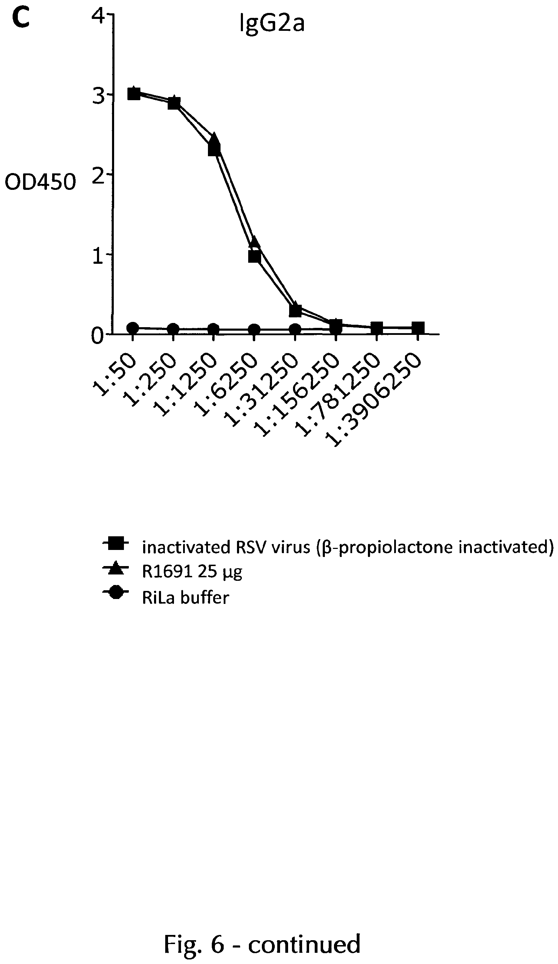

- **Figure 6:**: shows that the RSV-F long mRNA vaccine induces antibody titers against the RSV-F protein comparable to those against inactivated RSV. Female BALB/c mice were intradermally (i.d.) injected with the RSV-F long mRNA vaccine (160 µg of R1691) or Ringer-Lactate (RiLa buffer) as buffer control. One group was intramuscularly (i.m.) injected with 10 µg of the inactivated RSV long vaccine. All animals received boost injections on days 21 and 35, blood samples were collected on day 49 for the determination of antibody titers of pooled sera as described in Example 2. As can be seen, the RSV-F long mRNA vaccine induces anti-F protein antibodies of the IgG1 and IgG2a subclasses. Antibody titers are displayed in the graph (n=5 mice/group).
- **Figure 7:**: shows that the RSV-F long mRNA vaccine (R1691) induces a long-lived immune response in mice. The experiment was performed as described in Example 3 and antibody total IgG titers determined by ELISA. As can be seen, the antibody titers are stable for at least eleven months after the last boost vaccination.
- **Figure 8:**: shows that the RSV-F long mRNA vaccine (R1691) induces RSV Fusion (F) protein-specific multifunctional CD8⁺ T cells in mice.
The experiment was performed as described in Example 4 and T cells were analysed by intracellular cytokine staining for the antigen-specific induction of cytokines. Cells were stimulated with a RSV-F peptide (stim. F; KYKNAVTEL) or a control influenza HA peptide (stim. HA; IYSTVASSL). The line in the graph represents the median value (n=5 mice/group).
As can be seen, the RSV-F long mRNA vaccine induces RSV Fusion (F) protein-specific multifunctional CD8⁺ T cells in contrast to the vaccine based on inactivated RSV which is not able to induce F protein-specific CD8⁺ T cells.

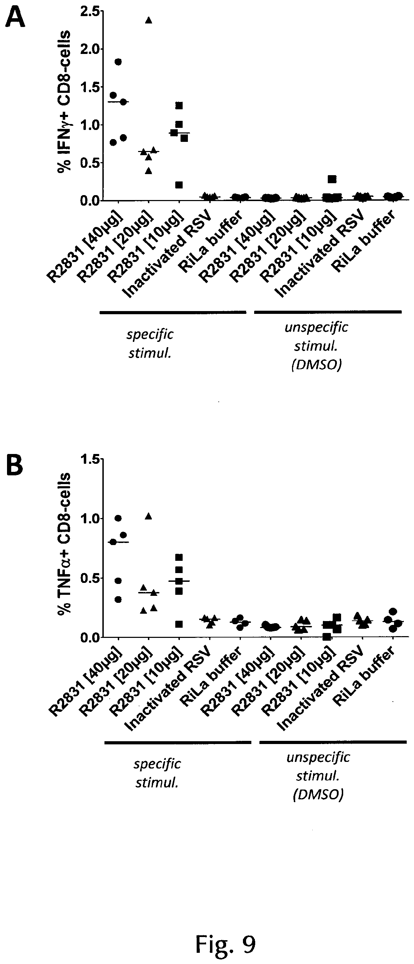

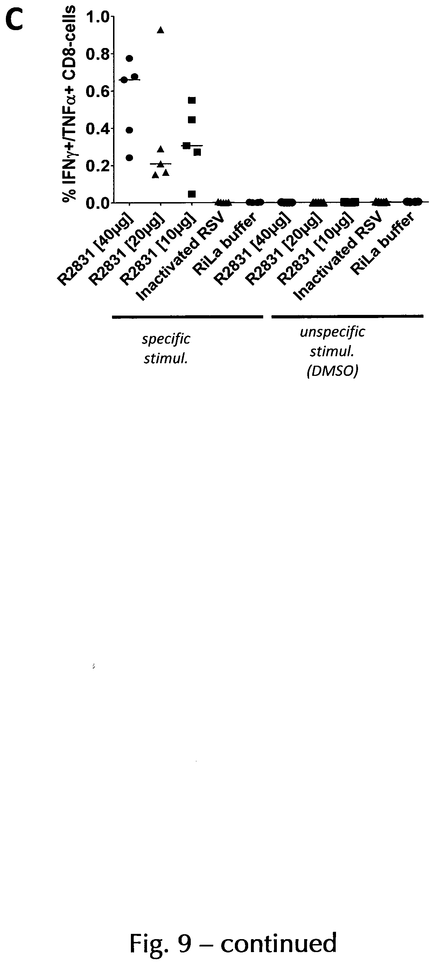

- **Figure 9:**: shows that the RSV-N mRNA vaccine (R2831) induces Nucleoprotein (N)-specific multifunctional CD8⁺ T cells in mice.
The experiment was performed as described in Example 5 and T cells were analysed by intracellular cytokine staining for the antigen-specific induction of cytokines after stimulation with ProMix RSV-N (15mer peptides). The line in the graph represents the median value (n=5 mice/group).
As can be seen, the RSV-N mRNA vaccine induces RSV Nucleoprotein (N)-specific multifunctional CD8⁺ T cells in contrast to the vaccine based on inactivated RSV which is not able to induce N protein-specific CD8⁺ T cells.

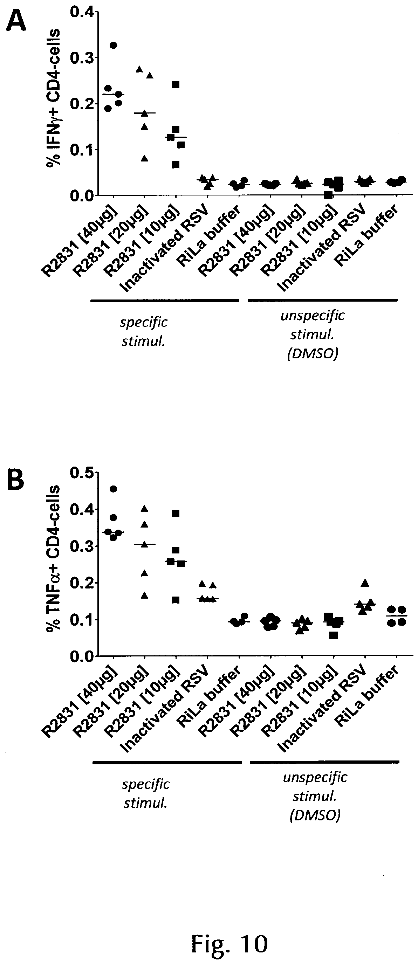

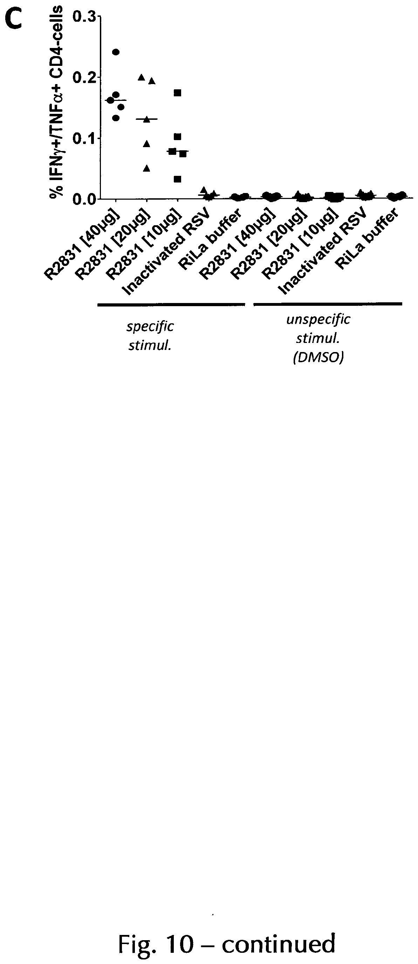

- **Figure 10:**: shows that the RSV-N mRNA vaccine (R2831) induces Nucleoprotein (N)-specific multifunctional CD4⁺ T cells in mice.
The experiment was performed as described in Example 5 and T cells were analysed by intracellular cytokine staining for the antigen-specific induction of cytokines after stimulation with ProMix RSV-N (15mer peptides). The line in the graph represents the median value (n=5 mice/group).
As can be seen, the RSV-N mRNA vaccine induces RSV Nucleoprotein (N)-specific multifunctional CD4⁺ T cells in contrast to the vaccine based on inactivated RSV which is not able to induce N protein-specific CD4⁺ T cells.

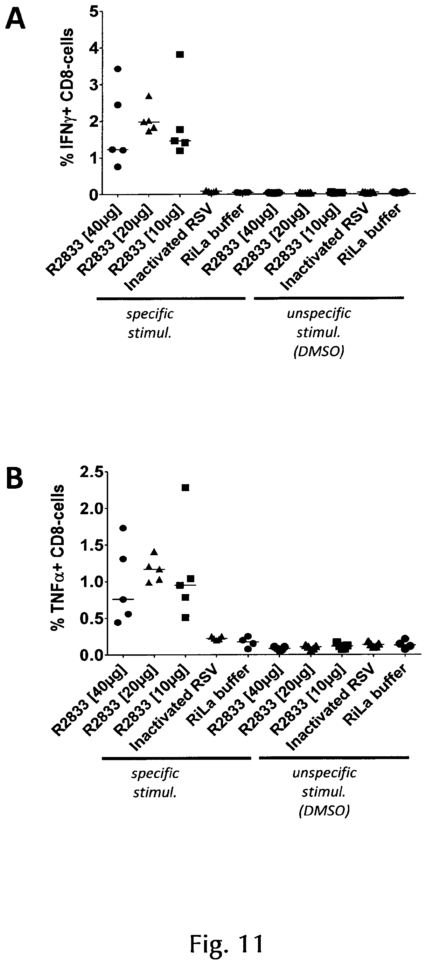

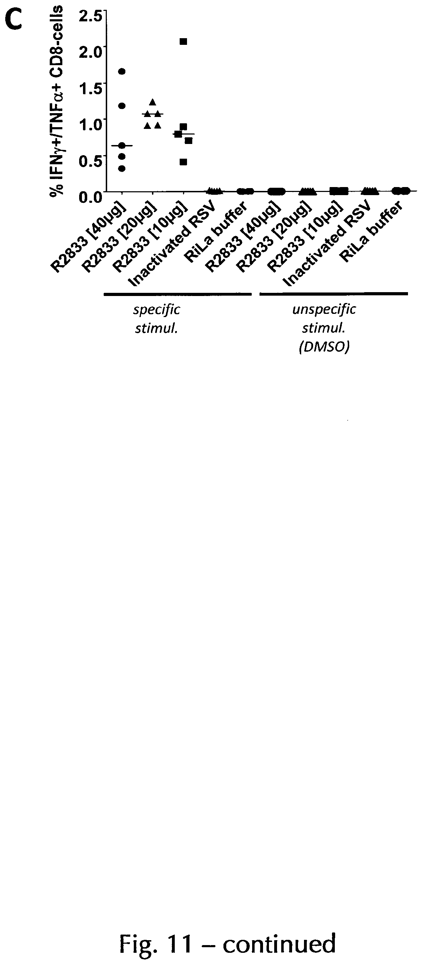

- **Figure 11:**: shows that the RSV-M₂₋₁ mRNA vaccine (R2833) induces M₂₋₁-specific multifunctional CD8⁺ T cells in mice.
The experiment was performed as described in Example 5 and T cells were analysed by intracellular cytokine staining for the antigen-specific induction of cytokines after stimulation with a M₂₋₁ specific 9mer peptide. The line in the graph represents the median value (n=5 mice/group).
As can be seen, the RSV-M₂₋₁ mRNA vaccine induces RSV RSV-M₂₋₁-specific multifunctional CD8⁺ T cells in contrast to the vaccine based on inactivated RSV which is not able to induce M₂₋₁ protein-specific CD8⁺ T cells.

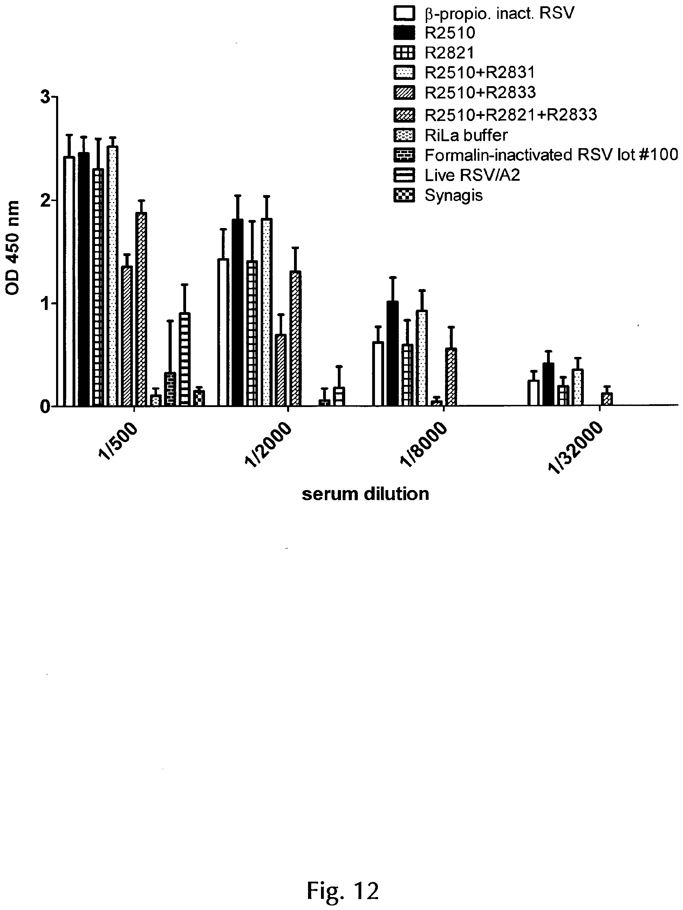

- **Figure 12:**: shows that RSV-F mRNA vaccines either alone (RSV-F = R2510; RSV-Fdel554-574 mutant = R2821) or in combination with mRNAs encoding other RSV proteins (RSV-N = R2831; RSV-M₂₋₁ = R2833) induce humoral immune responses in cotton rats.
The experiment was performed as described in Example 6 and RSV-F specific total IgG antibody titers were determined by ELISA on day 49. Serum was analyzed in different dilution, as given below the graph.

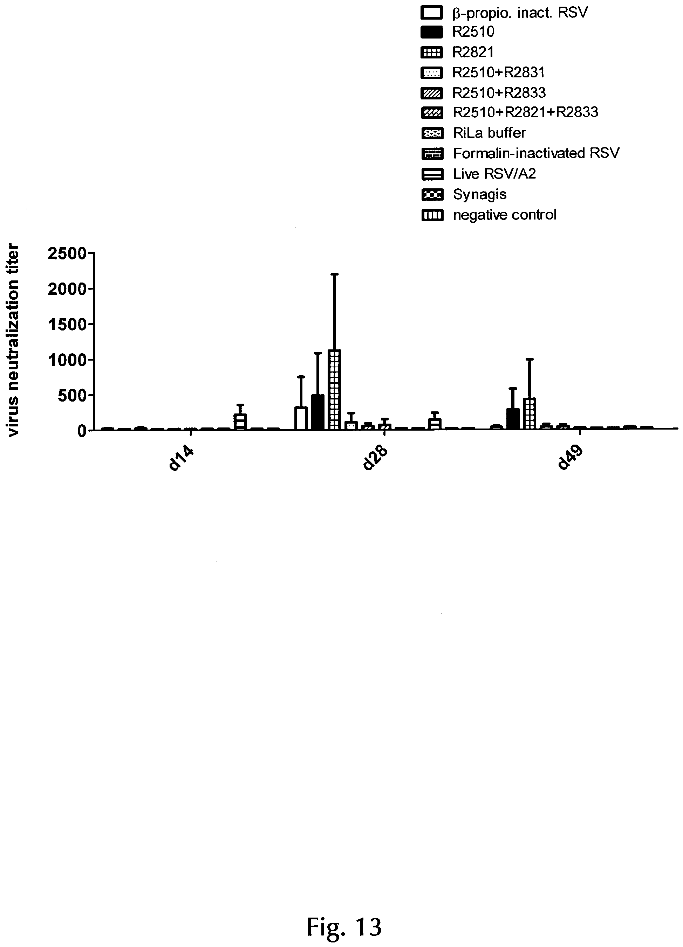

- **Figure 13:**: shows that RSV-F mRNA vaccines either alone (RSV-F, R2510; RSV-Fdel554-574 mutant, R2821) or in combination with mRNAs encoding other RSV proteins (RSV-N = R2831; RSV-M₂₋₁ = R2833) induce the formation of functional antibodies in cotton rats as shown by virus neutralizing antibody titers.
The experiment was performed as described in Example 6 and virus neutralizing titers on day 49 were determined by plaque reduction assay.

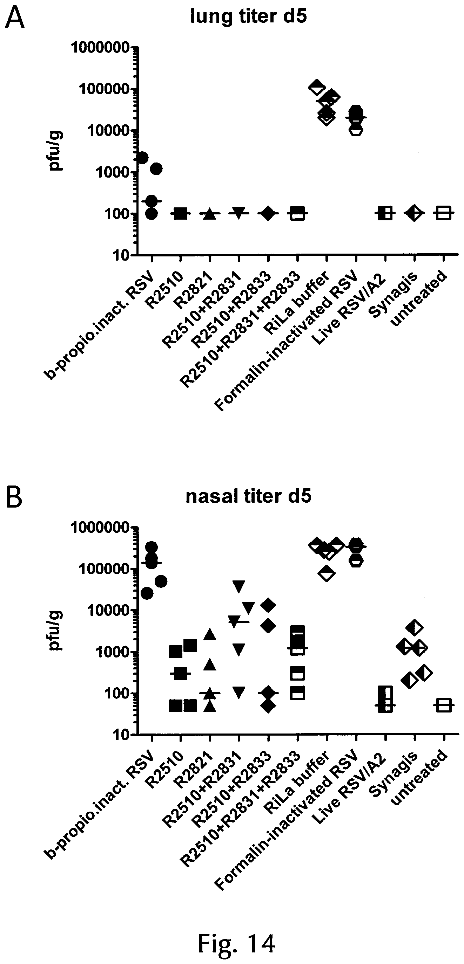

- **Figure 14:**: shows that RSV-F mRNA vaccines either alone (RSV-F = R2510; RSV-Fdel554-574 mutant = R2821) or in combination with mRNAs encoding other RSV proteins (RSV-N = R2831; RSV-M₂₋₁ = R2833) reduce lung and nasal titers in cotton rats challenged with RSV virus.
The experiment was performed as described in Example 6.
(A) Lung titers on day 5 after RSV challenge infection. All animal groups vaccinated with mRNA vaccines showed virus titers below the level of detection of the performed virus titration demonstrating protection of vaccinated cotton rats in terms of viral lung titers. In comparison to the mRNA vaccines the vaccine based on formalin-inactivated virus were not able to prevent virus titers in the lung.
(B) Nasal titers on day 5 after RSV challenge infection. The viral titer in the nasal tissue was also strongly reduced in groups vaccinated with mRNA. In comparison to the mRNA vaccines the vaccine based on formalin-inactivated virus were not able to reduce the nasal virus titers.

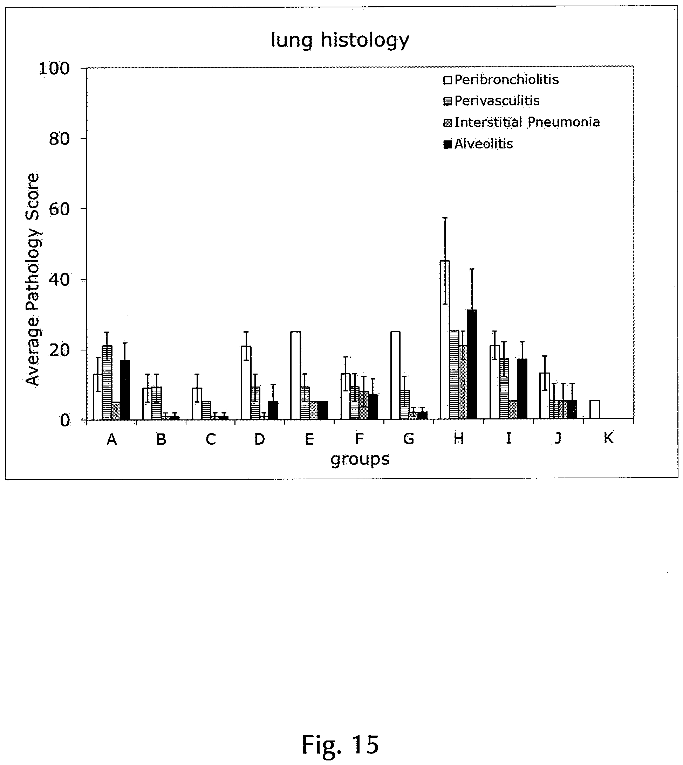

- **Figure 15:**: shows the results of the lung histopathology analysis from the RSV cotton rat challenge study described in Example 6.

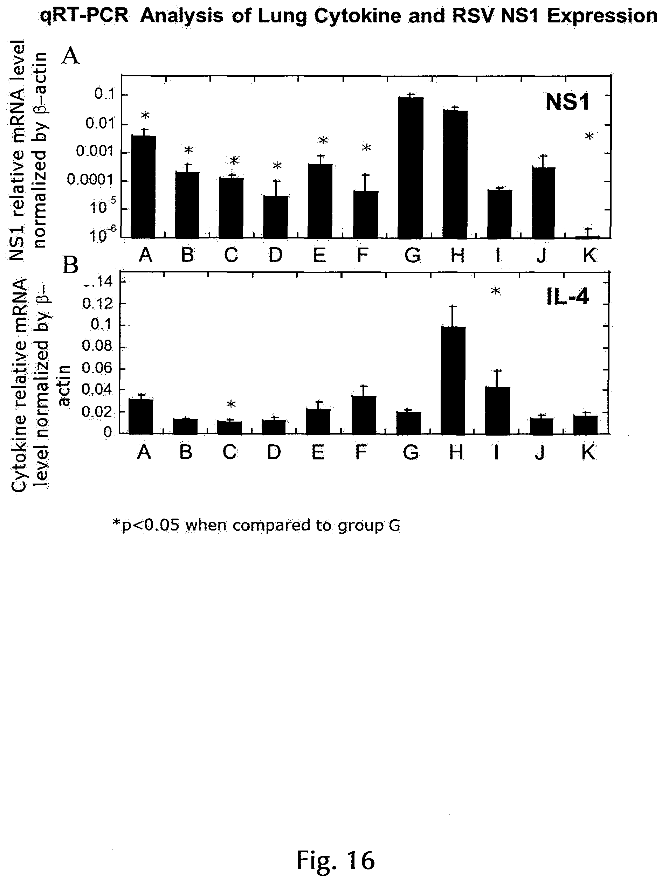

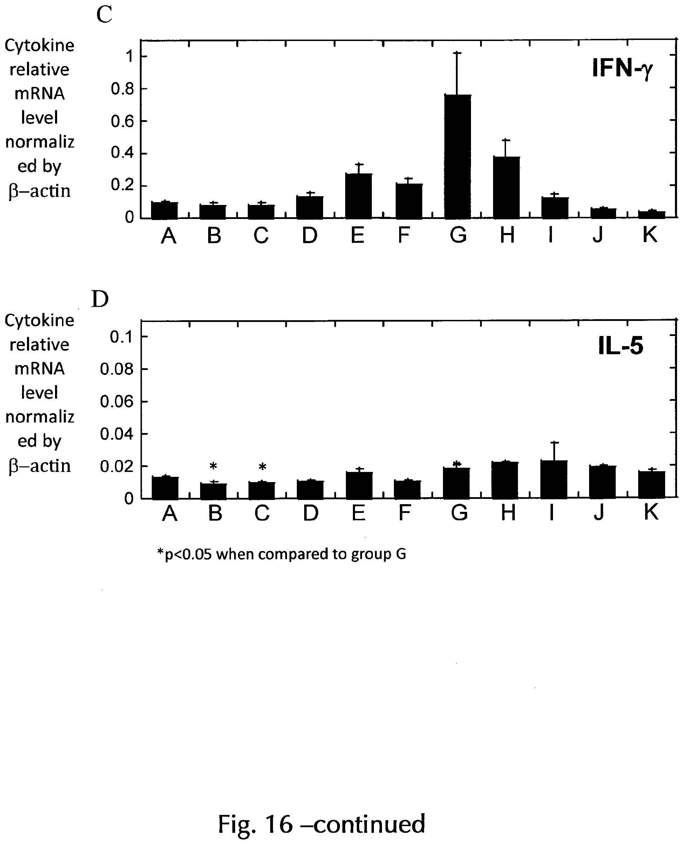

- **Figure 16:**: shows the results of the quantitative reverse transcription polymerase chain reaction (RT-PCR) of viral genome copy numbers (by measuring copy numbers of the RSV NS-1 gene) or expressed cytokines from lung tissue of the RSV infected animals (or controls) of the RSV cotton rat challenge study described in Example 6.

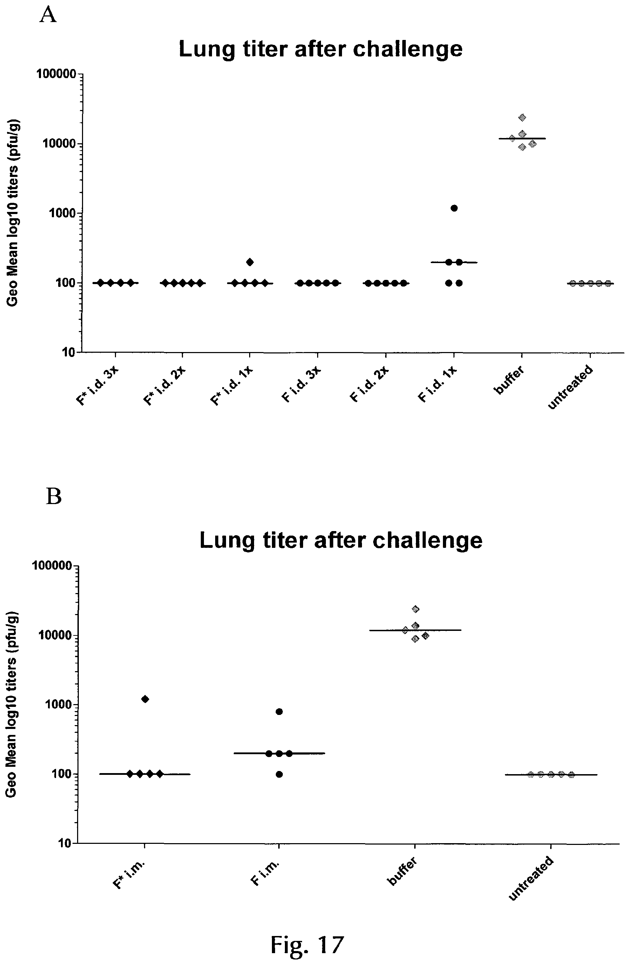

- **Figure 17:**: shows that RSV-F mRNA vaccines (RSV-F = R2510; RSV F^{∗} (RSV-Fdel554-574 mutant) = R2821) reduce lung titers in cotton rats challenged with RSV virus. The experiment was performed as described in Example 7.
(A) Lung titers on day 5 after RSV challenge infection. All animal groups vaccinated intradermally with mRNA vaccines showed reduced virus titers compared to the buffer control group demonstrating protection of vaccinated cotton rats in terms of viral lung titers. Already one single dose of RSV-F mRNA vaccines efficiently reduced viral titers in the lung.
(B) Lung titers on day 5 after RSV challenge infection. The viral titer in the lung was also strongly reduced in groups intramuscularly vaccinated with mRNA.

### Examples

The examples shown in the following are merely illustrative and shall describe the present invention in a further way. These examples shall not be construed to limit the present invention thereto.

### Example 1: Preparation of the mRNA vaccine

### 1. Preparation of DNA and mRNA constructs

For the present examples DNA sequences, encoding the RSV-F protein (R1691 and R2510), the RSV-F del554-574 (R2821) mutant protein, the RSV N-protein (R2831) and the RSV M2-1 protein (R2833) of the RSV long strain were prepared and used for subsequent in vitro transcription reactions. The RSV-Fdel554-574 mutant protein has been described previously (Oomens et al. 2006. J. Virol. 80(21):10465-77).

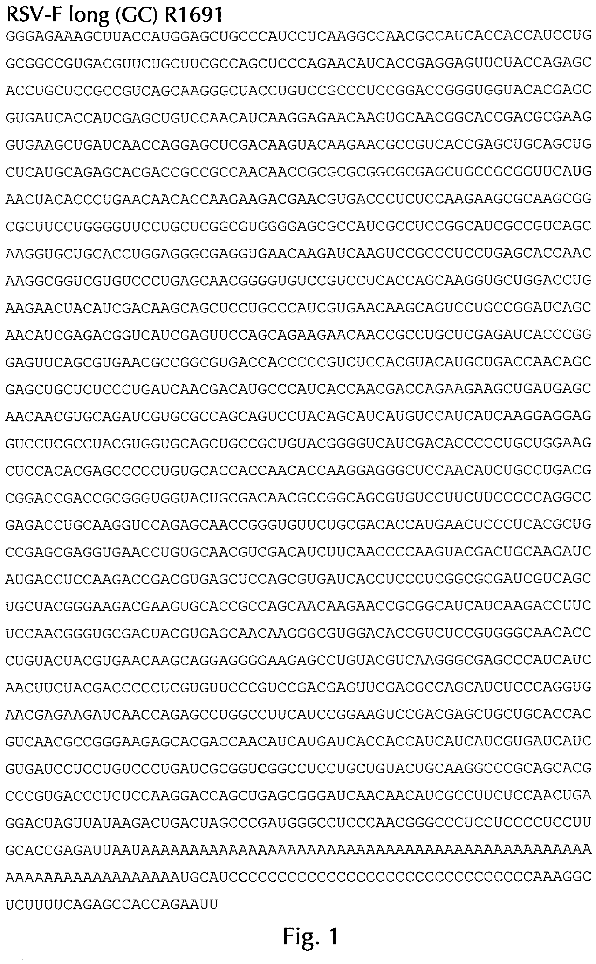

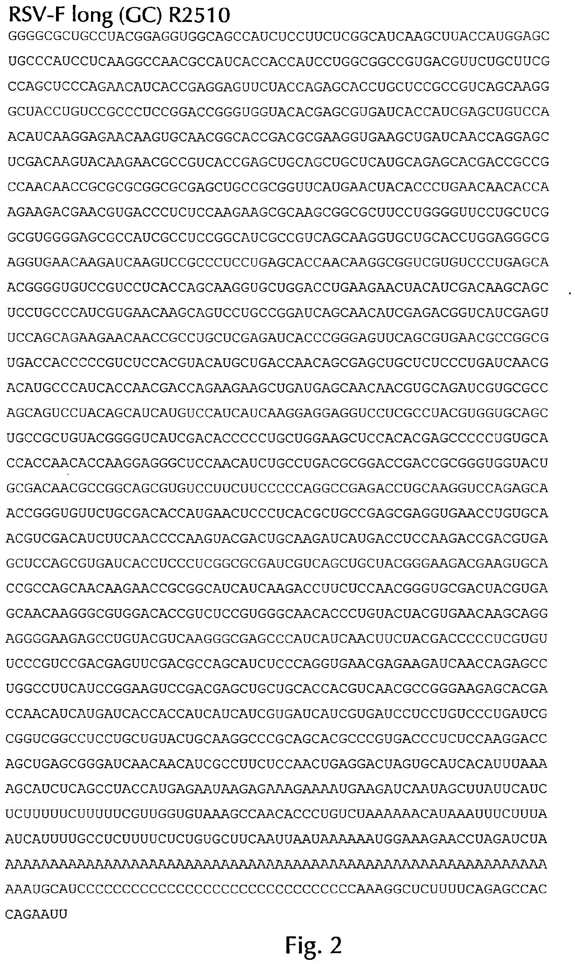

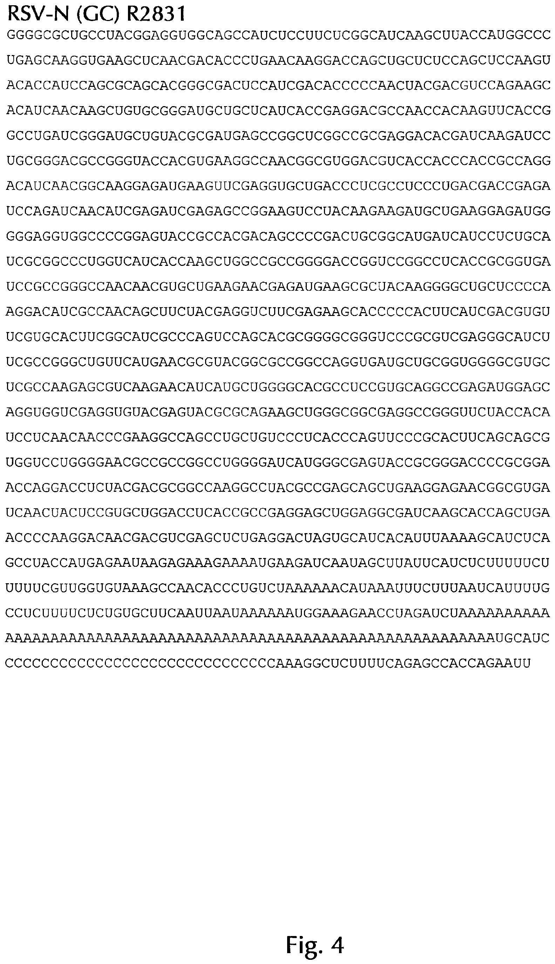

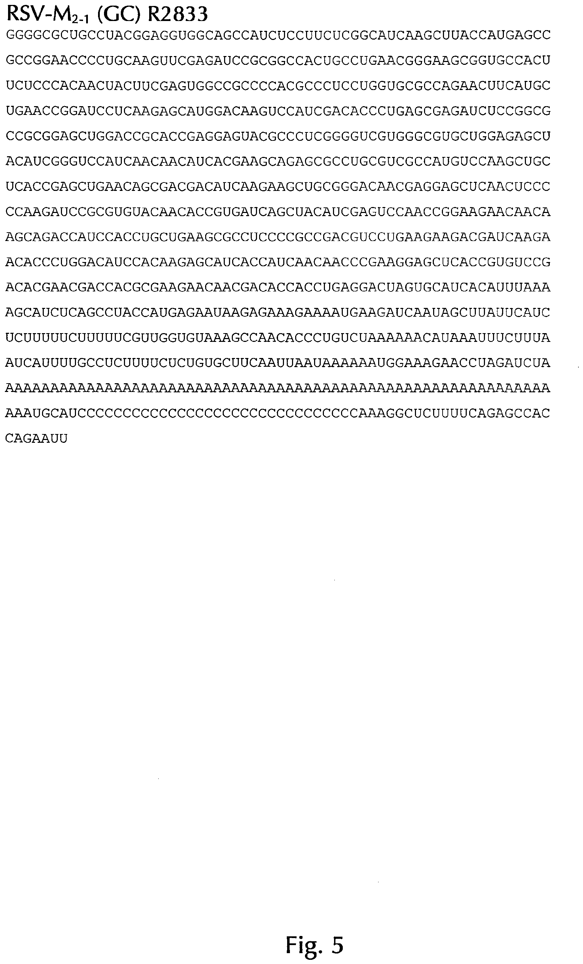

According to a first preparation, the DNA sequences coding for the above mentioned mRNAs were prepared. The construct R1691 was prepared by modifying the wild type coding sequence by introducing a GC-optimized sequence for stabilization, followed by a stabilizing sequence derived from the alpha-globin-3'-UTR (muag (mutated alpha-globin-3'-UTR) according to SEQ ID No. 29), a stretch of 64 adenosines (poly(A)-sequence), a stretch of 30 cytosines (poly(C)-sequence), and a histone stem loop according to SEQ ID No. 30. In SEQ ID NO: 31 (see Figure 1) the sequence of the corresponding mRNA is shown. The constructs R2510, R2821, R2831 and R2833 were prepared by introducing a 5'-TOP-UTR derived from the ribosomal protein 32L according to SEQ ID No. 23, modifying the wild type coding sequence by introducing a GC-optimized sequence for stabilization, followed by a stabilizing sequence derived from the albumin-3'-UTR (albumin7 according to SEQ ID No. 25), a stretch of 64 adenosines (poly(A)-sequence), a stretch of 30 cytosines (poly(C)-sequence), and a histone stem loop according to SEQ ID No. 30. In SEQ ID NOs: 32-35 (see Figure 2-5) the sequences of the corresponding mRNAs are shown.

**Table 1: mRNA constructs**

| **RNA** | **Antigen** | **Figure** | **SEQ ID NO.** |
|---|---|---|---|
| R1691 | RSV F | 1 | SEQ ID NO. 31 |
| R2510 | RSV F | 2 | SEQ ID NO. 32 |
| R2821 | RSV Fdel554-574 | 3 | SEQ ID NO. 33 |
| R2831 | RSV N | 4 | SEQ ID NO. 34 |
| R2833 | RSV M₂₋₁ | 5 | SEQ ID NO. 35 |

### 2. In vitro transcription

The respective DNA plasmids prepared according to paragraph 1 were transcribed *in vitro* using T7 polymerase in the presence of a CAP analog (m⁷GpppG). Subsequently the mRNA was purified using PureMessenger® (CureVac, Tübingen, Germany; WO2008/077592A1).

### 3. Reagents

Complexation Reagent: protamine

### 4. Preparation of the vaccine

The mRNA was complexed with protamine by addition of protamine to the mRNA in the ratio (1:2) (w/w) (adjuvant component). After incubation for 10 minutes, the same amount of free mRNA used as antigen-providing RNA was added.

For example: RSV-F long vaccine (R1691): comprising an adjuvant component consisting of mRNA coding for RSV F protein long (R1691) according to SEQ ID NO. 31 complexed with protamine in a ratio of 2:1 (w/w) and the antigen-providing free mRNA coding for RSV F protein long (R1691) according to SEQ ID NO. 31 (ratio 1:1; complexed RNA:free RNA).

### Example 2: Induction of a humoral immune response by the RSV-F long mRNA vaccine in mice

### Immunization

On day zero, BALB/c mice were intradermally (i.d.) injected with the RSV-F long mRNA vaccine (R1691 according to Example 1; 25 µg/mouse/vaccination day) or Ringer-lactate (RiLa) as buffer control as shown in Table 2. A control group was intramuscularly (i.m.) injected with 10 µg of the inactivated RSV long vaccine. The inactivated "Respiratory Syncytial Virus Antigen" (inactivated RSV long) was purchased from the INSTITUT VIRION/SERION GmbH-SERION IMMUNDIAGNOSTICA GmbH. The inactivated virus was diluted in sterile PBS, so that a final concentration of 0.2 µg/µL was achieved. All animals received boost injections on day 14 and day 28. Blood samples were collected on day 42 for the determination of anti-RSV F antibody titers.

**Table 2: Animal groups**

| **Group** | **Strain sex** | **No. mice** | **Route volume** | **Vaccine dose** | **Vaccination schedule** |
|---|---|---|---|---|---|
| 1 | BALB/c Female | 5 | i.d. | R1691 | d0: prime, d14: boost, d28: boost, |
| | | | 2x50 µl | 2 µg | d42: blood collection |
| 2 | BALB/c Female | 5 | i.m. | Inactivated RSV long | d0: prime, d14: boost, d28: boost, |
| | | | 2x25 µl | 10 µg | d42: blood collection |
| 3 | BALB/c Female | 5 | i.d. | 80% Ringer Lactate (RiLa) buffer | d0: prime, d14: boost, d28: boost, |
| | | | 2x50 µl | | d42: blood collection |

### Determination of anti-RSV F protein antibodies by ELISA

ELISA plates are coated with recombinant human RSV fusion glycoprotein (rec.hu F-protein, final conc.: 5 µg/mL) (Sino Biological Inc.). Coated plates are incubated using given serum dilutions and binding of specific antibodies to the F protein is detected using biotinylated isotype specific anti-mouse antibodies in combination with streptavidin-HRP (horse radish peroxidase) with ABTS substrate.

### Results

As can be seen in Figure 6, the RSV-F long mRNA vaccine induces antibody titers (total IgG, IgG1 and IgG2a) against the RSV F protein comparable to those against inactivated RSV.

### Example 3: Induction of a long-lived humoral immune response by the RSV-F long mRNA vaccine in mice

### Immunization

BALB/c mice were intradermally (i.d.) injected with 20 µg of the RSV-F long mRNA vaccine (R1691) or Ringer Lactate (RiLa) buffer according to the vaccination schedule shown in Table 3.
Blood was collected 2 weeks, 4 months and 11 months after the last immunization.

**Table 3: Animal groups**

| **Group** | **Strain sex** | **Number of mice** | **Route volume** | **Vaccine dose** | **Vaccination schedule (day)** |
|---|---|---|---|---|---|
| 1 | BALB/c Female | 5 | i.d. | R1691 | d0, d14, d28 |
| | | | 100 µl | 20 µg | |
| 4 | BALB/c Female | 5 | i.d. | 80% RiLa buffer | d0, d14, d28 |
| | | | 100 µl | | |

### Results

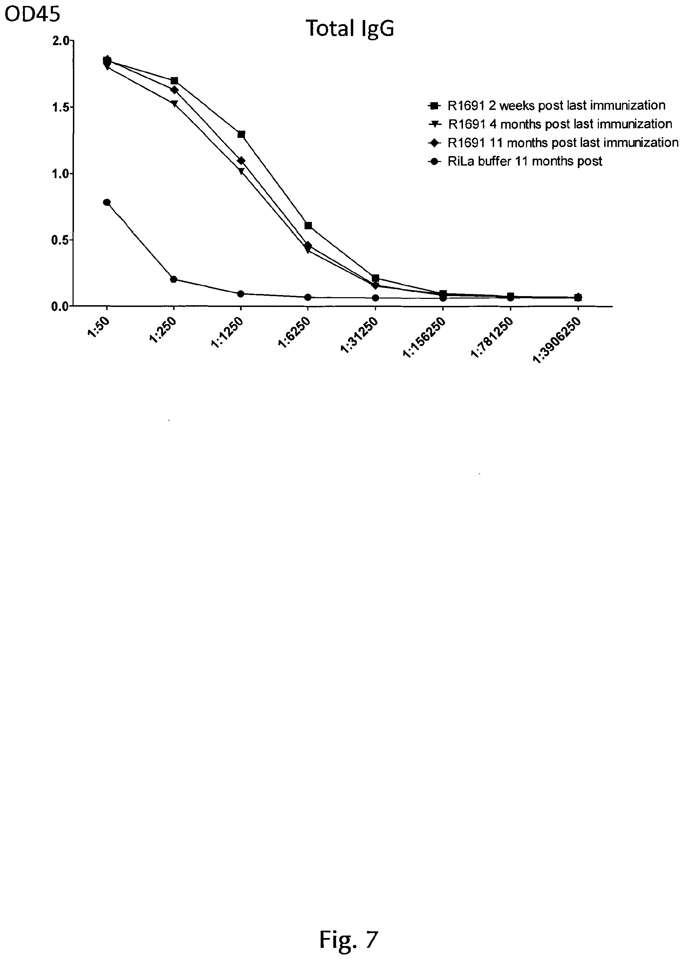

As can be seen in Figure 7, the RSV-F long mRNA vaccine induced a long-lived immune response as demonstrated by stable antibody titers for at least 11 months after the last boost vaccination.

### Example 4: Induction of a cellular immune response by the RSV-F long mRNA vaccine in mice

### Immunization

On day zero, BALB/c mice were intradermally (i.d.) injected with the RSV-F long mRNA vaccine R1691 (20 µg/mouse/vaccination day) or Ringer-lactate (RiLa) as buffer control as shown in Table 4. A control group was intramuscularly (i.m.) injected with 10 µg of the inactivated RSV long vaccine. The inactivated "Respiratory Syncytial Virus Antigen" (inactivated RSV long) was purchased from the INSTITUT VIRION/SERION GmbH-SERION IMMUNDIAGNOSTICA GmbH. The inactivated virus was diluted in sterile PBS, so that a final concentration of 0.2 µg/µL was achieved.

All animals received boost injections on days 14 and 28. Spleens were collected on day 34 for the analysis of antigen-specific T cells.

**Table 4: Animal groups**

| **Group** | **Strain sex** | **Number of mice** | **Route volume** | **Vaccine dose** | **Vaccination schedule** |
|---|---|---|---|---|---|
| 1 | BALB/c Female | 5 | i.d. | R1691 | d0: prime, d14: boost, d28: boost, |
| | | | 2x 50 µl | 20 µg | d34: spleen collection |
| 2 | BALB/c Female | 5 | i.m. | Inactivated RSV long | d0: prime, d14: boost, d28: boost, |
| | | | 2x 25 µl | 10 µg | d34: spleen collection |
| 3 | BALB/c Female | 5 | i.d. | Ringer Lactate (RiLa) buffer | d0: prime, d14: boost, d28: boost, |
| | | | 2x 50 µl | | d34: spleen collection |

### Intracellular cytokine staining

Splenocytes from vaccinated and control mice were isolated according to a standard protocol. Briefly, isolated spleens were grinded through a cell strainer and washed in PBS/1%FBS followed by red blood cell lysis. After an extensive washing step with PBS/1%FBS splenocytes were seeded into 96-well plates (2x10⁶ cells/well). The next day cells were stimulated with a RSV-F peptide (KYKNAVTEL; 5 µg/ml; H-2kd-restructed T-cell epitope) or an irrelevant control peptide derived from the influenza HA protein (IYSTVASSL; 5 µg/ml; purchased from EMC Microcollections) and 2.5 µg/ml of an anti-CD28 antibody (BD Biosciences) for 6 hours at 37°C in the presence of the mixture of GolgiPlug™/Golgistop™ (Protein transport inhibitors containing Brefeldin A and Monensin, respectively; BD Biosciences). After stimulation cells were washed and stained for intracellular cytokines using the Cytofix/Cytoperm reagent (BD Biosciences) according to the manufacturer's instructions. The following antibodies were used for staining: CD8-PECy7 (1:200), CD3-FITC (1:200), IL2-PerCP-Cy5.5 (1:100), TNFα-PE (1:100), IFNγ-APC (1:100) (eBioscience), CD4-BD Horizon V450 (1:200) (BD Biosciences) and incubated with Fcγ-block diluted 1:100. Aqua Dye was used to distinguish live/dead cells (Invitrogen). Cells were collected using a Canto II flow cytometer (Beckton Dickinson). Flow cytometry data were analysed using FlowJo software (Tree Star, Inc.). Statistical analysis was performed using GraphPad Prism software, Version 5.01. Statistical differences between groups were assessed by the Mann Whitney test.

### Results

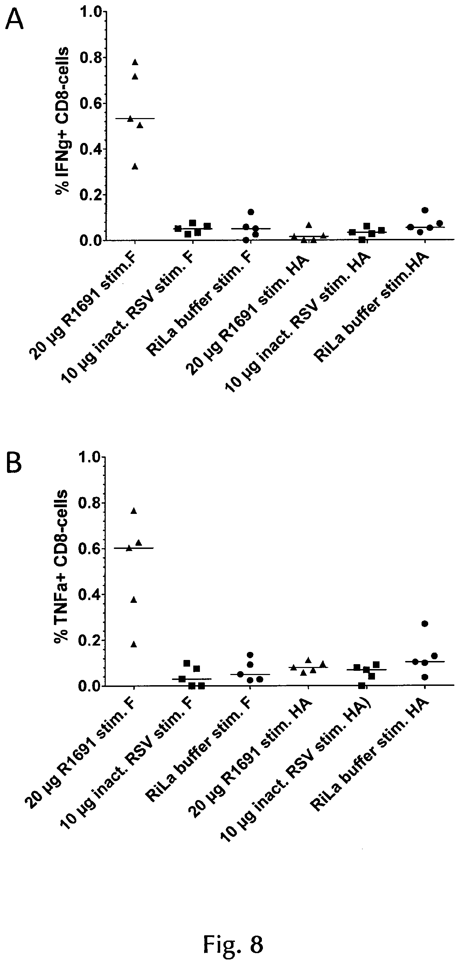

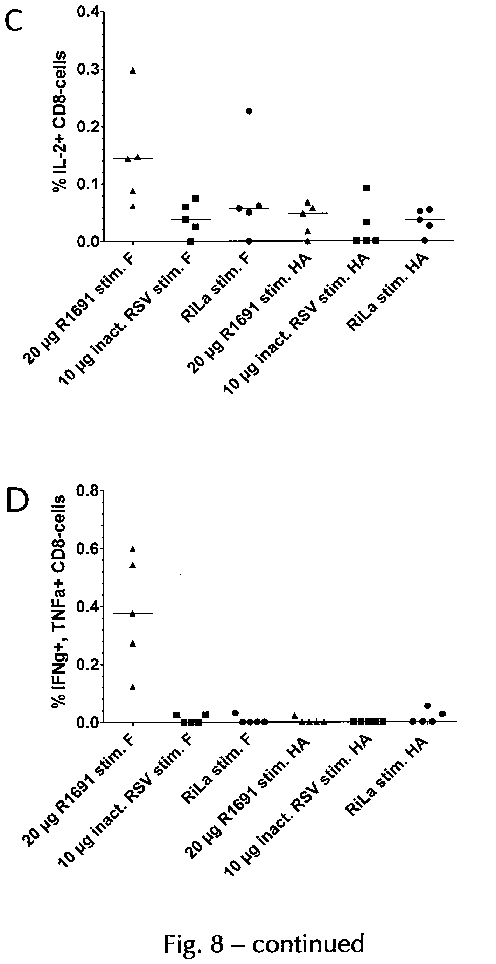

As can be seen from Figure 8, the RSV-F long mRNA vaccine (R1691) induced IFNγ positive, TNFα positive and IFNγ/TNFα double-positive multifunctional CD8⁺ T cells directed against RSV F protein. Surprisingly the vaccine based on inactivated RSV virus was not able to induce antigen-specific CD8⁺ T cells.

### Example 5: Induction of cellular immune responses by the RSV-N and RSV-M₂₋₁ mRNA vaccines in mice

### Immunization

On day zero, BALB/c mice were intradermally (i.d.) injected with different doses of the RSV-N mRNA vaccine R2831, the RSV-M₂₋₁ mRNA vaccine R2833 or Ringer-lactate (RiLa) as buffer control as shown in Table 5. A control group was intramuscularly (i.m.) injected with 10 µg of the inactivated RSV long vaccine. The inactivated "Respiratory Syncytial Virus Antigen" (inactivated RSV long) was purchased from the INSTITUT VIRION/SERION GmbH-SERION IMMUNDIAGNOSTICA GmbH. The inactivated virus was diluted in sterile PBS, so that a final concentration of 0.2 µg/µL was achieved. All animals received boost injections on days 7 and 21. Spleens were collected on day 27 for the analysis of antigen-specific T cells.

**Table 5: Animal groups**

| **Group** | **Strain sex** | **No. mice** | **Route volume** | **Vaccine dose** | **Vaccination schedule** |
|---|---|---|---|---|---|
| 1 | BALB/c Female | 5 | i.d. | R2831 RSV-N | d0: prime, d7: boost, d21: boost, |
| | | | 1x 50 µl | 40 µg | d27: spleen collection |
| 2 | BALB/c Female | 5 | i.d. | R2831 RSV-N | d0: prime, d7: boost, d21: boost, |
| | | | 1x 50 µl | 20 µg | d27: spleen collection |
| 3 | BALB/c Female | 5 | i.d. | R2831 RSV-N | d0: prime, d7: boost, d21: boost, |
| | | | 1x 50 µl | 10 µg | d27: spleen collection |
| 4 | BALB/c Female | 5 | i.d. | R2833 RSV-M₂₋₁ | d0: prime, d7: boost, d21: boost, |
| | | | 1x 50 µl | 40 µg | d27: spleen collection |
| 5 | BALB/c Female | 5 | i.d. | R2833 RSV-M₂₋₁ | d0: prime, d7: boost, d21: boost, |
| | | | 1x 50 µl | 20 µg | d27: spleen collection |
| 6 | BALB/c Female | 5 | i.d. | R2833 RSV-M₂₋₁ | d0: prime, d7: boost, d21: boost, |
| | | | 1x 50 µl | 10 µg | d27: spleen collection |
| 7 | BALB/c Female | 5 | i.m. | Inactiv. RSV long | d0: prime, d7: boost, d21: boost, |
| | | | 1x 50 µl | 10 µg | d27: spleen collection |
| 8 | BALB/c Female | 5 | i.d. | 100% RiLa buffer | d0: prime, d7: boost, d21: boost, |
| | | | 1x 50 µl | | d27: spleen collection |

Intracellular cytokine staining was performed as described in Example 4 except that cells were treated with the following stimulators at:
M2-1 peptide (5µg/ml) group 4 to 8; (SYIGSINNI from Prolmmune); ProMix N (1 µg/ml) 1-3, group 7 and 8; (PX39 from Proimmune); control: medium+ DMSO + anti-CD28, group 1-8 as descripted above.

### Results

As can be seen from Figure 9, the RSV-N mRNA vaccine (R2831) induced IFNγ positive, TNFα positive and IFNγ/TNFα double-positive multifunctional CD8⁺ T cells directed against RSV N protein in mice.

Surprisingly the vaccine based on inactivated RSV virus was not able to induce antigen-specific CD8⁺ T cells.

As can be seen from Figure 10, the RSV-N mRNA vaccine (R2831) induced IFNγ positive, TNFα positive and IFNγ/TNFα double-positive multifunctional CD4⁺ T cells directed against RSV N protein in mice.

Surprisingly the vaccine based on inactivated RSV virus was not able to induce antigen-specific CD4⁺ T cells.

As can be seen from Figure 11, the RSV-M₂₋₁ mRNA vaccine (R2833) induced IFNγ positive, TNFα positive and IFNγ/TNFα double-positive multifunctional CD8⁺ T cells directed against RSV M₂₋₁ protein in mice.

Surprisingly the vaccine based on inactivated RSV virus was not able to induce antigen-specific CD8⁺ T cells.

### Example 6: RSV cotton rat challenge study I

For the development of RSV vaccines the cotton rat is an accepted animal model, especially for the challenge infection. Cotton rats respond to formalin-inactivated RSV virus vaccine preparations with enhanced lung pathology. This allows the evaluation of the safety of a vaccination in terms of enhanced disease phenomenon.

To broaden and optimize the RSV-specific immune response, mRNA vaccines encoding different RSV proteins (RSV F, mutant RSV-Fdel554-574, N and M₂₋₁) were prepared according to Example 1. In order to assess the effect of single or combined vaccines, these vaccines were administered either alone or in combination (cocktail vaccine) as shown in Table 5. Vaccine volumes of 2x50µl were injected intradermally (i.d.) into the back skin of cotton rats. Additional groups were immunized intramuscularly (i.m.) with β-propiolactone inactivated RSV (INSTITUT VIRION/SERION GmbH-SERION IMMUNDIAGNOSTICA GmbH), formalin-inactivated RSV (Sigmovir) or live RSV/A2 (Sigmovir) (10⁵ plaque forming units, pfu) to compare their immunogenicity to mRNA vaccines. Another group received i.m. injections of the monoclonal anti-RSV antibody SYNAGIS® (Palivizumab) as passive immunization. SYNAGIS ® was administered with a dose of 15 mg/kg on the day prior to RSV challenge infection. Therefore the animals were weighed and the appropriate amount of SYNAGIS ® was calculated according to the animals' weight. The maximal volume for i.m. injection was 200 µl per 100g rat. After immunization the cotton rats were challenged by intranasal (i.n.) infection with RSV/A2 virus (10⁵ PFU in 100 µl; Sigmovir).

**Table 5: Animal groups**

| **Groups** | **Vaccine dose** | **Volume** | **Antigen** | **Route** | **# of administrations** | **N per group** | **Vaccination (day)** | **Bleed (day)** |
|---|---|---|---|---|---|---|---|---|
| A | β-propiolactone inactivated RSV | 100 µl | -- | IM | 3 | 5 | 0,14,28 | 14,28,49 |
| | 20 µg | | | | | | | |
| B | R2510 | 2x50 µl | RSV F | ID | 3 | 5 | 0,14,28 | 14,28,49 |
| | 80 µg | | | | | | | |
| C | R2821 | 2x50 µl | RSV F mutant | ID | 3 | 5 | 0,14,28 | 14,28,49 |
| | 80 µg | | | | | | | |
| D | R2510 + R2831 "cocktail I" | 2x50 µl | RSVF+ RSV N | ID | 3 | 5 | 0,14,28 | 14,28,49 |
| | each 40 µg | | | | | | | |
| E | R2510 + R2833 "cocktail II" | 2x50 µl | RSV F+ RSV M₂₋₁ | ID | 3 | 5 | 0,14,28 | 14,28,49 |
| | each 40 µg | | | | | | | |
| F | R2510+ R2831+R2833 "cocktail III" | 2x50 µl | RSV F+ RSV M₂₋₁ + RSV N | ID | 3 | 5 | 0,14,28 | 14,28,49 |
| | each 26.666 µg | | | | | | | |
| G | RiLa | 2x50 µl | -- | ID | 3 | 5 | 0,14,28 | 14,28,49 |
| H | FI-RSV Lot#100 (diluted 1:100 in PBS) | 100 µl | -- | IM | 2 | 5 | 0,28 | 28,49 |
| I | Live RSV/A2 10⁵ pfu | 100 µl | -- | IM | 1 | 5 | 0 | 49 |
| J | SYNAGIS ® (15 mg/kg) | | -- | IM | 1 | 5 | 48 | 49 |
| K | Neg. control | | -- | N/A | N/A | 5 | -- | -- |

The following assays were performed to analyze the immune responses: RSV F-protein serum IgG ELISA, RSV virus neutralizing antibody titers (VNT), RSV viral titrations and pulmonary histopathology.

### RSV F-protein serum IgG ELISA

The induction of anti-RSV F protein antibodies were determined by ELISA according to Example 2.

### RSV virus neutralizing antibody titers (VNT)

Sera were analysed by the virus neutralization test (VNT). Briefly, sera samples were diluted 1:10 with EMEM, heat inactivated and serially diluted further 1:4. Diluted sera samples were incubated with RSV (25-50 PFU) for 1 hour at room temperature and inoculated in duplicates onto confluent HEp-2 monolayers in 24 well plates. After one hour incubation at 37°C in a 5% CO₂ incubator, the wells were overlayed with 0.75% Methylcellulose medium. After 4 days of incubation, the overlay was removed and the cells were fixed with 0.1% crystal violet stain for one hour and then rinsed and air dried. The corresponding reciprocal neutralizing antibody titers were determined at the 60% reduction end-point of the virus control.

### RSV viral titrations and pulmonary histopathology

On day 54 nasal tissue was harvested and homogenized for viral titrations. The lung was harvested en bloc and tri-sected for viral titration (left section), histopathology (right section), and PCR analysis (lingular lobe). In addition, RSV viral genome copy numbers (by measuring copy numbers of the RSV NS-1 gene) and cytokine mRNA levels were determined by quantitative reverse transcription polymerase chain reaction (qRT-PCR).

### Results

As can be seen from Figure 12, the RSV-F mRNA vaccines either alone (RSV-F = R2510; RSV-Fdel554-574 mutant = R2821) or in combination with mRNAs encoding other RSV proteins (RSV-N = R2831; RSV-M2-1 = R2833), induce RSV F specific humoral immune responses in cotton rats as shown by total IgG antibody titers on day 49.

As can be seen from Figure 13, the RSV-F mRNA vaccines either alone (RSV-F = R2510; RSV-Fdel554-574 mutant = R2821) or in combination with mRNAs encoding other RSV proteins (RSV-N, R2831 = RSV-M2-1 = R2833), induce the formation of RSV specific functional antibodies in cotton rats as shown by virus neutralizing antibody titers.

As can be seen from Figure 14, the RSV-F mRNA vaccines either alone (RSV-F = R2510; RSV-Fdel554-574 mutant = R2821) or in combination with mRNAs encoding other RSV proteins (RSV-N = R2831; RSV-M2-1 = R2833), reduce lung and nasal viral titers in cotton rats challenged with RSV virus.

As can be seen in Figure 14A, all animal groups vaccinated with mRNA vaccines showed virus titers below the level of detection of the performed virus titration demonstrating protection of vaccinated cotton rats in terms of viral lung titers. By contrast, the Formalin-inactivated virus vaccine reduced only minimally the lung virus titer compared to the RiLa buffer control group. The effect of the β-propiolactone inactivated RSV vaccine was more pronounced but did not reduce the virus lung titer below the detection level in all animals of this group. As can be seen in Figure 14B, the viral titer in the nasal tissue was also strongly reduced in groups vaccinated with mRNA. Nasal viral titers of the Formalin-inactivated virus were comparable to the viral titer in the RiLa vaccinated group. The P-propiolactone inactivated virus vaccine was more effective (at least for two of five animals). In contrast thereto, all mRNA vaccinated groups had reduced nasal virus titer compared to RiLa vaccinated group.

As can be seen from Figure 15, the lung histopathology analysis from the RSV cotton rat challenge study reveals different pathology scores for the various animal groups. From the histopathology it can be concluded that none of the mRNA vaccinated groups displayed enhanced lung pathology as it is the case for the group that was vaccinated using the Formalin-inactivated RSV vaccine. The average pathology scores for peribronchiolitis (PB), perivasculitis (PV), insterstitial pneumonia (IP) and alveolitis (A) are much lower for all groups vaccinated with mRNA compared to group H (Formalin-inactivated RSV). In addition the groups being vaccinated with R2510 (group B; RSV F) or R2821 (group C; RSV F mutant) seem to exhibit reduced lung pathology compared to the RiLa buffer vaccinated and subsequently RSV infected group (G).

As can be seen in Figure 16, the quantitative RT-PCR reveals different expression patterns for the various animal groups. The quantification of RSV genome copies by measuring the RSV NS-1 gene is displayed in A. Genome copy numbers are reduced by vaccination using mRNA vaccines compared to the RiLa buffer control (group G). This is not the case for the group that was vaccinated using formalin-inactivated-RSV (group H). As it is shown in B, the vaccination using the formalin-inactivated-RSV vaccine (group H) induces enhanced expression of the TH2 cytokine IL-4 compared to the control group that was vaccinated with RiLa buffer (group G). By contrast, the vaccination with mRNA R2821 encoding the RSV-F mutant significantly reduced IL-4 mRNA expression compared to the RiLa control group in the lung after RSV challenge infection. C. Expression of INF-γ mRNA. D. Expression of IL-5 mRNA. The expression of IL-5 is significant reduced in groups vaccinated using R2510 or R2821 compared to RiLa buffer vaccinated animals. The expression of the viral NS-1 RNA or cytokine mRNAs, which were isolated from lung tissue, is measured on day 5 post-challenge. The statistical analysis was performed with the student T-test (* p<0.05 when compared to group G (RiLa control)).

### Example 7: RSV cotton rat challenge study II

mRNA vaccines encoding RSV F protein (F) or mutant RSV-F protein (F*) (RSV F del554-574) were prepared according to Example 1. In order to assess the effect of single or several vaccinations (prime and boost vaccinations), these vaccines were administered once, twice or 3 times (as shown in Table 6). Vaccine volumes of 2x50µl were injected intradermally (i.d.) into the back skin of cotton rats. Additional groups were immunized intramuscularly (i.m.) with vaccine volumes of 1x100µl into the right hind leg. As a control, one group was injected intradermally with Ringer-Lactate buffer (buffer). After immunization, the cotton rats were challenged by intranasal (i.n.) infection with RSV/A2 virus (10⁵ PFU in 100 µl; Sigmovir). As a control, one group was not treated and remained unchallenged with virus (untreated).

**Table 5: Animal groups**

| **Groups** | **Vaccine dose** | **Volume** | **Antigen** | **Route** | **# of administrations** | **N per group** | **Vaccination (day)** | **Challenge (day)** |
|---|---|---|---|---|---|---|---|---|
| F* i.d. 3x | R2821 80 µg | 2x50 µl | RSV F mutant | ID | 3 | 5 | 0,14,28 | 49 |
| F* i.d. 2x | R2821 80 µg | 2x50 µl | RSV F mutant | ID | 2 | 5 | 0,14 | 49 |
| F* i.d. 1x | R2821 80 µg | 2x50 µl | RSV F mutant | ID | 1 | 5 | 0 | 49 |
| F i.d. 3x | R2510 80 µg | 2x50 µl | RSV F | ID | 3 | 5 | 0,14,28 | 49 |
| F i.d. 2x | R2510 80 µg | 2x50 µl | RSV F | ID | 2 | 5 | 0,14 | 49 |
| F i.d. 1x | R2510 80 µg | 2x50 µl | RSV F | ID | 1 | 5 | 0 | 49 |
| F* i.m. | R2821 80 µg | 1x100 µl | RSV F mutant | IM | 2 | 5 | 0,14 | 49 |
| F i.m. | R2510 80 µg | 1x100 µl | RSV F | IM | 2 | 5 | 0,14 | 49 |
| Buffer | - | 2x50 µl | | ID | 3 | 5 | 0,14,28 | 49 |
| untreated | - | | -- | N/A | N/A | 5 | -- | -- |

### RSV viral titrations

The determination of RSV viral titers was conducted as described in Example 6.

### Results

As shown in Figure 17A, already one single intradermal vaccination with mRNA vaccines coding for RSV F protein (F) or mutant RSV-F protein (F*) (RSV F del554-574) was highly efficient in reducing the viral titer in the lung compared to the buffer control group. A second and third vaccination ("boost vaccinations) reduced the viral titers below detection level.

As shown in Figure 17B, already two intramuscular vaccinations with mRNA vaccines coding for RSV F protein (F) or mutant RSV-F protein (F*) (RSV F del554-574) strongly reduced the viral titer in the lung compared to the buffer control group.

## Claims

1. mRNA sequence comprising a coding region encoding a mutant of fusion protein F of Respiratory syncytial virus (RSV), wherein the amino acid residues 554-574 of the amino acid sequence of the fusion protein F are deleted in the mutant of fusion protein F;
wherein the G/C content of the coding region is increased by substituting at least 70% of the substitutable codons in the coding region compared with the G/C content of the coding region of the wild type mRNA, and wherein the coded amino acid sequence of said GC-enriched mRNA is not being modified compared with the coded amino acid sequence of the wild type mRNA.

2. The mRNA sequence according to claim 1, wherein the mutant of fusion protein F is derived from the RSV strain ATCC VR-26 long.

3. The mRNA sequence according to claim 1 or 2 comprising additionally
a) a 5'-CAP structure,
b) a poly(A) sequence,
c) and optionally a poly (C) sequence.

4. The mRNA sequence according to claim 3, wherein the poly(A) sequence comprises a sequence of 25 to 400 adenosine nucleotides.

5. The mRNA sequence according to any one of claims 1 to 4 comprising additionally at least one histone stem-loop.

6. The mRNA sequence according to claim 5, wherein the at least one histone stem-loop is selected from following formulae (I) or (II):
formula (I) (stem-loop sequence without stem bordering elements):
formula (II) (stem-loop sequence with stem bordering elements):
wherein:
stem1 or stem2 bordering elements N₁₋₆ is a consecutive sequence of 1 to 6 N, wherein each N is independently from another selected from a nucleotide selected from A, U, T, G and C, or a nucleotide analogue thereof;
stem1 [N₀₋₂GN₃₋₅] is reverse complementary or partially reverse complementary with element stem2, and is a consecutive sequence between of 5 to 7 nucleotides;
wherein N₀₋₂ is a consecutive sequence of 0 to 2 N, wherein each N is independently from another selected from a nucleotide selected from A, U, T, G and C or a nucleotide analogue thereof;
wherein N₃₋₅ is a consecutive sequence of 3 to 5 N, wherein each N is independently from another selected from a nucleotide selected from A, U, T, G and C or a nucleotide analogue thereof, and
wherein G is guanosine or an analogue thereof, and may be optionally replaced by a cytidine or an analogue thereof, provided that its complementary nucleotide cytidine in stem2 is replaced by guanosine;
loop sequence [N₀₋₄(U/T)N₀₋₄] is located between elements stem1 and stem2, and is a consecutive sequence of 3 to 5 nucleotides;
wherein each N₀₋₄ is independent from another a consecutive sequence of 0 to 4 N, wherein each N is independently from another selected from a nucleotide selected from A, U, T, G and C or a nucleotide analogue thereof; and
wherein U/T represents uridine, or optionally thymidine;
stem2 [N₃₋₅CN₀₋₂] is reverse complementary or partially reverse complementary with element stem1, and is a consecutive sequence between of 5 to 7 nucleotides;
wherein N₃₋₅ is a consecutive sequence of 3 to 5 N, wherein each N is independently from another selected from a nucleotide selected from A, U, T, G and C or a nucleotide analogue thereof;
wherein N₀₋₂ is a consecutive sequence of 0 to 2 N, wherein each N is independently from another selected from a nucleotide selected from A, U, T, G and C or a nucleotide analogue thereof; and
wherein C is cytidine or an analogue thereof, and may be optionally replaced by a guanosine or an analogue thereof provided that its complementary nucleotide guanosine in stem1 is replaced by cytidine;
wherein
stem1 and stem2 are capable of base pairing with each other
forming a reverse complementary sequence, wherein base pairing may occur between stem1 and stem2, or
forming a partially reverse complementary sequence, wherein an incomplete base pairing may occur between stem1 and stem2.

7. The mRNA sequence according to claim 6, wherein the at least one histone stem-loop is selected from at least one of following formulae (Ia) or (IIa): formula (Ia) (stem-loop sequence without stem bordering elements) formula (IIa) (stem-loop sequence with stem bordering elements)

8. The mRNA sequence according to any of claims 1 to 7 comprising additionally a 3'-UTR element.

9. The mRNA sequence according to claim 8, wherein the at least one 3'UTR element comprises or consists of a nucleic acid sequence, which is derived from a 3'UTR of a gene providing a stable mRNA, wherein the 3'UTR element preferably comprises or consists of a nucleic acid sequence derived from a 3'UTR of a gene selected from the group consisting of an albumin gene, an α-globin gene, a β-globin gene, a tyrosine hydroxylase gene, a lipoxygenase gene, and a collagen alpha gene, wherein the 3'-UTR element more preferably comprises or consists of a nucleic acid sequence derived from a 3'UTR of α-globin gene, most preferably comprising the corresponding RNA sequence of the nucleic acid sequence according to SEQ ID NO. 29.

10. The mRNA sequence according to any of claims 8 or 9, wherein the mRNA sequence comprises, preferably in 5'- to 3'-direction:
a.) a 5'-CAP structure, preferably m7GpppN;
b.) a coding region encoding the mutant of fusion protein F of Respiratory syncytial virus (RSV);
c.) a 3'-UTR element comprising or consisting of a nucleic acid sequence, which is derived from a alpha globin gene, preferably comprising the corresponding RNA sequence of the nucleic acid sequence according to SEQ ID NO. 29;
d.) a poly(A) sequence, preferably comprising 64 adenosines;
e.) a poly(C) sequence, preferably comprising 30 cytosines; and
f.) a histone-stem-loop, preferably comprising the corresponding RNA sequence to the nucleic acid sequence according to SEQ ID No 30.

11. The mRNA sequence according to claim 8, wherein the at least one 3'UTR element comprises or consist of a nucleic acid sequence, which is derived from the 3'UTR of a human albumin gene.

12. The mRNA sequence according to claim 11, wherein the 3'UTR element is derived from a nucleic acid sequence according to SEQ ID NO. 25, preferably from a corresponding RNA sequence.

13. The mRNA sequence according to any of claims 1 to 12 comprising additionally a 5'-UTR element, which comprises or consists of a nucleic acid sequence, which is derived from the 5'UTR of a TOP gene, preferably from a corresponding RNA sequence, preferably lacking the 5'TOP motif.

14. The mRNA sequence according to claim 13, wherein the 5'UTR element comprises or consists of a nucleic acid sequence, which is derived from a 5'UTR of a TOP gene encoding a ribosomal protein, preferably from a corresponding RNA sequence, preferably lacking the 5'TOP motif.

15. The mRNA sequence according to claim 14, wherein the 5'UTR element comprises or consists of a nucleic acid sequence, which is derived from a 5'UTR of a TOP gene encoding a ribosomal Large protein (RPL), preferably lacking the 5'TOP motif and more preferably comprising or consisting of a corresponding RNA sequence of the nucleic acid sequence according to SEQ ID NO. 23.

16. The mRNA sequence according to claim 15, wherein the mRNA sequence comprises, preferably in 5'- to 3'-direction:
a.) a 5'-CAP structure;
b.) a 5'-UTR element which comprises or consists of a nucleic acid sequence, which is derived from the 5'-UTR of a TOP gene, preferably comprising or consisting of the corresponding RNA sequence of the nucleic acid sequence according to SEQ ID NO. 23;
c.) a coding region encoding the mutant of fusion protein F of Respiratory syncytial virus (RSV);
d.) a 3'UTR element comprising or consisting of a nucleic acid sequence, which is derived from a gene providing a stable mRNA, preferably comprising or consisting of the corresponding RNA sequence of a nucleic acid sequence according to SEQ ID NO. 18;
e.) a poly(A) sequence preferably comprising 64 adenosines;
f.) a poly(C) sequence, preferably comprising 30 cytosines; and
g.) a histone-stem-loop, preferably comprising the corresponding RNA sequence of the nucleic acid sequence according to SEQ ID No 30.

17. The mRNA sequence according to claim 16, wherein the mRNA sequence comprises the RNA sequence according to SEQ ID No. 33.

18. The mRNA sequence according to claims 1 to 17, wherein the mRNA sequence is associated with or complexed with a cationic or polycationic compound or a polymeric carrier, optionally in a weight ratio selected from a range of 6:1 (w/w) to 0.25:1 (w/w) of mRNA to cationic or polycationic compound and/or with a polymeric carrier; or optionally in a nitrogen/phosphate ratio of mRNA to cationic or polycationic compound and/or polymeric carrier in the range of 0.1-10.

19. The mRNA sequence according to claim 18, wherein the mRNA sequence is associated or complexed with a cationic protein or peptide, preferably protamine, or with a cationic lipid.

20. A composition comprising a plurality or more than one of mRNA sequences each according to any of claims 1 to 19.

21. Pharmaceutical composition comprising an mRNA sequence as defined according to any of claims 1 to 19 or a composition as defined according to claim 20 and optionally a pharmaceutically acceptable carrier.

22. Pharmaceutical composition according to claim 21, wherein the mRNA sequence is complexed at least partially with a cationic or polycationic compound and/or a polymeric carrier, preferably cationic proteins or peptides, most preferably protamine, or cationic lipids.

23. Pharmaceutical composition according to claim 22, wherein the ratio of complexed mRNA to free mRNA is selected from a range of 5:1 (w/w) to 1:10 (w/w).

24. Pharmaceutical composition according to any one of claims 21 to 23, for use as a vaccine.

25. Kit or kit of parts comprising the mRNA sequence as defined according to any of claims 1 to 19, the composition as defined according to claim 20, the pharmaceutical composition as defined according to any of claims 21 to 24 and optionally technical instructions with information on the administration and dosage of the components.

26. mRNA sequence as defined according to any of claims 1 to 19, composition as defined according to claim 20, pharmaceutical composition as defined according to any of claims 21 to 24, and kit or kit of parts as defined according to claim 25 for use as a medicament.

27. mRNA sequence as defined according to any of claims 1 to 19, composition as defined according to claim 20, pharmaceutical composition as defined according to any of claims 21 to 24, and kit or kit of parts as defined according to claim 25 for use in the treatment or prophylaxis of RSV infections.

28. mRNA sequence, composition, pharmaceutical composition and kit or kit of parts for use according to claim 27, wherein the treatment is combined with administration of RSV immue globuline, particularly Palivizumab.

## Patentansprüche

1. mRNA-Sequenz, umfassend eine kodierende Region, die eine Mutante des Fusionsproteins F des Respiratorischen Syncytial-Virus (RSV) kodiert, wobei die Aminosäurereste 554-574 der Aminosäuresequenz des Fusionsproteins F in der Mutante des Fusionsproteins F deletiert sind;
wobei der G/C-Gehalt der kodierenden Region durch Substitution von mindestens 70% der substituierbaren Codons in der kodierenden Region im Vergleich zum G/C-Gehalt der kodierenden Region der Wildtyp-mRNA erhöht wird, und wobei die kodierte Aminosäuresequenz der GC-angereicherten mRNA im Vergleich zur kodierten Aminosäuresequenz der Wildtyp-mRNA nicht modifiziert wird.

2. mRNA-Sequenz nach Anspruch 1, wobei die Mutante des Fusionsproteins F vom RSV-Stamm "ATCC VR-26 long" abgeleitet ist.

3. mRNA-Sequenz nach Anspruch 1 oder 2, zusätzlich umfassend
a) eine 5'-CAP-Struktur,
b) eine Poly(A)-Sequenz,
c) und optional eine Poly(C)-Sequenz.

4. mRNA-Sequenz nach Anspruch 3, wobei die Poly(A)-Sequenz eine Sequenz von 25 bis 400 Adenosin-Nukleotiden umfasst.

5. mRNA-Sequenz nach einem der Ansprüche 1 bis 4, die zusätzlich mindestens einen Histon-Stem-Loop umfasst.

6. mRNA-Sequenz nach Anspruch 5, wobei der mindestens eine Histon-Stem-Loop aus den folgenden Formeln (I) oder (II) ausgewählt ist:
Formel (I) (Stem-Loop-Sequenz ohne an den Stamm ("stem") angrenzende Elemente):
Formel (II) (Stem-Loop-Sequenz mit an den Stamm ("stem") angrenzenden Elementen):
wobei:
an stem1 oder stem2 angrenzende Elemente N₁₋₆ eine konsekutive Sequenz von 1 bis 6, bevorzugt von 2 bis 6, bevorzugter von 2 bis 5, noch bevorzugter von 3 bis 5, am meisten bevorzugt von 4 bis 5 oder 5 N ist, wobei jedes N unabhängig voneinander ausgewählt ist aus einem Nukleotid ausgewählt aus A, U, T, G und C, oder einem Nukleotidanalog davon;
stem1 [N₀₋₂GN₃₋₅] revers-komplementär oder teilweise reverskomplementär zu Element stem2 ist, und eine konsekutive Sequenz von 5 bis 7 Nukleotiden ist;
wobei N₀₋₂ eine konsekutive Sequenz von 0 bis 2, bevorzugt von 0 bis 1, bevorzugter von 1 N ist, wobei jedes N unabhängig voneinander ausgewählt ist aus einem Nukleotid, ausgewählt aus A, U, T, G und C oder einem Nukleotidanalog davon;
wobei N₃₋₅ eine konsekutive Sequenz von 3 bis 5, bevorzugt von 4 bis 5, bevorzugter von 4 N ist, wobei jedes N unabhängig voneinander ausgewählt ist aus einem Nukleotid, ausgewählt aus A, U, T, G und C oder einem Nukleotidanalog davon, und
wobei G Guanosin oder ein Analog davon ist und optional durch Cytidin oder ein Analog davon ersetzt sein kann, vorausgesetzt, dass sein komplementäres Nukleotid Cytidin in stem2 durch Guanosin ersetzt wird;
Loop-Sequenz [N₀₋₄(U/T)N₀₋₄] zwischen Elementen stem1 und stem2 gelegen ist, und eine konsekutive Sequenz von 3 bis 5 Nukleotiden, bevorzugter von 4 Nukleotiden ist;
wobei jedes N₀₋₄ unabhängig voneinander eine konsekutive Sequenz von 0 bis 4, bevorzugt von 1 bis 3, bevorzugter von 1 bis 2 N ist, wobei jedes N unabhängig voneinander ausgewählt ist aus einem Nukleotid, ausgewählt aus A, U, T, G und C oder einem Nukleotidanalog davon; und
wobei U/T Uridin darstellt, oder optional Thymidin;
stem2 [N₃₋₅CN₀₋₂] revers-komplementär oder teilweise reverskomplementär zu Element stem1 ist und eine konsekutive Sequenz von 5 bis 7 Nukleotiden ist;
wobei N₃₋₅ eine konsekutive Sequenz von 3 bis 5, bevorzugt von 4 bis 5, bevorzugter von 4 N ist, wobei jedes N unabhängig voneinander ausgewählt ist aus einem Nukleotid, ausgewählt aus A, U, T, G und C oder einem Nukleotidanalog davon;
wobei N₀₋₂ eine konsekutive Sequenz von 0 bis 2, bevorzugt von 0 bis 1, bevorzugter von 1 N ist, wobei jedes N unabhängig voneinander ausgewählt ist aus einem Nukleotid, ausgewählt aus A, U, T, G und C oder einem Nukleotidanalog davon; und
wobei C Cytidin oder ein Analog davon ist, und optional durch ein Guanosin oder ein Analog davon ersetzt sein kann, vorausgesetzt, dass sein komplementäres Nukleotid Guanosin in stem1 durch Cytidin ersetzt wird;
wobei
stem1 und stem2 zur Basenpaarung miteinander fähig sind und
eine revers-komplementäre Sequenz bilden, wobei zwischen stem1 und stem2 Basenpaarung auftreten kann, oder
eine teilweise revers-komplementäre Sequenz bilden, wobei eine unvollständige Basenpaarung zwischen stem1 und stem2 auftreten kann.

7. mRNA-Sequenz nach einem der Ansprüche 1 bis 6, wobei der mindestens eine Histon-Stem-Loop ausgewählt ist aus mindestens einer der folgenden Formeln (Ia) oder (IIa): Formel (Ia) (Stem-Loop-Sequenz ohne an den Stamm ("stem") angrenzende Elemente) Formel (IIa) (Stem-Loop-Sequenz mit an den Stamm ("stem") angrenzenden Elementen)

8. mRNA-Sequenz nach einem der Ansprüche 1 bis 7, die zusätzlich ein 3'-UTR-Element umfasst.

9. RNA-Sequenz nach Anspruch 8, wobei das mindestens eine 3'UTR-Element eine Nukleinsäuresequenz umfasst oder aus ihr besteht, die von einer 3'UTR eines Gens abgeleitet ist, das eine stabile mRNA bereitstellt; wobei das 3'UTR-Element bevorzugt eine Nukleinsäuresequenz umfasst oder aus ihr besteht, die von einer 3'UTR eines Gens abgeleitet ist, das aus der Gruppe bestehend aus einem Albumingen, einem α-Globin-Gen, einem β-Globin-Gen, einem Tyrosin-Hydroxylase-Gen, einem Lipoxygenase-Gen und einem Kollagen-alpha-Gen ausgewählt ist, wobei das 3'-UTR-Element bevorzugter eine von einer 3'UTR eines α-Globin-Gens abgeleitete Nukleinsäuresequenz umfasst oder aus ihr besteht, welche am bevorzugtesten die entsprechende RNA-Sequenz der Nukleinsäuresequenz gemäß SEQ ID NO. 29 umfasst.

10. mRNA-Sequenz nach einem der Ansprüche 8 oder 9, wobei die mRNA-Sequenz bevorzugt in 5'- bis 3'-Richtung umfasst:
a.) eine 5'-CAP-Struktur, bevorzugt m7GpppN;
b.) eine kodierende Region, die die Mutante des Fusionsproteins F des Respiratorischen Syncytial-Virus (RSV) kodiert;
c.) ein 3'-UTR-Element, das eine Nukleinsäuresequenz umfasst oder aus ihr besteht, die von einem Alpha-Globin-Gen abgeleitet ist, bevorzugt umfassend die entsprechende RNA-Sequenz der Nukleinsäuresequenz gemäß SEQ ID NO. 29;
d.) eine Poly(A)-Sequenz, die bevorzugt 64 Adenosine umfasst;
e.) eine Poly(C)-Sequenz, die bevorzugt 30 Cytosine umfasst; und
f.) ein Histon-Stem-Loop, der bevorzugt die entsprechende RNA-Sequenz zur Nukleinsäuresequenz gemäß SEQ ID NO. 30 umfasst.

11. mRNA-Sequenz nach Anspruch 8, wobei das mindestens eine 3'UTR-Element eine Nukleinsäuresequenz umfasst oder aus ihr besteht, die von der 3'UTR eines menschlichen Albumin-Gens abgeleitet ist.

12. mRNA-Sequenz nach Anspruch 11, wobei das 3'UTR-Element von einer Nukleinsäuresequenz gemäß SEQ ID NO. 25, bevorzugt von einer entsprechenden RNA-Sequenz, abgeleitet ist.

13. mRNA-Sequenz nach einem der Ansprüche 1 bis 12, die zusätzlich ein 5'-UTR-Element umfasst, das eine Nukleinsäuresequenz umfasst oder aus ihr besteht, die von der 5'UTR eines TOP-Gens, bevorzugt von einer entsprechenden RNA-Sequenz, welche bevorzugt das 5'TOP-Motiv nicht enthält, abgeleitet ist.

14. mRNA-Sequenz nach Anspruch 13, wobei das 5'UTR-Element eine Nukleinsäuresequenz umfasst oder aus ihr besteht, die von einem 5'UTR eines TOP-Gens abgeleitet ist, das für ein ribosomales Protein kodiert, bevorzugt von einer entsprechenden RNA-Sequenz, welche bevorzugt das 5'TOP-Motiv nicht enthält.

15. mRNA-Sequenz nach Anspruch 14, wobei das 5'UTR-Element eine Nukleinsäuresequenz umfasst oder aus ihr besteht, die von einer 5'UTR eines TOP-Gens abgeleitet ist, das für ein großes ribosomales Protein ("ribosomal Large protein", RPL) kodiert, welche bevorzugt das 5'TOP-Motiv nicht enthält und das mehr bevorzugt eine entsprechende RNA-Sequenz der Nukleinsäuresequenz gemäß SEQ ID NO. 23 umfasst oder aus ihr besteht.

16. mRNA-Sequenz nach Anspruch 15, wobei die mRNA-Sequenz bevorzugt in 5'- bis 3'-Richtung umfasst:
a.) eine 5'-CAP-Struktur;
b.) ein 5'-UTR-Element, das eine Nukleinsäuresequenz umfasst oder aus ihr besteht, die von der 5'-UTR eines TOP-Gens abgeleitet ist, bevorzugt umfassend oder bestehend aus der entsprechenden RNA-Sequenz der Nukleinsäuresequenz gemäß SEQ ID NO. 23;
c.) eine kodierende Region, die die Mutante des Fusionsproteins F des Respiratorischen Syncytial-Virus (RSV) kodiert;
d.) ein 3'UTR-Element, das eine Nukleinsäuresequenz umfasst oder aus ihr besteht, die von einem Gen abgeleitet ist, das eine stabile mRNA bereitstellt bevorzugt umfassend oder bestehend aus der entsprechenden RNA-Sequenz einer Nukleinsäuresequenz gemäß SEQ ID NO. 18;
e.) eine Poly(A)-Sequenz, die bevorzugt 64 Adenosine umfasst;
f.) eine Poly(C)-Sequenz, die bevorzugt 30 Cytosine umfasst; und
g.) einen Histon-Stem-Loop, die bevorzugt die entsprechende RNA-Sequenz der Nukleinsäuresequenz gemäß SEQ ID Nr. 30 umfasst.

17. mRNA-Sequenz nach Anspruch 16, wobei die mRNA-Sequenz die RNA-Sequenz nach SEQ ID Nr. 33 umfasst.

18. mRNA-Sequenz nach den Ansprüchen 1 bis 17, wobei die mRNA-Sequenz mit einer kationischen oder polykationischen Verbindung oder einem polymeren Träger assoziiert oder komplexiert ist, optional in einem Gewichtsverhältnis ausgewählt aus einem Bereich von 6:1 (w/w) bis 0,25:1 (w/w) von mRNA zu einer kationischen oder polykationischen Verbindung und/oder mit einem polymeren Träger; oder optional in einem Stickstoff/Phosphat-Verhältnis von mRNA zu einer kationischen oder polykationischen Verbindung und/oder einem polymeren Träger im Bereich von 0,1-10.

19. mRNA-Sequenz nach Anspruch 18, wobei die mRNA-Sequenz mit einem kationischen Protein oder Peptid, bevorzugt Protamin, oder mit einem kationischen Lipid assoziiert oder komplexiert ist.

20. Zusammensetzung umfassend eine Vielzahl oder mehr als eine von mRNA-Sequenzen, jeweils nach einem der Ansprüche 1 bis 19.

21. Pharmazeutische Zusammensetzung umfassend eine mRNA-Sequenz wie nach einem der Ansprüche 1 bis 19 definiert oder eine Zusammensetzung wie nach Anspruch 20 definiert und optional einen pharmazeutisch verträglichen Träger.

22. Pharmazeutische Zusammensetzung nach Anspruch 21, wobei die mRNA-Sequenz zumindest teilweise mit einer kationischen oder polykationischen Verbindung und/oder einem polymeren Träger, bevorzugt kationischen Proteinen oder Peptiden, am bevorzugtesten Protamin, oder kationischen Lipiden komplexiert ist.

23. Pharmazeutische Zusammensetzung nach Anspruch 22, wobei das Verhältnis von komplexierter mRNA zu freier mRNA aus einem Bereich von 5:1 (w/w) bis 1:10 (w/w) ausgewählt ist.

24. Pharmazeutische Zusammensetzung nach einem der Ansprüche 21 bis 23 zur Verwendung als Impfstoff.

25. Kit oder Kit von Teilen, umfassend die mRNA-Sequenz wie nach einem der Ansprüche 1 bis 19 definiert, die Zusammensetzung wie nach Anspruch 20 definiert, die pharmazeutische Zusammensetzung wie nach einem der Ansprüche 21 bis 24 definiert und optional technische Anweisungen mit Informationen über die Verabreichung und Dosierung der Komponenten.

26. mRNA-Sequenz wie nach einem der Ansprüche 1 bis 19 definiert, Zusammensetzung wie nach Anspruch 20 definiert, pharmazeutische Zusammensetzung wie nach einem der Ansprüche 21 bis 24 definiert, und Kit oder Kit von Teilen wie nach Anspruch 25 definiert zur Verwendung als Medikament.

27. mRNA-Sequenz wie nach einem der Ansprüche 1 bis 19 definiert, Zusammensetzung wie nach Anspruch 20 definiert, pharmazeutische Zusammensetzung wie nach einem der Ansprüche 21 bis 24 definiert, und Kit oder Kit von Teilen wie nach Anspruch 25 definiert zur Verwendung bei der Behandlung oder Prophylaxe von RSV-Infektionen.

28. mRNA-Sequenz, Zusammensetzung, pharmazeutische Zusammensetzung und Kit oder Kit von Teilen zur Verwendung nach Anspruch 27, wobei die Behandlung mit der Verabreichung von RSV-Immunglobulinen, insbesondere Palivizumab, kombiniert ist.

## Revendications

1. Séquence d'ARNm comprenant une région codante codant pour un mutant de la protéine de fusion F du virus syncytial respiratoire (RSV), dans laquelle les résidus d'acide aminé 554 à 574 de la séquence d'acides aminés de la protéine de fusion F sont délétés dans le mutant de la protéine de fusion F;
où la teneur en G/C de la région codante est augmentée en substituant au moins 70 % des codons substituables dans la région codante par rapport à la teneur en G/C de la région codante de l'ARNm sauvage, et où la séquence d'acides aminés codée dudit ARNm enrichi en GC n'est pas modifiée par rapport à la séquence d'acides aminés codée de l'ARNm sauvage.

2. Séquence d'ARNm selon la revendication 1, dans laquelle le mutant de la protéine de fusion F est dérivé de la souche de RSV ATCC VR-26 longue.

3. Séquence d'ARNm selon la revendication 1 ou 2 comprenant de plus
a) une structure 5'-CAP,
b) une séquence poly(A),
c) et éventuellement une séquence poly(C).

4. Séquence d'ARNm selon la revendication 3, dans laquelle la séquence poly(A) comprend une séquence de 25 à 400 nucléotides d'adénosine.

5. Séquence d'ARNm selon l'une quelconque des revendications 1 à 4 comprenant de plus au moins une tige-boucle d'histone.

6. Séquence d'ARNm selon la revendication 5, dans laquelle l'au moins une tige-boucle d'histone est choisie parmi les formules (I) ou (II) suivantes :
formule (I) (séquence de tige-boucle sans éléments bordant la tige) :
formule (II) (séquence de tige-boucle avec éléments bordant la tige) :
dans lesquelles :
les éléments bordants de tige 1 ou de tige 2
N₁₋₆
est une séquence consécutive de 1 à 6 N, où chaque N est indépendamment l'un de l'autre choisi parmi un nucléotide choisi parmi A, U, T, G et C, ou un de leurs analogues de nucléotide ;
la tige 1
[N₀₋₂GN₃₋₅]
est complémentaire inverse ou partiellement complémentaire inverse avec la tige 2 de l'élément, et est une séquence consécutive comprise entre 5 à 7 nucléotides ;
où N₀₋₂ est une séquence consécutive de 0 à 2 N, où chaque N est choisi indépendamment l'un de l'autre parmi un nucléotide choisi parmi A, U, T, G et C ou un de leurs analogues de nucléotide ;
où N₃₋₅ est une séquence consécutive de 3 à 5 N, où chaque N est indépendamment l'un de l'autre choisi parmi un nucléotide choisi parmi A, U, T, G et C ou un de leurs analogues de nucléotide, et
où G est une guanosine ou un de ses analogues, et peut être éventuellement remplacée par une cytidine ou un de ses analogues pourvu que son nucléotide cytidine complémentaire dans la tige 2 soit remplacé par une guanosine ;
la séquence de boucle
[N₀₋₄(U/T)N₀₋₁]
est située entre la tige 1 et la tige 2 d'éléments, et est une séquence consécutive de 3 à 5 nucléotides ;
où chaque N₀₋₄ est indépendant l'un de l'autre une séquence consécutive de 0 à 4 N, où chaque N est indépendamment l'un de l'autre choisi parmi un nucléotide choisi parmi A, U, T, G et C ou un de leurs analogues de nucléotide ; et
où U/T représente une uridine, ou éventuellement une thymidine ;
la tige 2
[N₃₋₅CN₀₋₂]
est complémentaire inverse ou partiellement complémentaire inverse avec la tige 1 de l'élément, et est une séquence consécutive entre 5 et 7 nucléotides ;
où N₃₋₅ est une séquence consécutive de 3 à 5 N, où chaque N est indépendamment l'un de l'autre choisi parmi un nucléotide choisi parmi A, U, T, G et C ou un de leurs analogues de nucléotide ;
où N₀₋₂ est une séquence consécutive de 0 à 2 N, où chaque N est indépendamment l'un de l'autre choisi parmi un nucléotide choisi parmi A, U, T, G et C ou un de leurs analogues de nucléotide ; et
où C est une cytidine ou un de ses analogues, et peut être éventuellement remplacée par une guanosine ou un de ses analogues pourvu que son nucléotide guanosine complémentaire dans la tige 1 soit remplacé par une cytidine ;
où
la tige 1 et la tige 2 sont capables d'appariement de bases l'une avec l'autre formant une séquence complémentaire inverse, où l'appariement de bases peut se produire entre la tige 1 et la tige 2, ou
formant une séquence partiellement complémentaire inverse, où un appariement de bases incomplet peut se produire entre la tige 1 et la tige 2.

7. Séquence d'ARNm selon la revendication 6, dans laquelle l'au moins une tige-boucle d'histone est choisie parmi une des formules (Ia) ou (IIa) suivantes : formule (la) (séquence tige-boucle sans éléments bordant la tige) formule (IIa) (séquence tige-boucle avec éléments bordant la tige)

8. Séquence d'ARNm selon l'une quelconque des revendications 1 à 7 comprenant de plus un élément 3'-UTR.

9. Séquence d'ARNm selon la revendication 8, dans laquelle l'au moins un élément 3'UTR comprend ou est constitué d'une séquence d'acide nucléique, qui est dérivée d'un 3'UTR d'un gène fournissant un ARNm stable, où l'élément 3'UTR comprend ou est constitué de préférence d'une séquence d'acide nucléique dérivée d'un 3'UTR d'un gène choisi dans le groupe constitué par un gène d'albumine, un gène d'α-globine, un gène de β-globine, un gène de tyrosine hydroxylase, un gène de lipoxygénase et un gène d'alpha collagène, où l'élément 3'-UTR comprend ou est constitué davantage de préférence d'une séquence d'acide nucléique dérivée d'un 3'UTR de gène d'a-globine, de préférence entre toutes comprenant la séquence d'ARN correspondante de la séquence d'acide nucléique selon SEQ ID n° 29.

10. Séquence d'ARNm selon l'une quelconque des revendications 8 ou 9, dans laquelle la séquence d'ARNm comprend, de préférence dans une direction 5' - 3' :
a) une structure 5'-CAP, de préférence m7GpppN ;
b) une région codante codant pour le mutant de la protéine de fusion F du virus syncytial respiratoire (RSV) ;
c) un élément 3'-UTR comprenant ou constitué d'une séquence d'acide nucléique, qui est dérivée d'un gène d'alpha globine, de préférence comprenant la séquence d'ARN correspondante de la séquence d'acide nucléique selon SEQ ID n° 29 ;
d) une séquence poly(A), comprenant de préférence 64 adénosines ;
e) une séquence poly(C), comprenant de préférence 30 cytosines ; et
f) une tige-boucle d'histone, comprenant la séquence d'ARN correspondante à la séquence d'acide nucléique selon SEQ ID n° 30.

11. Séquence d'ARNm selon la revendication 8, dans laquelle l'au moins un élément 3'UTR comprend ou est constitué d'une séquence d'acide nucléique, qui est dérivée du 3'UTR d'un gène d'albumine humain.

12. Séquence d'ARNm selon la revendication 11, dans laquelle l'élément 3'UTR est dérivé d'une séquence d'acide nucléique selon SEQ ID n° 25, de préférence à partir d'une séquence d'ARN correspondante.

13. Séquence d'ARNm selon l'une quelconque des revendications 1 à 12 comprenant de plus un élément 5'-UTR, lequel comprend ou est constitué d'une séquence d'acide nucléique, qui est dérivée du 5'UTR d'un gène TOP, de préférence à partir d'une séquence d'ARN correspondante, de préférence manquant le motif 5'TOP.

14. Séquence d'ARNm selon la revendication 13, dans laquelle l'élément 5'UTR comprend ou est constitué d'une séquence d'acide nucléique, qui est dérivée d'un 5'UTR d'un gène TOP codant pour une protéine ribosomique, de préférence à partir d'une séquence d'ARN correspondante, de préférence manquant le motif 5'TOP.

15. Séquence d'ARNm selon la revendication 14, dans laquelle l'élément 5'UTR comprend ou est constitué d'une séquence d'acide nucléique, qui est dérivée d'un 5'UTR d'un gène TOP codant pour une grande protéine ribosomique (RPL), de préférence manquant le motif 5'TOP et davantage de préférence comprenant ou étant constituée d'une séquence d'ARN correspondante de la séquence d'acide nucléique selon SEQ ID n°23.

16. Séquence d'ARNm selon la revendication 15, dans laquelle la séquence d'ARNm comprend, de préférence dans une direction 5' - 3' :
a) une structure 5'-CAP ;
b) un élément 5'-UTR qui comprend ou est constitué d'une séquence d'acide nucléique, qui est dérivée du 5'-UTR d'un gène TOP, de préférence comprenant ou constituée de la séquence d'ARN correspondante de la séquence d'acide nucléique selon SEQ ID n° 23 ;
c) une région codante codant pour le mutant de la protéine de fusion F du virus syncytial respiratoire (RSV) ;
d) un élément 3'UTR comprenant ou constitué d'une séquence d'acide nucléique, qui est dérivée d'un gène fournissant un ARNm stable, de préférence comprenant ou constituée de la séquence d'ARN correspondante d'une séquence d'acide nucléique selon SEQ ID n° 18 ;
e) une séquence poly(A) comprenant de préférence 64 adénosines ;
f) une séquence poly(C), comprenant de préférence 30 cytosines ; et
g) une tige-boucle d'histone, de préférence comprenant la séquence d'ARN correspondante de la séquence d'acide nucléique selon SEQ ID n° 30.

17. Séquence d'ARNm selon la revendication 16, dans laquelle la séquence d'ARNm comprend la séquence d'ARN selon SEQ ID n° 33.

18. Séquence d'ARNm selon les revendications 1 à 17, dans laquelle la séquence d'ARNm est associée avec ou complexée avec un composé cationique ou polycationique ou un véhicule polymère, éventuellement dans un rapport en poids choisi dans une plage de 6 : 1 (p/p) à 0,25 : 1 (p/p) d'ARNm au composé cationique ou polycationique et/ou avec le véhicule polymère ; ou éventuellement dans un rapport azote/phosphate de l'ARNm au composé cationique ou polycationique et/ou véhicule polymère dans la plage de 0,1 à 10.

19. Séquence d'ARNm selon la revendication 18, dans laquelle la séquence d'ARNm est associée ou complexée avec une protéine ou un peptide cationique, de préférence une protamine, ou avec un lipide cationique.

20. Composition comprenant une pluralité d'une ou de plus d'une des séquences d'ARNm chacune selon l'une quelconque des revendications 1 à 19.

21. Composition pharmaceutique comprenant une séquence d'ARNm telle que définie selon l'une quelconque des revendications 1 à 19 ou composition telle que définie selon la revendication 20 et éventuellement un véhicule pharmaceutiquement acceptable.

22. Composition pharmaceutique selon la revendication 21, dans laquelle la séquence d'ARNm est complexée au moins partiellement avec un composé cationique ou polycationique et/ou un véhicule polymère, de préférence des protéines ou des peptides cationiques, de préférence entre toutes une protamine, ou des lipides cationiques.

23. Composition pharmaceutique selon la revendication 22, dans laquelle le rapport d'ARNm complexé à ARNm libre est choisi dans une plage de 5 : 1 (p/p) à 1 : 10 (p/p).

24. Composition pharmaceutique selon l'une quelconque des revendications 21 à 23, pour une utilisation comme vaccin.

25. Kit ou parties de kit comprenant la séquence d'ARNm telle que définie selon l'une quelconque des revendications 1 à 19, la composition telle que définie selon la revendication 20, la composition pharmaceutique telle que définie selon l'une quelconque des revendications 21 à 24 et éventuellement des instructions techniques avec des informations sur l'administration et la posologie des composants.

26. Séquence d'ARNm telle que définie selon l'une quelconque des revendications 1 à 19, composition telle que définie selon la revendication 20, composition pharmaceutique telle que définie selon l'une quelconque des revendications 21 à 24 et kit ou parties de kit tel que défini selon la revendication 25 pour une utilisation comme médicament.

27. Séquence d'ARNm telle que définie selon l'une quelconque des revendications 1 à 19, composition telle que définie selon la revendication 20, composition pharmaceutique telle que définie selon l'une quelconque des revendications 21 à 24 et kit ou parties de kit tel que défini selon la revendication 25 pour une utilisation dans le traitement ou la prophylaxie des infections par RSV.

28. Séquence d'ARNm, composition, composition pharmaceutique et kit ou parties de kit pour une utilisation selon la revendication 27, où le traitement est combiné avec l'administration d'une globuline immune au RSV, en particulier le palivizumab.


### SEQUENCE LISTING

<110> CureVac GmbH
<120> Respiratory syncytial virus (RSV) vaccine
<130> CU01P151WO1
<160> 37
<170> PatentIn version 3.5
<210> 1
   <211> 574
   <212> PRT
   <213> respiratory syncytial virus
<400> 1
   Met Glu Leu Pro Ile Leu Lys Ala Asn Ala Ile Thr Thr Ile Leu Ala
   1               5                   10                  15
   Ala Val Thr Phe Cys Phe Ala Ser Ser Gln Asn Ile Thr Glu Glu Phe
               20                  25                  30
   Tyr Gln Ser Thr Cys Ser Ala Val Ser Lys Gly Tyr Leu Ser Ala Leu
           35                  40                  45
   Arg Thr Gly Trp Tyr Thr Ser Val Ile Thr Ile Glu Leu Ser Asn Ile
       50                  55                  60
   Lys Glu Asn Lys Cys Asn Gly Thr Asp Ala Lys Val Lys Leu Ile Asn
   65                  70                  75                  80
   Gln Glu Leu Asp Lys Tyr Lys Asn Ala Val Thr Glu Leu Gln Leu Leu
                   85                  90                  95
   Met Gln Ser Thr Thr Ala Ala Asn Asn Arg Ala Arg Arg Glu Leu Pro
               100                 105                 110
   Arg Phe Met Asn Tyr Thr Leu Asn Asn Thr Lys Lys Thr Asn Val Thr
           115                 120                 125
   Leu Ser Lys Lys Arg Lys Arg Arg Phe Leu Gly Phe Leu Leu Gly Val
           130                 135                 140
   Gly Ser Ala Ile Ala Ser Gly Ile Ala Val Ser Lys Val Leu His Leu
   145                 150                 155                 160
   Glu Gly Glu Val Asn Lys Ile Lys Ser Ala Leu Leu Ser Thr Asn Lys
                   165                 170                 175
   Ala Val Val Ser Leu Ser Asn Gly Val Ser Val Leu Thr Ser Lys Val
               180                 185                 190
   Leu Asp Leu Lys Asn Tyr Ile Asp Lys Gln Leu Leu Pro Ile Val Asn
           195                 200                 205
   Lys Gln Ser Cys Arg Ile Ser Asn Ile Glu Thr Val Ile Glu Phe Gln
       210                 215                 220
   Gln Lys Asn Asn Arg Leu Leu Glu Ile Thr Arg Glu Phe Ser Val Asn
   225                 230                 235                 240
   Ala Gly Val Thr Thr Pro Val Ser Thr Tyr Met Leu Thr Asn Ser Glu
                   245                 250                 255
   Leu Leu Ser Leu Ile Asn Asp Met Pro Ile Thr Asn Asp Gln Lys Lys
               260                 265                 270
   Leu Met Ser Asn Asn Val Gln Ile Val Arg Gln Gln Ser Tyr Ser Ile
           275                 280                 285
   Met Ser Ile Ile Lys Glu Glu Val Leu Ala Tyr Val Val Gln Leu Pro
       290                 295                 300
   Leu Tyr Gly Val Ile Asp Thr Pro Cys Trp Lys Leu His Thr Ser Pro
   305                 310                 315                 320
   Leu Cys Thr Thr Asn Thr Lys Glu Gly Ser Asn Ile Cys Leu Thr Arg
                   325                 330                 335
       Thr Asp Arg Gly Trp Tyr Cys Asp Asn Ala Gly Ser Val Ser Phe Phe
   340 345 350
   Pro Gln Ala Glu Thr Cys Lys Val Gln Ser Asn Arg Val Phe Cys Asp
   355 360 365
   Thr Met Asn Ser Leu Thr Leu Pro Ser Glu Val Asn Leu Cys Asn Val
   370 375 380
   Asp Ile Phe Asn Pro Lys Tyr Asp Cys Lys Ile Met Thr Ser Lys Thr
   385 390 395 400
   Asp Val Ser Ser Ser Val Ile Thr Ser Leu Gly Ala Ile Val Ser Cys
   405 410 415
   Tyr Gly Lys Thr Lys Cys Thr Ala Ser Asn Lys Asn Arg Gly Ile Ile
   420 425 430
   Lys Thr Phe Ser Asn Gly Cys Asp Tyr Val Ser Asn Lys Gly Val Asp
   435 440 445
   Thr Val Ser Val Gly Asn Thr Leu Tyr Tyr Val Asn Lys Gln Glu Gly
   450 455 460
   Lys Ser Leu Tyr Val Lys Gly Glu Pro Ile Ile Asn Phe Tyr Asp Pro
   465 470 475 480
   Leu Val Phe Pro Ser Asp Glu Phe Asp Ala Ser Ile Ser Gln Val Asn
   485 490 495
   Glu Lys Ile Asn Gln Ser Leu Ala Phe Ile Arg Lys Ser Asp Glu Leu
   500 505 510
   Leu His His Val Asn Ala Gly Lys Ser Thr Thr Asn Ile Met Ile Thr
   515 520 525
   Thr Ile Ile Ile Val Ile Ile Val Ile Leu Leu Ser Leu Ile Ala Val
   530 535 540
   Gly Leu Leu Leu Tyr Cys Lys Ala Arg Ser Thr Pro Val Thr Leu Ser
   545 550 555 560
   Lys Asp Gln Leu Ser Gly Ile Asn Asn Ile Ala Phe Ser Asn
   565 570
<210> 2
   <211> 298
   <212> PRT
   <213> respiratory syncytial virus
<400> 2
   Met Ser Lys Asn Lys Asp Gln Arg Thr Ala Lys Thr Leu Glu Lys Thr
   1 5 10 15
   Trp Asp Thr Leu Asn His Leu Leu Phe Ile Ser Ser Gly Leu Tyr Lys
   20 25 30
   Leu Asn Leu Lys Ser Ile Ala Gln Ile Thr Leu Ser Ile Leu Ala Met
   35 40 45
   Ile Ile Ser Thr Ser Leu Ile Ile Thr Ala Ile Ile Phe Ile Ala Ser
   50 55 60
   Ala Asn His Lys Val Thr Leu Thr Thr Ala Ile Ile Gln Asp Ala Thr
   65 70 75 80
   Ser Gln Ile Lys Asn Thr Thr Pro Thr Tyr Leu Thr Gln Asp Pro Gln
   85 90 95
   Leu Gly Ile Ser Phe Ser Asn Leu Ser Glu Ile Thr Ser Gln Thr Thr
   100 105 110
   Thr Ile Leu Ala Ser Thr Thr Pro Gly Val Lys Ser Asn Leu Gln Pro
   115 120 125
   Thr Thr Val Lys Thr Lys Asn Thr Thr Thr Thr Gln Thr Gln Pro Ser
   130 135 140
   Lys Pro Thr Thr Lys Gln Arg Gln Asn Lys Pro Pro Asn Lys Pro Asn
   145 150 155 160
   Asn Asp Phe His Phe Glu Val Phe Asn Phe Val Pro Cys Ser Ile Cys
   165 170 175
   Ser Asn Asn Pro Thr Cys Trp Ala Ile Cys Lys Arg Ile Pro Asn Lys
   180 185 190
   Lys Pro Gly Lys Lys Thr Thr Thr Lys Pro Thr Lys Lys Pro Thr Phe
   195 200 205
   Lys Thr Thr Lys Lys Asp Leu Lys Pro Gln Thr Thr Lys Pro Lys Glu
   210 215 220
   Val Pro Thr Thr Lys Pro Thr Glu Glu Pro Thr Ile Asn Thr Thr Lys
   225 230 235 240
   Thr Asn Ile Thr Thr Thr Leu Leu Thr Asn Asn Thr Thr Gly Asn Pro
   245 250 255
   Lys Leu Thr Ser Gln Met Glu Thr Phe His Ser Thr Ser Ser Glu Gly
   260 265 270
   Asn Leu Ser Pro Ser Gln Val Ser Thr Thr Ser Glu His Pro Ser Gln
   275 280 285
   Pro Ser Ser Pro Pro Asn Thr Thr Arg Gln
   290 295
<210> 3
   <211> 64
   <212> PRT
   <213> respiratory syncytial virus
<400> 3
   Met Glu Asn Thr Ser Ile Thr Ile Glu Phe Ser Ser Lys Phe Trp Pro
   1 5 10 15
   Tyr Phe Thr Leu Ile His Met Ile Thr Thr Ile Ile Ser Leu Leu Ile
   20 25 30
   Ile Ile Ser Ile Met Thr Ala Ile Leu Asn Lys Leu Cys Glu Tyr Asn
           35                  40                  45
   Val Phe His Asn Lys Thr Phe Glu Leu Pro Arg Ala Arg Val Asn Thr
       50                  55                  60
<210> 4
   <211> 256
   <212> PRT
   <213> respiratory syncytial virus
<400> 4
   Met Glu Thr Tyr Val Asn Lys Leu His Glu Gly Ser Thr Tyr Thr Ala
   1               5                   10                  15
   Ala Val Gln Tyr Asn Val Leu Glu Lys Asp Asp Asp Pro Ala Ser Leu
               20                  25                  30
   Thr Ile Trp Val Pro Met Phe Gln Ser Ser Met Pro Ala Asp Leu Leu
           35                  40                  45
   Ile Lys Glu Leu Ala Asn Val Asn Ile Leu Val Lys Gln Ile Ser Thr
       50                  55                  60
   Pro Lys Gly Pro Ser Leu Arg Val Met Ile Asn Ser Arg Ser Ala Leu
   65                  70                  75                  80
   Leu Ala Gln Met Pro Ser Lys Phe Thr Ile Cys Ala Asn Val Ser Leu
                   85                  90                  95
   Asp Glu Arg Ser Lys Leu Ala Tyr Asp Val Thr Thr Pro Cys Glu Ile
               100                 105                 110
   Lys Ala Cys Ser Leu Thr Cys Leu Lys Ser Lys Asn Met Leu Thr Thr
           115                 120                 125
   Val Lys Asp Leu Thr Met Lys Thr Leu Asn Pro Thr His Asp Ile Ile
       130                 135                 140
   Ala Leu Cys Glu Phe Glu Asn Ile Val Thr Ser Lys Lys Val Ile Ile
   145 150 155 160
   Pro Thr Tyr Leu Arg Ser Ile Ser Val Arg Asn Lys Asp Leu Asn Thr
   165 170 175
   Leu Glu Asn Ile Thr Thr Thr Glu Phe Lys Asn Ala Ile Thr Asn Ala
   180 185 190
   Lys Ile Ile Pro Tyr Ser Gly Leu Leu Leu Val Ile Thr Val Thr Asp
   195 200 205
   Asn Lys Gly Ala Phe Lys Tyr Ile Lys Pro Gln Ser Gln Phe Ile Val
   210 215 220
   Asp Leu Gly Ala Tyr Leu Glu Lys Glu Ser Ile Tyr Tyr Val Thr Thr
   225 230 235 240
   Asn Trp Lys His Thr Ala Thr Arg Phe Ala Ile Lys Pro Met Glu Asp
   245 250 255
<210> 5
   <211> 391
   <212> PRT
   <213> respiratory syncytial virus
<400> 5
   Met Ala Leu Ser Lys Val Lys Leu Asn Asp Thr Leu Asn Lys Asp Gln
   1 5 10 15
   Leu Leu Ser Ser Ser Lys Tyr Thr Ile Gln Arg Ser Thr Gly Asp Ser
   20 25 30
   Ile Asp Thr Pro Asn Tyr Asp Val Gln Lys His Ile Asn Lys Leu Cys
   35 40 45
   Gly Met Leu Leu Ile Thr Glu Asp Ala Asn His Lys Phe Thr Gly Leu
   50 55 60
   Ile Gly Met Leu Tyr Ala Met Ser Arg Leu Gly Arg Glu Asp Thr Ile
   65                  70                  75                  80
   Lys Ile Leu Arg Asp Ala Gly Tyr His Val Lys Ala Asn Gly Val Asp
                   85                  90                  95
   Val Thr Thr His Arg Gln Asp Ile Asn Gly Lys Glu Met Lys Phe Glu
               100                 105                 110
   Val Leu Thr Leu Ala Ser Leu Thr Thr Glu Ile Gln Ile Asn Ile Glu
           115                 120                 125
   Ile Glu Ser Arg Lys Ser Tyr Lys Lys Met Leu Lys Glu Met Gly Glu
       130                 135                 140
   Val Ala Pro Glu Tyr Arg His Asp Ser Pro Asp Cys Gly Met Ile Ile
   145                 150                 155                 160
   Leu Cys Ile Ala Ala Leu Val Ile Thr Lys Leu Ala Ala Gly Asp Arg
                   165                 170                 175
   Ser Gly Leu Thr Ala Val Ile Arg Arg Ala Asn Asn Val Leu Lys Asn
               180                 185                 190
   Glu Met Lys Arg Tyr Lys Gly Leu Leu Pro Lys Asp Ile Ala Asn Ser
           195                 200                 205
   Phe Tyr Glu Val Phe Glu Lys His Pro His Phe Ile Asp Val Phe Val
       210                 215                 220
   His Phe Gly Ile Ala Gln Ser Ser Thr Arg Gly Gly Ser Arg Val Glu
   225                 230                 235                 240
   Gly Ile Phe Ala Gly Leu Phe Met Asn Ala Tyr Gly Ala Gly Gln Val
                   245                 250                 255
   Met Leu Arg Trp Gly Val Leu Ala Lys Ser Val Lys Asn Ile Met Leu
               260                 265                 270
   Gly His Ala Ser Val Gln Ala Glu Met Glu Gln Val Val Glu Val Tyr
   275 280 285
   Glu Tyr Ala Gln Lys Leu Gly Gly Glu Ala Gly Phe Tyr His Ile Leu
   290 295 300
   Asn Asn Pro Lys Ala Ser Leu Leu Ser Leu Thr Gln Phe Pro His Phe
   305 310 315 320
   Ser Ser Val Val Leu Gly Asn Ala Ala Gly Leu Gly Ile Met Gly Glu
   325 330 335
   Tyr Arg Gly Thr Pro Arg Asn Gln Asp Leu Tyr Asp Ala Ala Lys Ala
   340 345 350
   Tyr Ala Glu Gln Leu Lys Glu Asn Gly Val Ile Asn Tyr Ser Val Leu
   355 360 365
   Asp Leu Thr Ala Glu Glu Leu Glu Ala Ile Lys His Gln Leu Asn Pro
   370 375 380
   Lys Asp Asn Asp Val Glu Leu
   385 390
<210> 6
   <211> 2165
   <212> PRT
   <213> respiratory syncytial virus
<400> 6
   Met Asp Pro Ile Ile Asn Gly Asn Ser Ala Asn Val Tyr Leu Thr Asp
   1 5 10 15
   Ser Tyr Leu Lys Gly Val Ile Ser Phe Ser Glu Cys Asn Ala Leu Gly
   20 25 30
   Ser Tyr Ile Phe Asn Gly Pro Tyr Leu Lys Asn Asp Tyr Thr Asn Leu
   35 40 45
   Ile Ser Arg Gln Asn Pro Leu Ile Glu His Met Asn Leu Lys Lys Leu
   50 55 60
   Asn Ile Thr Gln Ser Leu Ile Ser Lys Tyr His Lys Gly Glu Ile Lys
   65                  70                  75                  80
   Leu Glu Glu Pro Thr Tyr Phe Gln Ser Leu Leu Met Thr Tyr Lys Ser
                   85                  90                  95
   Met Thr Ser Leu Glu Gln Ile Ala Thr Thr Asn Leu Leu Lys Lys Ile
               100                 105                 110
   Ile Arg Arg Ala Ile Glu Ile Ser Asp Val Lys Val Tyr Ala Ile Leu
           115                 120                 125
   Asn Lys Leu Gly Leu Lys Glu Lys Asp Lys Ile Lys Ser Asn Asn Gly
       130                 135                 140
   Gln Asp Glu Asp Asn Ser Val Ile Thr Thr Ile Ile Lys Asp Asp Ile
   145                 150                 155                 160
   Leu Ser Ala Val Lys Asp Asn Gln Ser His Leu Lys Ala Asp Lys Asn
                   165                 170                 175
   His Ser Thr Lys Gln Lys Asp Thr Ile Lys Thr Thr Leu Leu Lys Lys
               180                 185                 190
   Leu Met Cys Ser Met Gln His Pro Pro Ser Trp Leu Ile His Trp Phe
           195                 200                 205
   Asn Leu Tyr Thr Lys Leu Asn Asn Ile Leu Thr Gln Tyr Arg Ser Asn
       210                 215                 220
   Glu Val Lys Asn His Gly Phe Ile Leu Ile Asp Asn Gln Thr Leu Ser
   225                 230                 235                 240
   Gly Phe Gln Phe Ile Leu Asn Gln Tyr Gly Cys Ile Val Tyr His Lys
                   245                 250                 255
   Glu Leu Lys Arg Ile Thr Val Thr Thr Tyr Asn Gln Phe Leu Thr Trp
               260                 265                 270
   Lys Asp Ile Ser Leu Ser Arg Leu Asn Val Cys Leu Ile Thr Trp Ile
   275 280 285
   Ser Asn Cys Leu Asn Thr Leu Asn Lys Ser Leu Gly Leu Arg Cys Gly
   290 295 300
   Phe Asn Asn Val Ile Leu Thr Gln Leu Phe Leu Tyr Gly Asp Cys Ile
   305 310 315 320
   Leu Lys Leu Phe His Asn Glu Gly Phe Tyr Ile Ile Lys Glu Val Glu
   325 330 335
   Gly Phe Ile Met Ser Leu Ile Leu Asn Ile Thr Glu Glu Asp Gln Phe
   340 345 350
   Arg Lys Arg Phe Tyr Asn Ser Met Leu Asn Asn Ile Thr Asp Ala Ala
   355 360 365
   Asn Lys Ala Gln Lys Asn Leu Leu Ser Arg Val Cys His Thr Leu Leu
   370 375 380
   Asp Lys Thr Val Ser Asp Asn Ile Ile Asn Gly Arg Trp Ile Ile Leu
   385 390 395 400
   Leu Ser Lys Phe Leu Lys Leu Ile Lys Leu Ala Gly Asp Asn Asn Leu
   405 410 415
   Asn Asn Leu Ser Glu Leu Tyr Phe Leu Phe Arg Ile Phe Gly His Pro
   420 425 430
   Met Val Asp Glu Arg Gln Ala Met Asp Ala Val Lys Val Asn Cys Asn
   435 440 445
   Glu Thr Lys Phe Tyr Leu Leu Ser Ser Leu Ser Met Leu Arg Gly Ala
   450 455 460
   Phe Ile Tyr Arg Ile Ile Lys Gly Phe Val Asn Asn Tyr Asn Arg Trp
   465 470 475 480
   Pro Thr Leu Arg Asn Ala Ile Val Leu Pro Leu Arg Trp Leu Thr Tyr
                   485                 490                 495
   Tyr Lys Leu Asn Thr Tyr Pro Ser Leu Leu Glu Leu Thr Glu Arg Asp
               500                 505                 510
   Leu Ile Val Leu Ser Gly Leu Arg Phe Tyr Arg Glu Phe Arg Leu Pro
           515                 520                 525
   Lys Lys Val Asp Leu Glu Met Ile Ile Asn Asp Lys Ala Ile Ser Pro
       530                 535                 540
   Pro Lys Asn Leu Ile Trp Thr Ser Phe Pro Arg Asn Tyr Met Pro Ser
   545                 550                 555                 560
   His Ile Gln Asn Tyr Ile Glu His Glu Lys Leu Lys Phe Ser Glu Ser
                   565                 570                 575
   Asp Lys Ser Arg Arg Val Leu Glu Tyr Tyr Leu Arg Asp Asn Lys Phe
               580                 585                 590
   Asn Glu Cys Asp Leu Tyr Asn Cys Val Val Asn Gln Ser Tyr Leu Asn
           595                 600                 605
   Asn Pro Asn His Val Val Ser Leu Thr Gly Lys Glu Arg Glu Leu Ser
       610                 615                 620
   Val Gly Arg Met Phe Ala Met Gln Pro Gly Met Phe Arg Gln Val Gln
   625                 630                 635                 640
   Ile Leu Ala Glu Lys Met Ile Ala Glu Asn Ile Leu Gln Phe Phe Pro
                   645                 650                 655
   Glu Ser Leu Thr Arg Tyr Gly Asp Leu Glu Leu Gln Lys Ile Leu Glu
               660                 665                 670
   Leu Lys Ala Gly Ile Ser Asn Lys Ser Asn Arg Tyr Asn Asp Asn Tyr
           675                 680                 685
   Asn Asn Tyr Ile Ser Lys Cys Ser Ile Ile Thr Asp Leu Ser Lys Phe
   690 695 700
   Asn Gln Ala Phe Arg Tyr Glu Thr Ser Cys Ile Cys Ser Asp Val Leu
   705 710 715 720
   Asp Glu Leu His Gly Val Gln Ser Leu Phe Ser Trp Leu His Leu Thr
   725 730 735
   Ile Pro His Val Thr Ile Ile Cys Thr Tyr Arg His Ala Pro Pro Tyr
   740 745 750
   Ile Arg Asp His Ile Val Asp Leu Asn Asn Val Asp Glu Gln Ser Gly
   755 760 765
   Leu Tyr Arg Tyr His Met Gly Gly Ile Glu Gly Trp Cys Gln Lys Leu
   770 775 780
   Trp Thr Ile Glu Ala Ile Ser Leu Leu Asp Leu Ile Ser Leu Lys Gly
   785 790 795 800
   Lys Phe Ser Ile Thr Ala Leu Ile Asn Gly Asp Asn Gln Ser Ile Asp
   805 810 815
   Ile Ser Lys Pro Val Arg Leu Met Glu Gly Gln Thr His Ala Gln Ala
   820 825 830
   Asp Tyr Leu Leu Ala Leu Asn Ser Leu Lys Leu Leu Tyr Lys Glu Tyr
   835 840 845
   Ala Gly Ile Gly His Lys Leu Lys Gly Thr Glu Thr Tyr Ile Ser Arg
   850 855 860
   Asp Met Gln Phe Met Ser Lys Thr Ile Gln His Asn Gly Val Tyr Tyr
   865 870 875 880
   Pro Ala Ser Ile Lys Lys Val Leu Arg Val Gly Pro Trp Ile Asn Thr
   885 890 895
   Ile Leu Asp Asp Phe Lys Val Ser Leu Glu Ser Ile Gly Ser Leu Thr
             900                 905                 910
   Gln Glu Leu Glu Tyr Arg Gly Glu Ser Leu Leu Cys Ser Leu Ile Phe
           915                 920                 925
   Arg Asn Val Trp Leu Tyr Asn Gln Ile Ala Leu Gln Leu Lys Asn His
       930                 935                 940
   Ala Leu Cys Asn Asn Lys Leu Tyr Leu Asp Ile Leu Lys Val Leu Lys
   945                 950                 955                 960
   His Leu Lys Thr Phe Phe Asn Leu Asp Asn Ile Asp Thr Ala Leu Thr
                   965                 970                 975
   Leu Tyr Met Asn Leu Pro Met Leu Phe Gly Gly Gly Asp Pro Asn Leu
               980                 985                 990
   Leu Tyr Arg Ser Phe Tyr Arg Arg  Thr Pro Asp Phe Leu  Thr Glu Ala
           995                 1000                 1005
   Ile Val  His Ser Val Phe Ile  Leu Ser Tyr Tyr Thr  Asn His Asp
       1010                 1015                 1020
   Leu Lys  Asp Lys Leu Gln Asp  Leu Ser Asp Asp Arg  Leu Asn Lys
       1025                 1030                 1035
   Phe Leu  Thr Cys Ile Ile Thr  Phe Asp Lys Asn Pro  Asn Ala Glu
       1040                 1045                 1050
   Phe Val  Thr Leu Met Arg Asp  Pro Gln Ala Leu Gly  Ser Glu Arg
       1055                 1060                 1065
   Gln Ala  Lys Ile Thr Ser Glu  Ile Asn Arg Leu Ala  Val Thr Glu
       1070                 1075                 1080
   Val Leu  Ser Thr Ala Pro Asn  Lys Ile Phe Ser Lys  Ser Ala Gln
       1085                 1090                 1095
   His Tyr  Thr Thr Thr Glu Ile  Asp Leu Asn Asp Ile  Met Gln Asn
       1100                 1105                 1110
   Ile Glu  Pro Thr Tyr Pro His  Gly Leu Arg Val Val  Tyr Glu Ser
       1115                 1120                 1125
   Leu Pro  Phe Tyr Lys Ala Glu  Lys Ile Val Asn Leu  Ile Ser Gly
       1130                 1135                 1140
   Thr Lys  Ser Ile Thr Asn Ile  Leu Glu Lys Thr Ser  Ala Ile Asp
       1145                 1150                 1155
   Leu Thr  Asp Ile Asp Arg Ala  Thr Glu Met Met Arg  Lys Asn Ile
       1160                 1165                 1170
   Thr Leu  Leu Ile Arg Ile Leu  Pro Leu Asp Cys Asn  Arg Asp Lys
       1175                 1180                 1185
   Arg Glu  Ile Leu Ser Met Glu  Asn Leu Ser Ile Thr  Glu Leu Ser
       1190                 1195                 1200
   Lys Tyr  Val Arg Glu Arg Ser  Trp Ser Leu Ser Asn  Ile Val Gly
       1205                 1210                 1215
   Val Thr  Ser Pro Ser Ile Met  Tyr Thr Met Asp Ile  Lys Tyr Thr
       1220                 1225                 1230
   Thr Ser  Thr Ile Ala Ser Gly  Ile Ile Ile Glu Lys  Tyr Asn Val
       1235                 1240                 1245
   Asn Ser  Leu Thr Arg Gly Glu  Arg Gly Pro Thr Lys  Pro Trp Val
       1250                 1255                 1260
   Gly Ser  Ser Thr Gln Glu Lys  Lys Thr Met Pro Val  Tyr Asn Arg
       1265                 1270                 1275
   Gln Val  Leu Thr Lys Lys Gln  Arg Asp Gln Ile Asp  Leu Leu Ala
       1280                 1285                 1290
   Lys Leu Asp Trp Val Tyr Ala Ser Ile Asp Asn Lys Asp Glu Phe
   1295 1300 1305
   Met Glu Glu Leu Ser Ile Gly Thr Leu Gly Leu Thr Tyr Glu Lys
   1310 1315 1320
   Ala Lys Lys Leu Phe Pro Gln Tyr Leu Ser Val Asn Tyr Leu His
   1325 1330 1335
   Arg Leu Thr Val Ser Ser Arg Pro Cys Glu Phe Pro Ala Ser Ile
   1340 1345 1350
   Pro Ala Tyr Arg Thr Thr Asn Tyr His Phe Asp Thr Ser Pro Ile
   1355 1360 1365
   Asn Arg Ile Leu Thr Glu Lys Tyr Gly Asp Glu Asp Ile Asp Ile
   1370 1375 1380
   Val Phe Gln Asn Cys Ile Ser Phe Gly Leu Ser Leu Met Ser Val
   1385 1390 1395
   Val Glu Gln Phe Thr Asn Val Cys Pro Asn Arg Ile Ile Leu Ile
   1400 1405 1410
   Pro Lys Leu Asn Glu Ile His Leu Met Lys Pro Pro Ile Phe Thr
   1415 1420 1425
   Gly Asp Val Asp Ile His Lys Leu Lys Gln Val Ile Gln Lys Gln
   1430 1435 1440
   His Met Phe Leu Pro Asp Lys Ile Ser Leu Thr Gln Tyr Val Glu
   1445 1450 1455
   Leu Phe Leu Ser Asn Lys Thr Leu Lys Ser Gly Ser His Val Asn
   1460 1465 1470
   Ser Asn Leu Ile Leu Ala His Lys Ile Ser Asp Tyr Phe His Asn
   1475 1480 1485
   Thr Tyr Ile Leu Ser Thr Asn Leu Ala Gly His Trp Ile Leu Ile
   1490 1495 1500
   Ile Gln Leu Met Lys Asp Ser Lys Gly Ile Phe Glu Lys Asp Trp
   1505 1510 1515
   Gly Glu Gly Tyr Ile Thr Asp His Met Phe Ile Asn Leu Lys Val
   1520 1525 1530
   Phe Phe Asn Ala Tyr Lys Thr Tyr Leu Leu Cys Phe His Lys Gly
   1535 1540 1545
   Tyr Gly Lys Ala Lys Leu Glu Cys Asp Met Asn Thr Ser Asp Leu
   1550 1555 1560
   Leu Cys Val Leu Glu Leu Ile Asp Ser Ser Tyr Trp Lys Ser Met
   1565 1570 1575
   Ser Lys Val Phe Leu Glu Gln Lys Val Ile Lys Tyr Ile Leu Ser
   1580 1585 1590
   Gln Asp Ala Ser Leu His Arg Val Lys Gly Cys His Ser Phe Lys
   1595 1600 1605
   Leu Trp Phe Leu Lys Arg Leu Asn Val Ala Glu Phe Thr Val Cys
   1610 1615 1620
   Pro Trp Val Val Asn Ile Asp Tyr His Pro Thr His Met Lys Ala
   1625 1630 1635
   Ile Leu Thr Tyr Ile Asp Leu Val Arg Met Gly Leu Ile Asn Ile
   1640 1645 1650
   Asp Arg Ile His Ile Lys Asn Lys His Lys Phe Asn Asp Glu Phe
   1655 1660 1665
   Tyr Thr Ser Asn Leu Phe Tyr Ile Asn Tyr Asn Phe Ser Asp Asn
   1670 1675 1680
   Thr His Leu Leu Thr Lys His Ile Arg Ile Ala Asn Ser Glu Leu
       1685                1690                1695
   Glu Asn Asn Tyr Asn Lys Leu Tyr His Pro Thr Pro Glu Thr Leu
       1700                1705                1710
   Glu Asn Ile Leu Ala Asn Pro Ile Lys Ser Asn Asp Lys Lys Thr
       1715                1720                1725
   Leu Asn Asp Tyr Cys Ile Gly Lys Asn Val Asp Ser Ile Met Leu
       1730                1735                1740
   Pro Leu Leu Ser Asn Lys Lys Leu Val Lys Ser Ser Ala Met Ile
       1745                1750                1755
   Arg Thr Asn Tyr Ser Lys Gln Asp Leu Tyr Asn Leu Phe Pro Thr
       1760                1765                1770
   Val Val Ile Asp Arg Ile Ile Asp His Ser Gly Asn Thr Ala Lys
       1775                1780                1785
   Ser Asn Gln Leu Tyr Thr Thr Thr Ser His Gln Ile Ser Leu Val
       1790                1795                1800
   His Asn Ser Thr Ser Leu Tyr Cys Met Leu Pro Trp His His Ile
       1805                1810                1815
   Asn Arg Phe Asn Phe Val Phe Ser Ser Thr Gly Cys Lys Ile Ser
       1820                1825                1830
   Ile Glu Tyr Ile Leu Lys Asp Leu Lys Ile Lys Asp Pro Asn Cys
       1835                1840                1845
   Ile Ala Phe Ile Gly Glu Gly Ala Gly Asn Leu Leu Leu Arg Thr
       1850                1855                1860
   Val Val Glu Leu His Pro Asp Ile Arg Tyr Ile Tyr Arg Ser Leu
       1865                1870                1875
   Lys Asp Cys Asn Asp His Ser Leu Pro Ile Glu Phe Leu Arg Leu
   1880 1885 1890
   Tyr Asn Gly His Ile Asn Ile Asp Tyr Gly Glu Asn Leu Thr Ile
   1895 1900 1905
   Pro Ala Thr Asp Ala Thr Asn Asn Ile His Trp Ser Tyr Leu His
   1910 1915 1920
   Ile Lys Phe Ala Glu Pro Ile Ser Leu Phe Val Cys Asp Ala Glu
   1925 1930 1935
   Leu Pro Val Thr Val Asn Trp Ser Lys Ile Ile Ile Glu Trp Ser
   1940 1945 1950
   Lys His Val Arg Lys Cys Lys Tyr Cys Ser Ser Val Asn Lys Cys
   1955 1960 1965
   Thr Leu Ile Val Lys Tyr His Ala Gln Asp Asp Ile Asp Phe Lys
   1970 1975 1980
   Leu Asp Asn Ile Thr Ile Leu Lys Thr Tyr Val Cys Leu Gly Ser
   1985 1990 1995
   Lys Leu Lys Gly Ser Glu Val Tyr Leu Val Leu Thr Ile Gly Pro
   2000 2005 2010
   Ala Asn Ile Phe Pro Val Phe Asn Val Val Gln Asn Ala Lys Leu
   2015 2020 2025
   Ile Leu Ser Arg Thr Lys Asn Phe Ile Met Pro Lys Lys Ala Asp
   2030 2035 2040
   Lys Glu Ser Ile Asp Ala Asn Ile Lys Ser Leu Ile Pro Phe Leu
   2045 2050 2055
   Cys Tyr Pro Ile Thr Lys Lys Gly Ile Asn Thr Ala Leu Ser Lys
   2060 2065 2070
   Leu Lys Ser Val Val Ser Gly Asp Ile Leu Ser Tyr Ser Ile Ala
   2075 2080 2085
   Gly Arg Asn Glu Val Phe Ser Asn Lys Leu Ile Asn His Lys His
   2090 2095 2100
   Met Asn Ile Leu Lys Trp Phe Asn His Val Leu Asn Phe Arg Ser
   2105 2110 2115
   Thr Glu Leu Asn Tyr Asn His Leu Tyr Met Val Glu Ser Thr Tyr
   2120 2125 2130
   Pro Tyr Leu Ser Glu Leu Leu Asn Ser Leu Thr Thr Asn Glu Leu
   2135 2140 2145
   Lys Lys Leu Ile Lys Ile Thr Gly Ser Leu Leu Tyr Asn Phe His
   2150 2155 2160
   Asn Glu
   2165
<210> 7
   <211> 194
   <212> PRT
   <213> respiratory syncytial virus
<400> 7
   Met Ser Arg Arg Asn Pro Cys Lys Phe Glu Ile Arg Gly His Cys Leu
   1 5 10 15
   Asn Gly Lys Arg Cys His Phe Ser His Asn Tyr Phe Glu Trp Pro Pro
   20 25 30
   His Ala Leu Leu Val Arg Gln Asn Phe Met Leu Asn Arg Ile Leu Lys
   35 40 45
   Ser Met Asp Lys Ser Ile Asp Thr Leu Ser Glu Ile Ser Gly Ala Ala
   50 55 60
   Glu Leu Asp Arg Thr Glu Glu Tyr Ala Leu Gly Val Val Gly Val Leu
   65 70 75 80
   Glu Ser Tyr Ile Gly Ser Ile Asn Asn Ile Thr Lys Gln Ser Ala Cys
   85 90 95
   Val Ala Met Ser Lys Leu Leu Thr Glu Leu Asn Ser Asp Asp Ile Lys
   100 105 110
   Lys Leu Arg Asp Asn Glu Glu Leu Asn Ser Pro Lys Ile Arg Val Tyr
   115 120 125
   Asn Thr Val Ile Ser Tyr Ile Glu Ser Asn Arg Lys Asn Asn Lys Gln
   130 135 140
   Thr Ile His Leu Leu Lys Arg Leu Pro Ala Asp Val Leu Lys Lys Thr
   145 150 155 160
   Ile Lys Asn Thr Leu Asp Ile His Lys Ser Ile Thr Ile Asn Asn Pro
   165 170 175
   Lys Glu Leu Thr Val Ser Asp Thr Asn Asp His Ala Lys Asn Asn Asp
   180 185 190
   Thr Thr
<210> 8
   <211> 90
   <212> PRT
   <213> respiratory syncytial virus
<400> 8
   Met Thr Met Pro Lys Ile Met Ile Leu Pro Asp Lys Tyr Pro Cys Ser
   1 5 10 15
   Ile Thr Ser Ile Leu Ile Thr Ser Arg Cys Arg Val Thr Met Tyr Asn
   20 25 30
   Arg Lys Asn Thr Leu Tyr Phe Asn Gln Asn Asn Pro Asn Asn His Met
   35 40 45
   Tyr Ser Pro Asn Gln Thr Phe Asn Glu Ile His Trp Thr Ser Gln Asp
   50 55 60
   Leu Ile Asp Thr Ile Gln Asn Phe Leu Gln His Leu Gly Val Ile Glu
   65 70 75 80
   Asp Ile Tyr Thr Ile Tyr Ile Leu Val Ser
   85 90
<210> 9
   <211> 241
   <212> PRT
   <213> respiratory syncytial virus
<400> 9
   Met Glu Lys Phe Ala Pro Glu Phe His Gly Glu Asp Ala Asn Asn Arg
   1 5 10 15
   Ala Thr Lys Phe Leu Glu Ser Ile Lys Gly Lys Phe Thr Ser Pro Lys
   20 25 30
   Asp Pro Lys Lys Lys Asp Ser Ile Ile Ser Val Asn Ser Ile Asp Ile
   35 40 45
   Glu Val Thr Lys Glu Ser Pro Ile Thr Ser Asn Ser Thr Ile Ile Asn
   50 55 60
   Pro Thr Asn Glu Thr Asp Asp Asn Ala Gly Asn Lys Pro Asn Tyr Gln
   65 70 75 80
   Arg Lys Pro Leu Val Ser Phe Lys Glu Asp Pro Ile Pro Ser Asp Asn
   85 90 95
   Pro Phe Ser Lys Leu Tyr Lys Glu Thr Ile Glu Thr Phe Asp Asn Asn
   100 105 110
   Glu Glu Glu Ser Ser Tyr Ser Tyr Glu Glu Ile Asn Asp Gln Thr Asn
   115 120 125
   Asp Asn Ile Thr Ala Arg Leu Asp Arg Ile Asp Glu Lys Leu Ser Glu
   130 135 140
   Ile Leu Gly Met Leu His Thr Leu Val Val Ala Ser Ala Gly Pro Thr
   145 150 155 160
   Ser Ala Arg Asp Gly Ile Arg Asp Ala Met Val Gly Leu Arg Glu Glu
   165 170 175
   Met Ile Glu Lys Ile Arg Thr Glu Ala Leu Met Thr Asn Asp Arg Leu
   180 185 190
   Glu Ala Met Ala Arg Leu Arg Asn Glu Glu Ser Glu Lys Met Ala Lys
   195 200 205
   Asp Thr Ser Asp Glu Val Ser Leu Asn Pro Thr Ser Glu Lys Leu Asn
   210 215 220
   Asn Leu Leu Glu Gly Asn Asp Ser Asp Asn Asp Leu Ser Leu Glu Asp
   225 230 235 240
   Phe
<210> 10
   <211> 139
   <212> PRT
   <213> respiratory syncytial virus
<400> 10
   Met Gly Ser Asn Ser Leu Ser Met Ile Lys Val Arg Leu Gln Asn Leu
   1 5 10 15
   Phe Asp Asn Asp Glu Val Ala Leu Leu Lys Ile Thr Cys Tyr Thr Asp
   20 25 30
   Lys Leu Ile His Leu Thr Asn Ala Leu Ala Lys Ala Val Ile His Thr
   35 40 45
   Ile Lys Leu Asn Gly Ile Val Phe Val His Val Ile Thr Ser Ser Asp
       50                  55                  60
   Ile Cys Pro Asn Asn Asn Ile Val Val Lys Ser Asn Phe Thr Thr Met
   65                  70                  75                  80
   Pro Val Leu Gln Asn Gly Gly Tyr Ile Trp Glu Met Met Glu Leu Thr
                   85                  90                  95
   His Cys Ser Gln Pro Asn Gly Leu Ile Asp Asp Asn Cys Glu Ile Lys
               100                 105                 110
   Phe Ser Lys Lys Leu Ser Asp Ser Thr Met Thr Asn Tyr Met Asn Gln
           115                 120                 125
   Leu Ser Glu Leu Leu Gly Phe Asp Leu Asn Pro
       130                 135
<210> 11
   <211> 124
   <212> PRT
   <213> respiratory syncytial virus
<400> 11
   Met Asp Thr Thr His Asn Asp Thr Thr Pro Gln Arg Leu Met Ile Thr
   1               5                   10                  15
   Asp Met Arg Pro Leu Ser Leu Glu Thr Thr Ile Thr Ser Leu Thr Arg
               20                  25                  30
   Asp Ile Ile Thr His Arg Phe Ile Tyr Leu Ile Asn His Glu Cys Ile
           35                  40                  45
   Val Arg Lys Leu Asp Glu Arg Gln Ala Thr Phe Thr Phe Leu Val Asn
       50                  55                  60
   Tyr Glu Met Lys Leu Leu His Lys Val Gly Ser Thr Lys Tyr Lys Lys
   65                  70                  75                  80
   Tyr Thr Glu Tyr Asn Thr Lys Tyr Gly Thr Phe Pro Met Pro Ile Phe
   85 90 95
   Ile Asn His Asp Gly Phe Leu Glu Cys Ile Gly Ile Lys Pro Thr Lys
   100 105 110
   His Thr Pro Ile Ile Tyr Lys Tyr Asp Leu Asn Pro
   115 120
<210> 12
   <211> 1725
   <212> RNA
   <213> respiratory syncytial virus
<400> 12
   auggaguugc caauccucaa agcaaaugca auuaccacaa uccucgcugc agucacauuu 60
   ugcuuugcuu cuagucaaaa caucacugaa gaauuuuauc aaucaacaug cagugcaguu 120
   agcaaaggcu aucuuagugc ucuaagaacu gguugguaua cuaguguuau aacuauagaa 180
   uuaaguaaua ucaaggaaaa uaaguguaau ggaacagaug cuaagguaaa auugauaaac 240
   caagaauuag auaaauauaa aaaugcugua acagaauugc aguugcucau gcaaagcaca 300
   acagcagcaa acaaucgagc cagaagagaa cuaccaaggu uuaugaauua uacacucaac 360
   aauaccaaaa aaaccaaugu aacauuaagc aagaaaagga aaagaagauu ucuugguuuu 420
   uuguuaggug uuggaucugc aaucgccagu ggcauugcug uaucuaaggu ccugcacuua 480
   gaaggagaag ugaacaagau caaaagugcu cuacuaucca caaacaaggc cguagucagc 540
   uuaucaaaug gaguuagugu cuuaaccagc aaaguguuag accucaaaaa cuauauagau 600
   aaacaauugu uaccuauugu gaauaagcaa agcugcagaa uaucaaauau agaaacugug 660
   auagaguucc aacaaaagaa caacagacua cuagagauua ccagggaauu uaguguuaau 720
   gcagguguaa cuacaccugu aagcacuuac auguuaacua auagugaauu auugucauua 780
   aucaaugaua ugccuauaac aaaugaucag aaaaaguuaa uguccaacaa uguucaaaua 840
   guuagacagc aaaguuacuc uaucaugucc auaauaaaag aggaagucuu agcauaugua 900
   guacaauuac cacuauaugg ugugauagau acaccuuguu ggaaauuaca cacauccccu 960
   cuauguacaa ccaacacaaa agaaggguca aacaucuguu uaacaagaac ugacagagga 1020
   ugguacugug acaaugcagg aucaguaucu uucuucccac aagcugaaac auguaaaguu 1080
   caaucgaauc gaguauuuug ugacacaaug aacaguuuaa cauuaccaag ugaaguaaau 1140
   cucugcaaug uugacauauu caaucccaaa uaugauugua aaauuaugac uucaaaaaca 1200
   gauguaagca gcuccguuau cacaucucua ggagccauug ugucaugcua uggcaaaacu 1260
   aaauguacag cauccaauaa aaaucgugga aucauaaaga cauuuucuaa cgggugugau 1320
   uauguaucaa auaaaggggu ggacacugug ucuguaggua acacauuaua uuauguaaau 1380
   aagcaagaag gcaaaagucu cuauguaaaa ggugaaccaa uaauaaauuu cuaugaccca 1440
   uuaguauucc ccucugauga auuugaugca ucaauaucuc aagucaauga gaagauuaac 1500
   cagaguuuag cauuuauucg uaaauccgau gaauuauuac aucauguaaa ugcugguaaa 1560
   ucaaccacaa auaucaugau aacuacuaua auuauaguga uuauaguaau auuguuauca 1620
   uuaauugcug uuggacugcu ccuauacugu aaggccagaa gcacaccagu cacacuaagc 1680
   aaggaucaac ugagugguau aaauaauauu gcauuuagua acuga 1725
<210> 13
   <211> 897
   <212> RNA
   <213> respiratory syncytial virus
<400> 13
   auguccaaaa acaaggacca acgcaccgcu aagacacuag aaaagaccug ggacacucuc 60
   aaucauuuau uauucauauc aucgggcuua uauaaguuaa aucuuaaauc uauagcacaa 120
   aucacauuau ccauucuggc aaugauaauc ucaacuucac uuauaauuac agccaucaua 180
   uucauagccu cggcaaacca caaagucaca cuaacaacug caaucauaca agaugcaaca 240
   agccagauca agaacacaac cccaacauac cucacucagg auccucagcu uggaaucagc 300
   uucuccaauc ugucugaaau uacaucacaa accaccacca uacuagcuuc aacaacacca 360
   ggagucaagu caaaccugca acccacaaca gucaagacua aaaacacaac aacaacccaa 420
   acacaaccca gcaagcccac uacaaaacaa cgccaaaaca aaccaccaaa caaacccaau 480
   aaugauuuuc acuucgaagu guuuaacuuu guacccugca gcauaugcag caacaaucca 540
   accugcuggg cuaucugcaa aagaauacca aacaaaaaac caggaaagaa aaccaccacc 600
   aagccuacaa aaaaaccaac cuucaagaca accaaaaaag aucucaaacc ucaaaccacu 660
   aaaccaaagg aaguacccac caccaagccc acagaagagc caaccaucaa caccaccaaa 720
   acaaacauca caacuacacu gcucaccaac aacaccacag gaaauccaaa acucacaagu 780
   caaauggaaa ccuuccacuc aaccuccucc gaaggcaauc uaagcccuuc ucaagucucc 840
   acaacauccg agcacccauc acaacccuca ucuccaccca acacaacacg ccaguag 897
<210> 14
   <211> 195
   <212> RNA
   <213> respiratory syncytial virus
<400> 14
   auggaaaaua cauccauaac aauagaauuc ucaagcaaau ucuggccuua cuuuacacua 60
   auacacauga ucacaacaau aaucucuuug cuaaucauaa ucuccaucau gacugcaaua 120
   cuaaacaaac uuugugaaua uaacguauuc cauaacaaaa ccuuugaguu accaagagcu 180
   cgagucaaca cauag 195
<210> 15
   <211> 771
   <212> RNA
   <213> respiratory syncytial virus
<400> 15
   auggaaacau acgugaacaa gcuucacgaa ggcuccacau acacagcugc uguucaauac 60
   aauguccuag aaaaagacga ugacccugca ucacuuacaa uaugggugcc cauguuccaa 120
   ucaucuaugc cagcagauuu acuuauaaaa gaacuagcua augucaacau acuagugaaa 180
   caaauaucca cacccaaggg accuucacua agagucauga uaaacucaag aagugcauug 240
   cuagcacaaa ugcccagcaa auuuaccaua ugugcuaaug uguccuugga ugaaagaagc 300
   aaacuggcau augauguaac cacacccugu gaaaucaagg cauguagucu aacaugccua 360
   aaaucaaaaa auauguuaac uacaguuaaa gaucucacua ugaagacacu caaccccaca 420
   caugauauua uugcuuuaug ugaauuugaa aacauaguaa caucaaaaaa agucauaaua 480
   ccaacauacc uaagauccau cagugucaga aauaaagauc ugaacacacu ugaaaauaua 540
   acaaccacug aauucaaaaa ugccaucaca aaugcaaaaa ucaucccuua cucaggauua 600
   cuauuaguca ucacagugac ugacaacaaa ggagcauuca aauacauaaa gccgcaaagu 660
   caauucauag uagaucuugg agcuuaccua gaaaaagaaa guauauauua uguuaccaca 720
   aauuggaagc acacagcuac acgauuugca aucaaaccca uggaagauua a 771
<210> 16
   <211> 1176
   <212> RNA
   <213> respiratory syncytial virus
<400> 16
   auggcucuua gcaaagucaa guugaaugau acacucaaca aagaucaacu ucugucaucu 60
   agcaaauaca ccauccaacg gagcacagga gauaguauug auacuccuaa uuaugaugug 120
   cagaaacaca ucaauaaguu auguggcaug uuauuaauca cagaagaugc uaaucauaaa 180
   uucacugggu uaauagguau guuauaugcu augucuaggu uaggaagaga agacaccaua 240
   aaaauacuca gagaugcggg auaucaugua aaagcaaaug gaguagaugu aacaacacau 300
   cgucaagaca ucaaugggaa agaaaugaaa uuugaagugu uaacauuggc aagcuuaaca 360
   acugaaauuc aaaucaacau ugagauagaa ucuagaaaau ccuacaaaaa aaugcuaaaa 420
   gaaaugggag agguagcucc agaauacagg caugauucuc cugauugugg gaugauaaua 480
   uuauguauag cagcauuagu aauaaccaaa uuggcagcag gggauagauc uggucuuaca 540
   gccgugauua ggagagcuaa uaauguccua aaaaaugaaa ugaaacguua caaaggcuua 600
   cuacccaagg auauagccaa cagcuucuau gaaguguuug aaaaacaucc ccacuuuaua 660
   gauguuuuug uucauuuugg uauagcacaa ucuuccacca gagguggcag uagaguugaa 720
   gggauuuuug caggauuguu uaugaaugcc uauggugcag ggcaaguaau gcuacggugg 780
   ggagucuuag caaaaucagu uaaaaauauu auguuaggac augcuagugu gcaagcagaa 840
   auggaacaag uuguugaggu uuaugaauau gcccaaaaau uggguggaga agcaggauuc 900
   uaccauauau ugaacaaccc aaaagcauca uuauuaucuu ugacucaauu uccucacuuu 960
   uccaguguag uauuaggcaa ugcugcuggc cuaggcauaa ugggagagua cagagguaca 1020
   ccgaggaauc aagaucuaua ugaugcagca aaggcauaug cugaacaacu caaagaaaau 1080
   ggugugauua acuacagugu auuagacuug acagcagaag aacuagaggc uaucaaacau 1140
   cagcuuaauc caaaagauaa ugauguagag cuuuga 1176
<210> 17
   <211> 6498
   <212> RNA
   <213> respiratory syncytial virus
<400> 17
   auggauccca uuauuaaugg aaauucugcu aauguuuauc uaaccgauag uuauuuaaaa      60
   gguguuaucu cuuucucaga guguaaugcu uuaggaaguu acauauucaa ugguccuuau     120
   cucaaaaaug auuauaccaa cuuaauuagu agacaaaauc cauuaauaga acacaugaau     180
   cuaaagaaac uaaauauaac acaguccuua auaucuaagu aucauaaagg ugaaauaaaa     240
   uuagaagagc cuacuuauuu ucagucauua cuuaugacau acaagaguau gaccucguug     300
   gaacagauug cuaccacuaa uuuacuuaaa aagauaauaa gaagagcuau agaaauaagu     360
   gaugucaaag ucuaugcuau auugaauaaa cuagggcuua aagaaaagga caagauuaaa     420
   uccaacaaug gacaggauga agacaacuca guuauuacga ccauaaucaa agaugauaua     480
   cuuucagcug uuaaggauaa ucaaucucau cuuaaagcag acaaaaauca cucuacaaaa     540
   caaaaagaca caaucaaaac aacacucuug aagaaauuaa uguguucaau gcagcauccu     600
   ccaucauggu uaauacauug guuuaauuua uacacaaaau uaaacaacau auuaacacag     660
   uaucgaucaa augagguuaa aaaccauggg uuuauauuga uagauaauca aacucuuagu     720
   ggauuucaau uuauuuugaa ucaauauggu uguauaguuu aucauaagga acucaaaaga     780
   auuacuguga caaccuauaa ucaauucuug acauggaaag auauuagccu uaguagauua     840
   aauguuuguu uaauuacaug gauuaguaac ugcuugaaca cauuaaauaa aagcuuaggc     900
   uuaagaugcg gauucaauaa uguuaucuug acacaacuau uccuuuaugg ugauuguaua     960
   cuaaagcuau uucacaauga gggguucuac auaauaaaag agguagaggg auuuauuaug    1020
   ucucuaauuu uaaauauaac agaagaagau caauucagaa aacgauuuua uaauaguaug    1080
   cucaacaaca ucacagaugc ugcuaauaaa gcucagaaaa aucugcuauc aagaguaugu    1140
   cauacauuau uagauaagac aguauccgau aauauaauaa auggcagaug gauaauucua    1200
   uuaaguaagu uccuuaaauu aauuaagcuu gcaggugaca auaaccuuaa caaucugagu    1260
   gaacuauauu uuuuguucag aauauuugga cacccaaugg uagaugaaag acaagccaug    1320
   gaugcuguua aaguuaauug caaugagacc aaauuuuacu uguuaagcag uuugaguaug 1380
   uuaagaggug ccuuuauaua uagaauuaua aaaggguuug uaaauaauua caacagaugg 1440
   ccuacuuuaa gaaaugcuau uguuuuaccc uuaagauggu uaacuuacua uaaacuaaac 1500
   acuuauccuu cuuuguugga acuuacagaa agagauuuga uuguguuauc aggacuacgu 1560
   uucuaucgug aguuucgguu gccuaaaaaa guggaucuug aaaugauuau aaaugauaaa 1620
   gcuauaucac ccccuaaaaa uuugauaugg acuaguuucc cuagaaauua uaugccguca 1680
   cacauacaaa acuauauaga acaugaaaaa uuaaaauuuu ccgagaguga uaaaucaaga 1740
   agaguauuag aguauuauuu aagagauaac aaauucaaug aaugugauuu auacaacugu 1800
   guaguuaauc aaaguuaucu caacaacccu aaucaugugg uaucauugac aggcaaagaa 1860
   agagaacuca guguagguag aauguuugca augcaaccgg gaauguucag acagguucaa 1920
   auauuggcag agaaaaugau agcugaaaac auuuuacaau ucuuuccuga aagucuuaca 1980
   agauauggug aucuagaacu acaaaaaaua uuagaauuga aagcaggaau aaguaacaaa 2040
   ucaaaucgcu acaaugauaa uuacaacaau uacauuagua agugcucuau caucacagau 2100
   cucagcaaau ucaaucaagc auuucgauau gaaacgucau guauuuguag ugaugugcug 2160
   gaugaacugc augguguaca aucucuauuu uccugguuac auuuaacuau uccucauguc 2220
   acaauaauau gcacauauag gcaugcaccc cccuauauaa gagaucauau uguagaucuu 2280
   aacaauguag augaacaaag uggauuauau agauaucaca ugggugguau ugaagggugg 2340
   ugucaaaaac uauggaccau agaagcuaua ucacuauugg aucuaauauc ucucaaaggg 2400
   aaauucucaa uuacugcuuu aauuaauggu gacaaucaau caauagauau aagcaaacca 2460
   gucagacuca uggaagguca aacucaugcu caagcagauu auuugcuagc auuaaauagc 2520
   cuuaaauuac uguauaaaga guaugcaggc auaggucaca aauuaaaagg aacugagacu 2580
   uauauaucac gagauaugca auuuaugagu aaaacaauuc aacauaacgg uguauauuac 2640
   ccugcuagua uaaagaaagu ccuaagagug ggaccgugga uaaacacuau acuugaugau 2700
   uucaaaguga gucuagaauc uauagguagu uugacacaag aauuagaaua uagaggugaa 2760
   agucuauuau gcaguuuaau auuuagaaau guaugguuau auaaucaaau ugcucuacaa 2820
   uuaaaaaauc augcguuaug uaacaauaaa uuauauuugg acauauuaaa gguucugaaa 2880
   cacuuaaaaa ccuuuuuuaa ucuugauaau auugauacag cauuaacauu guauaugaau 2940
   uuacccaugu uauuuggugg uggugauccc aacuuguuau aucgaaguuu cuauagaaga 3000
   acuccugauu uccucacaga ggcuauaguu cacucugugu ucauacuuag uuauuauaca 3060
   aaccaugacu uaaaagauaa acuucaagau uugucagaug auagauugaa uaaguucuua 3120
   acaugcauaa ucacguuuga caaaaacccu aaugcugaau ucguaacauu gaugagagau 3180
   ccucaagcuu uagggucuga gagacaagcu aaaauuacua gugaaaucaa uagacuggca 3240
   guuacagagg uuuugaguac agcuccaaac aaaauauucu ccaaaagugc acaacauuau 3300
   accacuacag agauagaucu aaaugauauu augcaaaaua uagaaccuac auauccucac 3360
   gggcuaagag uuguuuauga aaguuuaccc uuuuauaaag cagagaaaau aguaaaucuu 3420
   auaucaggua caaaaucuau aacuaacaua cuggaaaaga cuucugccau agacuuaaca 3480
   gauauugaua gagccacuga gaugaugagg aaaaacauaa cuuugcuuau aaggauacuu 3540
   ccauuggauu guaacagaga uaaaagagaa auauugagua uggaaaaccu aaguauuacu 3600
   gaauuaagca aauauguuag ggaaagaucu uggucuuuau ccaauauagu ugguguuaca 3660
   ucacccagua ucauguauac aauggacauc aaauauacaa caagcacuau agcuaguggc 3720
   auaauuauag agaaauauaa uguuaacagu uuaacacgug gugagagagg accaacuaaa 3780
   ccauggguug guucaucuac acaagagaaa aaaacaaugc caguuuauaa uagacaaguu 3840
   uuaaccaaaa aacaaagaga ucaaauagau cuauuagcaa aauuggauug gguguaugca 3900
   ucuauagaua acaaggauga auucauggaa gaacucagca uaggaacccu uggguuaaca 3960
   uaugaaaagg ccaaaaaauu auuuccacaa uauuuaagug ucaacuauuu gcaucgccuu 4020
   acagucagua guagaccaug ugaauucccu gcaucaauac cagcuuauag aacaacaaau 4080
   uaucacuuug acacuagccc uauuaaucgc auauuaacag aaaaguaugg ugaugaagau 4140
   auugacauag uauuccaaaa cuguauaagc uuuggccuua gcuuaauguc aguaguagaa 4200
   caauuuacua auguaugucc uaacagaauu auucucauac cuaagcuuaa ugagauacau 4260
   uugaugaaac cucccauauu cacaggugau guugauauuc acaaguuaaa acaagugaua 4320
   caaaaacagc auauguuuuu accagacaaa auaaguuuga cucaauaugu ggaauuauuc 4380
   uuaaguaaca aaacacucaa aucuggaucu cauguuaauu cuaauuuaau auuggcacau 4440
   aaaauaucug acuauuuuca uaauacuuac auuuuaagua cuaauuuagc uggacauugg 4500
   auucuaauua uacaacuuau gaaagauucu aaagguauuu uugaaaaaga uuggggagag 4560
   ggauauauaa cugaucauau guuuauuaau uugaaaguuu ucuucaaugc uuauaagacc 4620
   uaucucuugu guuuucauaa agguuauggc aaagcaaaac uggaguguga uaugaacacu 4680
   ucagaucuuc uauguguauu ggaauuaaua gacaguaguu auuggaaguc uaugucuaag 4740
   guauuuuuag aacaaaaagu uaucaaauac auucuuagcc aagaugcaag uuuacauaga 4800
   guaaaaggau gucauagcuu caaauuaugg uuucuuaaac gucuuaaugu agcagaauuu 4860
   acaguuugcc cuuggguugu uaacauagau uaucauccaa cacauaugaa agcaauauua 4920
   acuuauauag aucuuguuag aaugggauug auaaauauag auagaauaca cauuaaaaau 4980
   aaacacaaau ucaaugauga auuuuauacu ucuaaucucu uuuacauuaa uuauaacuuc 5040
   ucagauaaua cucaucuauu aacuaaacau auaaggauug cuaauucaga auuagaaaau 5100
   aauuacaaca aauuauauca uccuacacca gaaacccuag agaauauacu agccaauccg 5160
   auuaaaagua augacaaaaa gacacugaac gacuauugua uagguaaaaa uguugacuca 5220
   auaauguuac cauuguuauc uaauaagaag cuuguuaaau cgucugcaau gauuagaacc 5280
   aauuacagca aacaagaccu guacaaucua uucccuacgg uugugaucga uagaauuaua 5340
   gaucauucag guaauacagc caaauccaac caacuuuaca cuacuacuuc ccaucaaaua 5400
   ucuuuagugc acaauagcac aucacuuuau ugcaugcuuc cuuggcauca uauuaauaga 5460
   uucaauuuug uauuuaguuc uacagguugu aaaauuagua uagaguauau uuuaaaagac 5520
   cuuaaaauua aagauccuaa uuguauagca uucauaggug aaggagcagg gaauuuauua 5580
   uugcguacag ugguggaacu ucauccugac auaagauaua uuuacagaag ucugaaagau 5640
   ugcaaugauc auaguuuacc uauugaguuu uuaaggcuau acaauggaca uaucaacauu 5700
   gauuauggug aaaauuugac cauuccugcu acagaugcaa ccaacaacau ucauuggucu 5760
   uauuuacaua uaaaguuugc ugaaccuauc agucuuuuug uaugugaugc cgaauugccu 5820
   guaacaguca acuggaguaa aauuauaaua gaauggagca agcauguaag aaaaugcaag 5880
   uacuguuccu caguuaauaa auguacguua auaguaaaau aucaugcuca agaugauauu 5940
   gauuucaaau uagacaauau aacuauauua aaaacuuaug uaugcuuagg caguaaguua 6000
   aagggaucgg agguuuacuu aguccuuaca auagguccug caaauauauu uccaguauuu 6060
   aauguaguac aaaaugcuaa auugauacua ucaagaacca aaaauuucau caugccuaag 6120
   aaagcugaua aagagucuau ugaugcaaau auuaaaaguu ugauacccuu ucuuuguuac 6180
   ccuauaacaa aaaaaggaau uaauacugca uugucaaaac uaaagagugu uguuagugga 6240
   gauauacuau cauauucuau agcuggacgg aaugaaguuu ucagcaauaa acuuauaaau 6300
   cauaagcaua ugaacaucuu aaagugguuc aaucauguuu uaaauuucag aucaacagaa 6360
   cuaaacuaua accauuuaua uaugguagaa ucuacauauc cuuaccuaag ugaauuguua 6420
   aacagcuuga caacuaauga acuuaaaaaa cugauuaaaa ucacagguag ucuguuauac 6480
   aacuuucaua augaauaa 6498
<210> 18
   <211> 585
   <212> RNA
   <213> respiratory syncytial virus
<400> 18
   augucacgaa ggaauccuug caaauuugaa auucgagguc auugcuugaa ugguaagaga 60
   ugucauuuua gucauaauua uuuugaaugg ccaccccaug cacugcucgu aagacaaaac 120
   uuuauguuaa acagaauacu uaagucuaug gauaaaagua uagauaccuu aucagaaaua 180
   aguggagcug cagaguugga cagaacagaa gaguaugcuc uugguguagu uggagugcua 240
   gagaguuaua uaggaucaau aaauaauaua acuaaacaau cagcaugugu ugccaugagc 300
   aaacuccuca cugaacucaa uagugaugau aucaaaaaac ugagagacaa ugaagagcua 360
   aauucaccca agauaagagu guacaauacu gucauaucau auauugaaag caacaggaaa 420
   aacaauaaac aaacuaucca ucuguuaaaa agauugccag cagacguauu gaagaaaacc 480
   aucaaaaaca cauuggauau ccacaagagc auaaccauca acaacccaaa agaauuaacu 540
   guuagugaua caaaugacca ugccaaaaau aaugauacua ccuga 585
<210> 19
   <211> 273
   <212> RNA
   <213> respiratory syncytial virus
<400> 19
   augaccaugc caaaaauaau gauacuaccu gacaaauauc cuuguaguau aacuuccaua 60
   cuaauaacaa guagauguag agucacuaug uauaaucgaa agaacacacu auauuucaau 120
   caaaacaacc caaauaacca uauguacuca ccgaaucaaa cauucaauga aauccauugg 180
   accucacaag acuugauuga cacaauucaa aauuuucuac agcaucuagg uguuauugag 240
   gauauauaua caauauauau auuaguguca uaa 273
<210> 20
   <211> 726
   <212> RNA
   <213> respiratory syncytial virus
<400> 20
   auggaaaagu uugcuccuga auuccaugga gaagaugcaa acaacagggc uacuaaauuc 60
   cuagaaucaa uaaagggcaa auucacauca ccuaaagauc ccaagaaaaa agauaguauc 120
   auaucuguca acucaauaga uauagaagua accaaagaaa gcccuauaac aucaaauuca 180
   accauuauua acccaacaaa ugagacagau gauaaugcag ggaacaagcc caauuaucaa 240
   agaaaaccuc uaguaaguuu caaagaagac ccuauaccaa gugauaaucc cuuuucaaaa 300
   cuauacaaag aaaccauaga gacauuugau aacaaugaag aagaaucuag cuauucauau 360
   gaagaaauaa augaucagac gaacgauaau auaacugcaa gauuagauag gauugaugaa 420
   aaauuaagug aaauacuagg aaugcuucac acauuaguag uagcaagugc aggaccuaca 480
   ucugcuaggg augguauaag agaugccaug guugguuuaa gagaagaaau gauagaaaaa 540
   aucagaacug aagcauuaau gaccaaugac agauuagaag cuauggcaag acucaggaau 600
   gaggaaagug aaaagauggc aaaagacaca ucagaugaag ugucucucaa uccaacauca 660
   gagaaauuga acaaccuguu ggaagggaau gauagugaca augaucuauc acuugaagau 720
   uucuga 726
<210> 21
   <211> 420
   <212> RNA
   <213> respiratory syncytial virus
<400> 21
   augggcagca auucguugag uaugauaaaa guuagauuac aaaauuuguu ugacaaugau 60
   gaaguagcau uguuaaaaau aacaugcuau acugacaaau uaauacauuu aacuaaugcu 120
   uuggcuaagg cagugauaca uacaaucaaa uugaauggca uuguguuugu gcauguuauu 180
   acaaguagug auauuugccc uaauaauaau auuguaguaa aauccaauuu cacaacaaug 240
   ccagugcuac aaaauggagg uuauauaugg gaaaugaugg aauuaacaca uugcucucaa        300
   ccuaaugguc uaauagauga caauugugaa auuaaauucu ccaaaaaacu aagugauuca        360
   acaaugacca auuauaugaa ucaauuaucu gaauuacuug gauuugaucu uaauccauaa 420
<210> 22
   <211> 375
   <212> RNA
   <213> respiratory syncytial virus
<400> 22
   auggacacaa cccacaauga uaccacacca caaagacuga ugaucacaga caugagaccg 60
   uugucacuug agacuacaau aacaucacua accagagaca ucauaacaca cagauuuaua 120
   uacuuaauaa aucaugaaug cauagugaga aaacuugaug aaagacaggc cacauuuaca 180
   uuccugguca acuaugaaau gaaacuauug cacaaaguag gaagcacuaa auauaaaaaa 240
   uauacugaau acaacacaaa auauggcacu uucccuaugc cgauauucau caaucaugau 300
   ggguucuuag aaugcauugg cauuaagccu acaaagcaua cucccauaau auacaaguau 360
   gaucucaauc cauag 375
<210> 23
   <211> 42
   <212> DNA
   <213> Homo sapiens
<400> 23
   ggcgctgcct acggaggtgg cagccatctc cttctcggca tc 42
<210> 24
   <211> 186
   <212> DNA
   <213> Homo sapiens
<400> 24
   catcacattt aaaagcatct cagcctacca tgagaataag agaaagaaaa tgaagatcaa 60
   aagcttattc atctgttttt ctttttcgtt ggtgtaaagc caacaccctg tctaaaaaac 120
   ataaatttct ttaatcattt tgcctctttt ctctgtgctt caattaataa aaaatggaaa 180
   gaatct 186
<210> 25
   <211> 186
   <212> DNA
   <213> Homo sapiens
<400> 25
   catcacattt aaaagcatct cagcctacca tgagaataag agaaagaaaa tgaagatcaa 60
   tagcttattc atctcttttt ctttttcgtt ggtgtaaagc caacaccctg tctaaaaaac 120
   ataaatttct ttaatcattt tgcctctttt ctctgtgctt caattaataa aaaatggaaa 180
   gaacct 186
<210> 26
   <211> 110
   <212> DNA
   <213> Homo sapiens
<400> 26
   gctggagcct cggtggccat gcttcttgcc ccttgggcct ccccccagcc cctcctcccc 60
   ttcctgcacc cgtacccccg tggtctttga ataaagtctg agtgggcggc 110
<210> 27
   <211> 108
   <212> DNA
   <213> Homo sapiens
<400> 27
   gctggagcct cggtagccgt tcctcctgcc cgctgggcct cccaacgggc cctcctcccc 60
   tccttgcacc ggcccttcct ggtctttgaa taaagtctga gtgggcag 108
<210> 28
   <211> 132
   <212> DNA
   <213> Homo sapiens
<400> 28
   gctcgctttc ttgctgtcca atttctatta aaggttcctt tgttccctaa gtccaactac 60
   taaactgggg gatattatga agggccttga gcatctggat tctgcctaat aaaaaacatt 120
   tattttcatt gc 132
<210> 29
   <211> 44
   <212> DNA
   <213> Artificial Sequence
<220>
   <223> Center, alpha-complex-binding portion of the 3'UTR of an alpha-globin gene (muag)
<400> 29
   gcccgatggg cctcccaacg ggccctcctc ccctccttgc accg 44
<210> 30
   <211> 24
   <212> DNA
   <213> Artificial Sequence
<220>
   <223> particular preferred histone stem-loop sequence
<400> 30
   caaaggctct tttcagagcc acca 24
<210> 31
   <211> 1942
   <212> RNA
   <213> Artificial Sequence
<220>
   <223> RSV-F long (GC) R1691
<400> 31
   gggagaaagc uuaccaugga gcugcccauc cucaaggcca acgccaucac caccauccug 60
   gcggccguga cguucugcuu cgccagcucc cagaacauca ccgaggaguu cuaccagagc 120
   accugcuccg ccgucagcaa gggcuaccug uccgcccucc ggaccgggug guacacgagc 180
   gugaucacca ucgagcuguc caacaucaag gagaacaagu gcaacggcac cgacgcgaag 240
   gugaagcuga ucaaccagga gcucgacaag uacaagaacg ccgucaccga gcugcagcug 300
   cucaugcaga gcacgaccgc cgccaacaac cgcgcgcggc gcgagcugcc gcgguucaug 360
   aacuacaccc ugaacaacac caagaagacg aacgugaccc ucuccaagaa gcgcaagcgg 420
   cgcuuccugg gguuccugcu cggcgugggg agcgccaucg ccuccggcau cgccgucagc     480
   aaggugcugc accuggaggg cgaggugaac aagaucaagu ccgcccuccu gagcaccaac     540
   aaggcggucg ugucccugag caacggggug uccguccuca ccagcaaggu gcuggaccug     600
   aagaacuaca ucgacaagca gcuccugccc aucgugaaca agcaguccug ccggaucagc     660
   aacaucgaga cggucaucga guuccagcag aagaacaacc gccugcucga gaucacccgg     720
   gaguucagcg ugaacgccgg cgugaccacc cccgucucca cguacaugcu gaccaacagc     780
   gagcugcucu cccugaucaa cgacaugccc aucaccaacg accagaagaa gcugaugagc     840
   aacaacgugc agaucgugcg ccagcagucc uacagcauca uguccaucau caaggaggag     900
   guccucgccu acguggugca gcugccgcug uacgggguca ucgacacccc cugcuggaag     960
   cuccacacga gcccccugug caccaccaac accaaggagg gcuccaacau cugccugacg    1020
   cggaccgacc gcggguggua cugcgacaac gccggcagcg uguccuucuu cccccaggcc    1080
   gagaccugca agguccagag caaccgggug uucugcgaca ccaugaacuc ccucacgcug    1140
   ccgagcgagg ugaaccugug caacgucgac aucuucaacc ccaaguacga cugcaagauc    1200
   augaccucca agaccgacgu gagcuccagc gugaucaccu cccucggcgc gaucgucagc    1260
   ugcuacggga agacgaagug caccgccagc aacaagaacc gcggcaucau caagaccuuc    1320
   uccaacgggu gcgacuacgu gagcaacaag ggcguggaca ccgucuccgu gggcaacacc    1380
   cuguacuacg ugaacaagca ggaggggaag agccuguacg ucaagggcga gcccaucauc    1440
   aacuucuacg acccccucgu guucccgucc gacgaguucg acgccagcau cucccaggug    1500
   aacgagaaga ucaaccagag ccuggccuuc auccggaagu ccgacgagcu gcugcaccac    1560
   gucaacgccg ggaagagcac gaccaacauc augaucacca ccaucaucau cgugaucauc    1620
   gugauccucc ugucccugau cgcggucggc cuccugcugu acugcaaggc ccgcagcacg    1680
   cccgugaccc ucuccaagga ccagcugagc gggaucaaca acaucgccuu cuccaacuga    1740
   ggacuaguua uaagacugac uagcccgaug ggccucccaa cgggcccucc uccccuccuu    1800
   gcaccgagau uaauaaaaaa aaaaaaaaaa aaaaaaaaaa aaaaaaaaaa aaaaaaaaaa    1860
   aaaaaaaaaa aaaaaaaaug cauccccccc cccccccccc cccccccccc ccccaaaggc    1920
   ucuuuucaga gccaccagaa uu                                             1942
<210> 32
   <211> 2107
   <212> RNA
   <213> Artificial Sequence
<220>
   <223> RSV-F long (GC) R2510
<400> 32
   ggggcgcugc cuacggaggu ggcagccauc uccuucucgg caucaagcuu accauggagc      60
   ugcccauccu caaggccaac gccaucacca ccauccuggc ggccgugacg uucugcuucg     120
   ccagcuccca gaacaucacc gaggaguucu accagagcac cugcuccgcc gucagcaagg     180
   gcuaccuguc cgcccuccgg accggguggu acacgagcgu gaucaccauc gagcugucca     240
   acaucaagga gaacaagugc aacggcaccg acgcgaaggu gaagcugauc aaccaggagc     300
   ucgacaagua caagaacgcc gucaccgagc ugcagcugcu caugcagagc acgaccgccg     360
   ccaacaaccg cgcgcggcgc gagcugccgc gguucaugaa cuacacccug aacaacacca     420
   agaagacgaa cgugacccuc uccaagaagc gcaagcggcg cuuccugggg uuccugcucg     480
   gcguggggag cgccaucgcc uccggcaucg ccgucagcaa ggugcugcac cuggagggcg     540
   aggugaacaa gaucaagucc gcccuccuga gcaccaacaa ggcggucgug ucccugagca     600
   acgggguguc cguccucacc agcaaggugc uggaccugaa gaacuacauc gacaagcagc      660
   uccugcccau cgugaacaag caguccugcc ggaucagcaa caucgagacg gucaucgagu     720
   uccagcagaa gaacaaccgc cugcucgaga ucacccggga guucagcgug aacgccggcg     780
   ugaccacccc cgucuccacg uacaugcuga ccaacagcga gcugcucucc cugaucaacg     840
   acaugcccau caccaacgac cagaagaagc ugaugagcaa caacgugcag aucgugcgcc     900
   agcaguccua cagcaucaug uccaucauca aggaggaggu ccucgccuac guggugcagc     960
   ugccgcugua cggggucauc gacacccccu gcuggaagcu ccacacgagc ccccugugca    1020
   ccaccaacac caaggagggc uccaacaucu gccugacgcg gaccgaccgc gggugguacu    1080
   gcgacaacgc cggcagcgug uccuucuucc cccaggccga gaccugcaag guccagagca    1140
   accggguguu cugcgacacc augaacuccc ucacgcugcc gagcgaggug aaccugugca    1200
   acgucgacau cuucaacccc aaguacgacu gcaagaucau gaccuccaag accgacguga    1260
   gcuccagcgu gaucaccucc cucggcgcga ucgucagcug cuacgggaag acgaagugca 1320
   ccgccagcaa caagaaccgc ggcaucauca agaccuucuc caacgggugc gacuacguga 1380
   gcaacaaggg cguggacacc gucuccgugg gcaacacccu guacuacgug aacaagcagg 1440
   aggggaagag ccuguacguc aagggcgagc ccaucaucaa cuucuacgac ccccucgugu 1500
   ucccguccga cgaguucgac gccagcaucu cccaggugaa cgagaagauc aaccagagcc 1560
   uggccuucau ccggaagucc gacgagcugc ugcaccacgu caacgccggg aagagcacga 1620
   ccaacaucau gaucaccacc aucaucaucg ugaucaucgu gauccuccug ucccugaucg 1680
   cggucggccu ccugcuguac ugcaaggccc gcagcacgcc cgugacccuc uccaaggacc 1740
   agcugagcgg gaucaacaac aucgccuucu ccaacugagg acuagugcau cacauuuaaa 1800
   agcaucucag ccuaccauga gaauaagaga aagaaaauga agaucaauag cuuauucauc 1860
   ucuuuuucuu uuucguuggu guaaagccaa cacccugucu aaaaaacaua aauuucuuua 1920
   aucauuuugc cucuuuucuc ugugcuucaa uuaauaaaaa auggaaagaa ccuagaucua 1980
   aaaaaaaaaa aaaaaaaaaa aaaaaaaaaa aaaaaaaaaa aaaaaaaaaa aaaaaaaaaa 2040
   aaaugcaucc cccccccccc cccccccccc ccccccccca aaggcucuuu ucagagccac 2100
   cagaauu 2107
<210> 33
   <211> 2044
   <212> RNA
   <213> Artificial Sequence
<220>
   <223> RSV-Fdel554-574 long (GC) R2821
<400> 33
   ggggcgcugc cuacggaggu ggcagccauc uccuucucgg caucaagcuu accauggagc 60
   ugcccauccu caaggccaac gccaucacca ccauccuggc ggccgugacg uucugcuucg 120
   ccagcuccca gaacaucacc gaggaguucu accagagcac cugcuccgcc gucagcaagg 180
   gcuaccuguc cgcccuccgg accggguggu acacgagcgu gaucaccauc gagcugucca 240
   acaucaagga gaacaagugc aacggcaccg acgcgaaggu gaagcugauc aaccaggagc 300
   ucgacaagua caagaacgcc gucaccgagc ugcagcugcu caugcagagc acgaccgccg 360
   ccaacaaccg cgcgcggcgc gagcugccgc gguucaugaa cuacacccug aacaacacca 420
   agaagacgaa cgugacccuc uccaagaagc gcaagcggcg cuuccugggg uuccugcucg 480
   gcguggggag cgccaucgcc uccggcaucg ccgucagcaa ggugcugcac cuggagggcg 540
   aggugaacaa gaucaagucc gcccuccuga gcaccaacaa ggcggucgug ucccugagca 600
   acgggguguc cguccucacc agcaaggugc uggaccugaa gaacuacauc gacaagcagc 660
   uccugcccau cgugaacaag caguccugcc ggaucagcaa caucgagacg gucaucgagu 720
   uccagcagaa gaacaaccgc cugcucgaga ucacccggga guucagcgug aacgccggcg 780
   ugaccacccc cgucuccacg uacaugcuga ccaacagcga gcugcucucc cugaucaacg 840
   acaugcccau caccaacgac cagaagaagc ugaugagcaa caacgugcag aucgugcgcc 900
   agcaguccua cagcaucaug uccaucauca aggaggaggu ccucgccuac guggugcagc 960
   ugccgcugua cggggucauc gacacccccu gcuggaagcu ccacacgagc ccccugugca 1020
   ccaccaacac caaggagggc uccaacaucu gccugacgcg gaccgaccgc gggugguacu 1080
   gcgacaacgc cggcagcgug uccuucuucc cccaggccga gaccugcaag guccagagca 1140
   accggguguu cugcgacacc augaacuccc ucacgcugcc gagcgaggug aaccugugca 1200
   acgucgacau cuucaacccc aaguacgacu gcaagaucau gaccuccaag accgacguga 1260
   gcuccagcgu gaucaccucc cucggcgcga ucgucagcug cuacgggaag acgaagugca 1320
   ccgccagcaa caagaaccgc ggcaucauca agaccuucuc caacgggugc gacuacguga 1380
   gcaacaaggg cguggacacc gucuccgugg gcaacacccu guacuacgug aacaagcagg 1440
   aggggaagag ccuguacguc aagggcgagc ccaucaucaa cuucuacgac ccccucgugu 1500
   ucccguccga cgaguucgac gccagcaucu cccaggugaa cgagaagauc aaccagagcc 1560
   uggccuucau ccggaagucc gacgagcugc ugcaccacgu caacgccggg aagagcacga 1620
   ccaacaucau gaucaccacc aucaucaucg ugaucaucgu gauccuccug ucccugaucg 1680
   cggucggccu ccugcuguac ugcaaggccc gcugaggacu agugcaucac auuuaaaagc 1740
   aucucagccu accaugagaa uaagagaaag aaaaugaaga ucaauagcuu auucaucucu 1800
   uuuucuuuuu cguuggugua aagccaacac ccugucuaaa aaacauaaau uucuuuaauc 1860
   auuuugccuc uuuucucugu gcuucaauua auaaaaaaug gaaagaaccu agaucuaaaa 1920
   aaaaaaaaaa aaaaaaaaaa aaaaaaaaaa aaaaaaaaaa aaaaaaaaaa aaaaaaaaaa 1980
   ugcauccccc cccccccccc cccccccccc ccccccaaag gcucuuuuca gagccaccag 2040
   aauu 2044
<210> 34
   <211> 1558
   <212> RNA
   <213> Artificial Sequence
<220>
   <223> RSV-N (GC) R2831
<400> 34
   ggggcgcugc cuacggaggu ggcagccauc uccuucucgg caucaagcuu accauggccc 60
   ugagcaaggu gaagcucaac gacacccuga acaaggacca gcugcucucc agcuccaagu 120
   acaccaucca gcgcagcacg ggcgacucca ucgacacccc caacuacgac guccagaagc 180
   acaucaacaa gcugugcggg augcugcuca ucaccgagga cgccaaccac aaguucaccg 240
   gccugaucgg gaugcuguac gcgaugagcc ggcucggccg cgaggacacg aucaagaucc 300
   ugcgggacgc cggguaccac gugaaggcca acggcgugga cgucaccacc caccgccagg 360
   acaucaacgg caaggagaug aaguucgagg ugcugacccu cgccucccug acgaccgaga 420
   uccagaucaa caucgagauc gagagccgga aguccuacaa gaagaugcug aaggagaugg 480
   gggagguggc cccggaguac cgccacgaca gccccgacug cggcaugauc auccucugca 540
   ucgcggcccu ggucaucacc aagcuggccg ccggggaccg guccggccuc accgcgguga 600
   uccgccgggc caacaacgug cugaagaacg agaugaagcg cuacaagggg cugcucccca 660
   aggacaucgc caacagcuuc uacgaggucu ucgagaagca cccccacuuc aucgacgugu 720
   ucgugcacuu cggcaucgcc caguccagca cgcggggcgg gucccgcguc gagggcaucu 780
   ucgccgggcu guucaugaac gcguacggcg ccggccaggu gaugcugcgg uggggcgugc 840
   ucgccaagag cgucaagaac aucaugcugg ggcacgccuc cgugcaggcc gagauggagc 900
   agguggucga gguguacgag uacgcgcaga agcugggcgg cgaggccggg uucuaccaca 960
   uccucaacaa cccgaaggcc agccugcugu cccucaccca guucccgcac uucagcagcg 1020
   ugguccuggg gaacgccgcc ggccugggga ucaugggcga guaccgcggg accccgcgga 1080
   accaggaccu cuacgacgcg gccaaggccu acgccgagca gcugaaggag aacggcguga 1140
   ucaacuacuc cgugcuggac cucaccgccg aggagcugga ggcgaucaag caccagcuga 1200
   accccaagga caacgacguc gagcucugag gacuagugca ucacauuuaa aagcaucuca 1260
   gccuaccaug agaauaagag aaagaaaaug aagaucaaua gcuuauucau cucuuuuucu 1320
   uuuucguugg uguaaagcca acacccuguc uaaaaaacau aaauuucuuu aaucauuuug 1380
   ccucuuuucu cugugcuuca auuaauaaaa aauggaaaga accuagaucu aaaaaaaaaa 1440
   aaaaaaaaaa aaaaaaaaaa aaaaaaaaaa aaaaaaaaaa aaaaaaaaaa aaaaugcauc 1500
   cccccccccc cccccccccc cccccccccc aaaggcucuu uucagagcca ccagaauu 1558
<210> 35
   <211> 967
   <212> RNA
   <213> Artificial Sequence
<220>
   <223> RSV-M2-1 (GC) R2833
<400> 35
   ggggcgcugc cuacggaggu ggcagccauc uccuucucgg caucaagcuu accaugagcc 60
   gccggaaccc cugcaaguuc gagauccgcg gccacugccu gaacgggaag cggugccacu 120
   ucucccacaa cuacuucgag uggccgcccc acgcccuccu ggugcgccag aacuucaugc 180
   ugaaccggau ccucaagagc auggacaagu ccaucgacac ccugagcgag aucuccggcg 240
   ccgcggagcu ggaccgcacc gaggaguacg cccucggggu cgugggcgug cuggagagcu 300
   acaucggguc caucaacaac aucacgaagc agagcgccug cgucgccaug uccaagcugc 360
   ucaccgagcu gaacagcgac gacaucaaga agcugcggga caacgaggag cucaacuccc 420
   ccaagauccg cguguacaac accgugauca gcuacaucga guccaaccgg aagaacaaca 480
   agcagaccau ccaccugcug aagcgccucc ccgccgacgu ccugaagaag acgaucaaga 540
   acacccugga cauccacaag agcaucacca ucaacaaccc gaaggagcuc accguguccg 600
   acacgaacga ccacgcgaag aacaacgaca ccaccugagg acuagugcau cacauuuaaa 660
   agcaucucag ccuaccauga gaauaagaga aagaaaauga agaucaauag cuuauucauc 720
   ucuuuuucuu uuucguuggu guaaagccaa cacccugucu aaaaaacaua aauuucuuua 780
   aucauuuugc cucuuuucuc ugugcuucaa uuaauaaaaa auggaaagaa ccuagaucua 840
   aaaaaaaaaa aaaaaaaaaa aaaaaaaaaa aaaaaaaaaa aaaaaaaaaa aaaaaaaaaa 900
   aaaugcaucc cccccccccc cccccccccc ccccccccca aaggcucuuu ucagagccac 960
   cagaauu 967
<210> 36
   <211> 75
   <212> DNA
   <213> Homo sapiens
<400> 36
   gcggctcggc cattttgtcc cagtcagtcc ggaggctgcg gctgcagaag taccgcctgc 60
   ggagtaactg caaag 75
<210> 37
   <211> 24
   <212> RNA
   <213> Artificial Sequence
<220>
   <223> histone stem-loop sequence
<400> 37
   caaaggcucu uuucagagcc acca 24